(19) Europäisches Patentamt
European Patent Office
Office européen des brevets

(11) Publication number: **0 512 570 B1**

# (12) EUROPEAN PATENT SPECIFICATION

(45) Date of publication of patent specification: **11.10.95**

(51) Int. Cl.[6]: **C07D 333/58**, C07D 307/81, C07D 209/14, C07D 407/12, C07D 405/12, C07D 409/12, A61K 31/44, A61K 31/40, A61K 31/38, A61K 31/335

(21) Application number: **92107863.0**

(22) Date of filing: **11.05.92**

## (54) Urea derivatives, processes for the preparation thereof and pharmaceutical composition comprising the same.

(30) Priority: **10.05.91 GB 9110133**
**14.06.91 GB 9112823**
**23.12.91 GB 9127277**

(43) Date of publication of application:
**11.11.92 Bulletin 92/46**

(45) Publication of the grant of the patent:
**11.10.95 Bulletin 95/41**

(84) Designated Contracting States:
**AT BE CH DE DK ES FR GB GR IT LI LU NL PT SE**

(56) References cited:
**EP-A- 0 354 994**
**EP-A- 0 399 422**
**WO-A-91/13871**
**US-A- 4 623 662**

**Journal of Medicinal Chemistry 32(10) (1989) 2318-2325**

(73) Proprietor: **FUJISAWA PHARMACEUTICAL CO., LTD.**
**4-7, Doshomachi 3-chome**
**Chuo-ku**
**Osaka-shi**
**Osaka 541 (JP)**

(72) Inventor: **Yoshikuni, Itoh**
**4-16-4-305, Azuma,**
**Tsukuba-shi**
**Ibaraki 305 (JP)**
Inventor: **Kazuhiko, Ohne**
**2-25-10, Matsushiro,**
**Tsukuba-shi**
**Ibaraki 305 (JP)**
Inventor: **Hirokazu, Tanaka**
**1-4-8, Ottominami,**
**Tsuchiura-shi**
**Ibaraki 300 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **Türk, Dietmar, Dr. rer. nat.**
**Türk, Gille, Hrabal, Leifert**
**Patentanwälte**
**Brucknerstrasse 20**
**D-40593 Düsseldorf (DE)**

## Description

This invention relates to new urea derivatives and pharmaceutically acceptable salts thereof.

More particularly, it relates to new urea derivatives and pharmaceutically acceptable salts thereof which have an inhibitory activity against acyl-CoA : cholesterol acyltransferase enzyme (hereinafter, ACAT), to processes for the preparation thereof, to a pharmaceutical composition comprising the same and to a method for the prevention and/or treatment of hypercholesterolemia, hyperlipidemia, atherosclerosis or diseases caused thereby.

One object of this invention is to provide new and useful urea derivatives and pharmaceutically acceptable salts which possess an inhibitory activity against ACAT.

Another object of this invention is to provide processes for preparation of said urea derivatives and salts thereof.

A further object of this invention is to provide a pharmaceutical composition comprising, as an active ingredient, said urea derivatives and pharmaceutically acceptable salts thereof.

Still further object of this invention is to provide a therapeutic method for the prevention and/or treatment of hypercholesterolemia, hyperlipidemia, atherosclerosis or diseases caused thereby in human beings or animals, using said urea derivatives and pharmaceutically acceptable salts thereof.

High levels of blood cholesterol and blood lipids are conditions which are involved in the onset of atherosclerosis.

It is well known that inhibition of ACAT-catalyzed cholesterol esterification could lead to diminish intestinal absorption of cholesterol as well as a decrease in the intracellular accumulation of cholesterol esters in the intima of the arterial wall. Therefore, ACAT inhibitors are useful for the prevention and/or treatment of hypercholesterolemia, hyperlipidemia, atherosclerosis or diseases caused thereby such as cardiac insufficiency (e.g. angina pectoris, and myocardial infarction), cerebrovascular disturbance (e.g. cerebral infarction, and cerebral apoplexy), arterial aneurism, peripheral vascular disease, xanthomas, and restenosis after percutaneous transluminal coronary angioplasty.

Some urea derivatives have been known as ACAT inhibitors, for example, in U.S. Patent Nos. 4,473,579 and 4,623,662, EP Patent Application Publication Nos. 0354994 and 0399422 and PCT Patent Application No. PCT/WO91/13871.

Furthermore, Journal of Medicinal Chemistry 32 (10) (1989) 2318-2325 discloses di- and trisubstituted urea derivatives as ACAT inhibitors, but lower alkyl substituted with benzofuranyl, benzothienyl or indolyl is not included as the substituent of said urea derivatives.

The object urea derivatives of this invention are new and can be represented by the following general formula (I) :

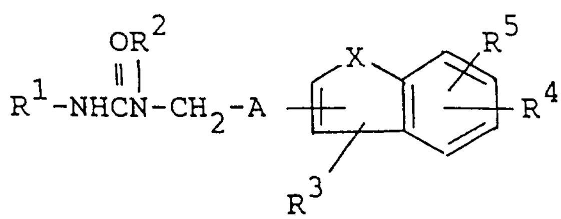

(I)

wherein

- $R^1$ is phenyl which may be substituted with $C_1$-$C_6$ alkyl, halogen, nitro, amino, $C_1$-$C_6$ alkylamino, $C_1$-$C_6$ alkoxy or acylamino,
- $R^2$ is hydrogen; $C_1$-$C_{20}$ alkyl; cyclo($C_3$-$C_{20}$)alkyl; or $C_1$-$C_6$ alkyl which is substituted with cyclo($C_3$-$C_6$)alkyl, cyclo($C_3$-$C_6$)alkenyl, furyl or phenyl optionally substituted with substituent(s) selected from the group consisting of $C_1$-$C_6$ alkyl, halogen, hydroxy and $C_1$-$C_6$ alkoxy;
- $R^3$ is hydrogen, $C_1$-$C_6$ alkyl or phenyl which may be substituted with $C_1$-$C_6$ alkyl, halogen, nitro, amino or $C_1$-$C_6$ alkylamino,
- $R^4$ is hydrogen, halogen, $C_1$-$C_6$ alkyl, $C_1$-$C_6$ alkoxy or phenyl which may be substituted with $C_1$-$C_6$ alkyl or halogen,
- $R^5$ is hydrogen, halogen, $C_1$-$C_6$ alkyl or phenyl which may be substituted with $C_1$-$C_6$ alkyl,
- A is a single bond or $C_1$-$C_6$ alkylene, and
- X is O, S or NH,

in which at least one of $R^3$, $R^4$ and $R^5$ is not hydrogen,

provided that $R^3$ is phenyl which may be substituted with $C_1$-$C_6$ alkyl, halogen, nitro, amino or $C_1$-$C_6$ alkylamino, or

$R^4$ is halogen, $C_1$-$C_6$ alkoxy or phenyl which may be substituted with $C_1$-$C_6$ alkyl or halogen, when $R^2$ is cyclo($C_3$-$C_{20}$)alkyl,

and pharmaceutically acceptable salts thereof.

The object compound (I) or its salt can be prepared by processes as illustrated in the following reaction schemes.

Process 1

$R^1$-NCO

(II)

+

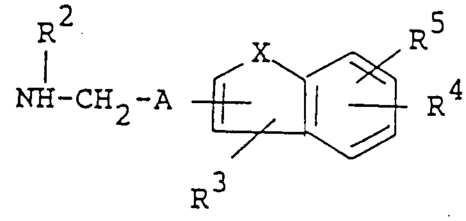

(III)
or its salt

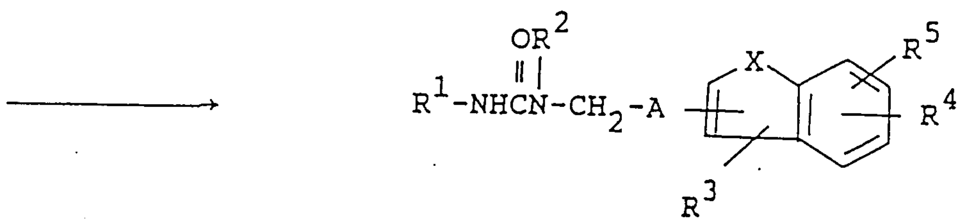

(I)
or its salt

Process 2

$R^1$-$NH_2$

(IV)
or its salt

+

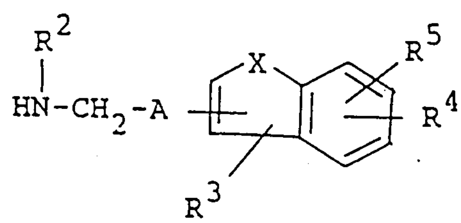

(III)
or its salt

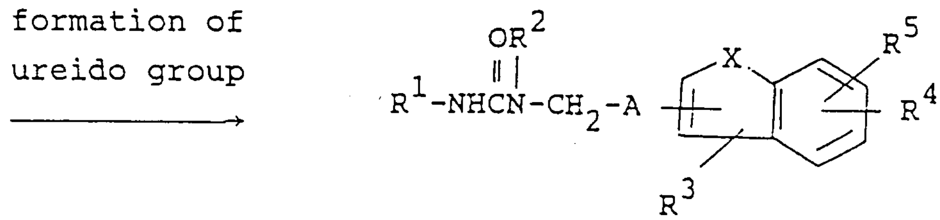


(I)

or its salt

Process 3

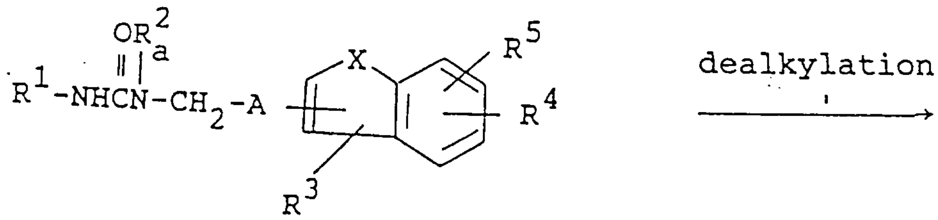


(Ia)

or its salt

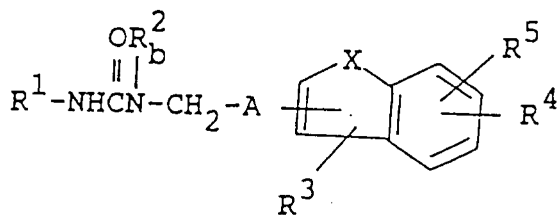

(Ib)

or its salt

Process 4

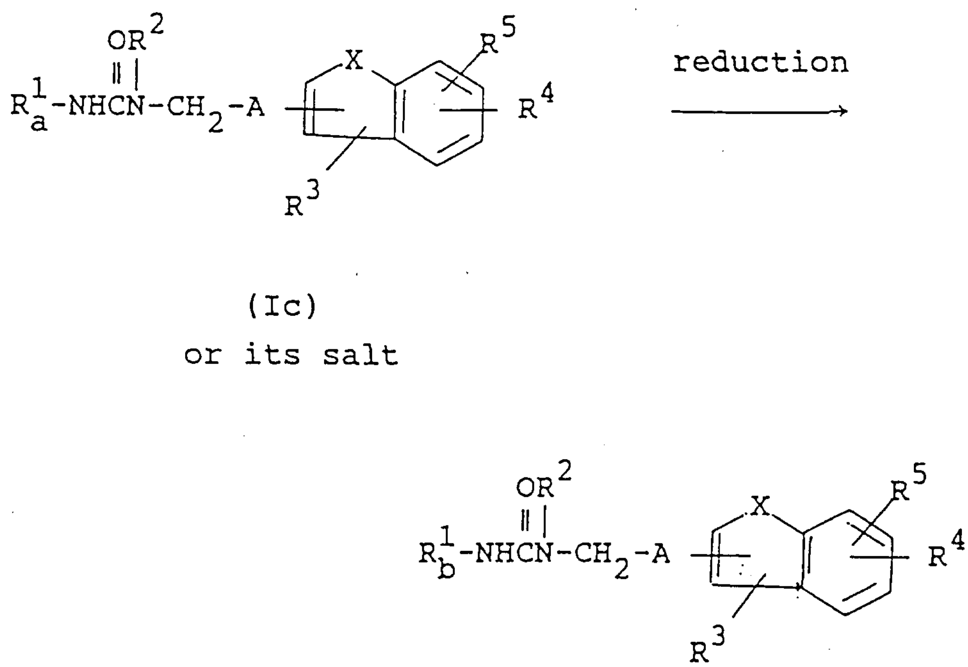


(Ic)
or its salt

(Id)
or its salt

Process 5

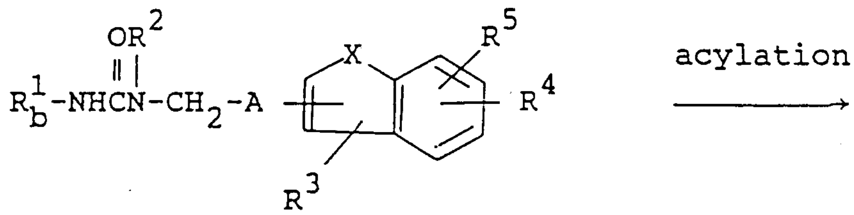


(Id)
or its salt

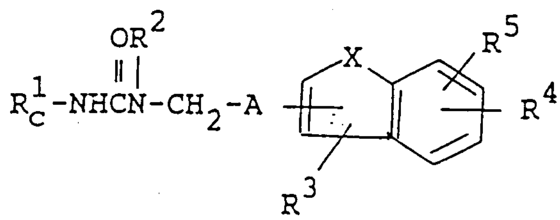

(Ie)
or its salt

Process 6

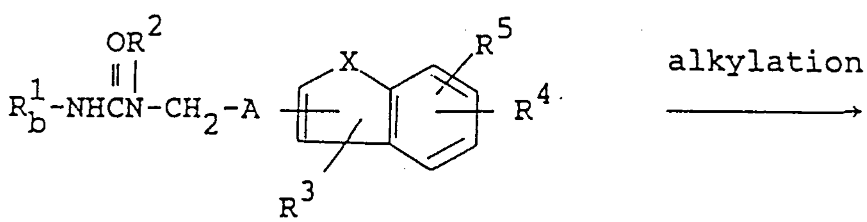


(Id)
or its salt

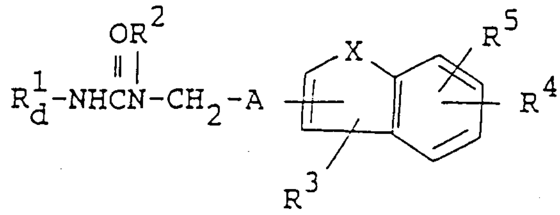

(If)

or its salt

wherein

| | |
|---|---|
| $R^1$, $R^2$, $R^3$, $R^4$, $R^5$, A and X | are each as defined above, |
| $R^2_a$ is | $C_1$-$C_6$ alkyl substituted with phenyl on $C_1$-$C_6$ alkyl-substituted phenyl, each of which is substituted with $C_1$-$C_6$ alkoxy, |
| $R^2_b$ is | $C_1$-$C_6$ alkyl substituted with phenyl on $C_1$-$C_6$ alkyl-substituted phenyl, each of which is substituted with hydroxy, |
| $R^1_a$ | is phenyl on $C_1$-$C_6$ alkyl-substituted phenyl, each of which is substituted with nitro, |
| $R^1_b$ | is phenyl on $C_1$-$C_6$ alkyl-substituted phenyl, each of which is substituted with amino, |
| $R^1_c$ | is phenyl on $C_1$-$C_6$ alkyl-substituted phenyl, each of which is substituted with acylamino, and |
| $R^1_d$ | is phenyl on $C_1$-$C_6$ alkyl-substituted phenyl, each of which is substituted with $C_1$-$C_6$ alkylamino. |

In the above and subsequent description of the present specification, suitable examples of the various definitions to be included within the scope of the invention are explained in detail in the following.

The term "$C_1$-$C_6$" is intended to mean a group having 1 to 6 carbon atom(s).

The $C_3$-$C_6$ moiety in the term "cyclo($C_3$-$C_6$)alkyl" is intended to mean a group having 3 to 6 carbon atoms.

The $C_3$-$C_6$ moiety in the term "cyclo($C_3$-$C_6$)alkenyl" is intended to mean a group having 3 to 6 carbon atoms.

The term "$C_1$-$C_{20}$ alkyl" may include $C_1$-$C_6$ alkyl and $C_7$-$C_{20}$ alkyl.

The term "cyclo($C_3$-$C_{20}$)alkyl" may include cyclo($C_3$-$C_6$)alkyl and cyclo($C_7$-$C_{20}$)alkyl.

Suitable "$C_1$-$C_6$ alkyl" and $C_1$-$C_6$ alkyl moiety in the terms "$C_1$-$C_6$ alkylamino" and "phenyl($C_1$-$C_6$)-alkyl" may be a straight or branched one such as methyl, ethyl, propyl, isopropyl, butyl, isobutyl, tert-butyl, pentyl, isopentyl, and hexyl, in which preferable one is methyl, ethyl, propyl, isopropyl, pentyl or isopentyl.

Suitable "cyclo($C_3$-$C_6$)alkyl" may be cyclopropyl, cyclobutyl, cyclopentyl or cyclohexyl.

Suitable "cyclo($C_3$-$C_6$)alkenyl" may be cyclopropenyl, cyclobutenyl, cyclopentenyl or cyclohexenyl.

The term "$C_7$-$C_{20}$" is intended to mean 7 to 20 carbon atoms.

Suitable "$C_7$-$C_{20}$ alkyl" may be a straight or branched one such as heptyl, octyl, nonyl, decyl, undecyl, dodecyl, tridecyl, tetradecyl, pentadecyl, hexadecyl, heptadecyl, octadecyl, nonadecyl, eicosyl, methylheptyl, methyloctyl, methylnonyl, methyldecyl, ethylheptyl, ethyloctyl, ethylnonyl, and ethyldecyl, in which preferable one is one having 7 to 10 carbon atoms and the most preferable one is heptyl or nonyl.

Suitable "cyclo($C_7$-$C_{20}$)alkyl" may be cycloheptyl, cyclooctyl, cyclononyl, cyclodecyl, cycloundecyl, cyclotridecyl, cyclotetradecyl, cyclopentadecyl, cyclohexadecyl, cycloheptadecyl, cyclooctadecyl, cyclononadecyl, and cycloeicosyl, in which preferable one is having 7 to 10 carbon atoms and the most preferable one is cycloheptyl.

Suitable "$C_1$-$C_6$ alkoxy" may be a straight or branched one such as methoxy, ethoxy, propoxy, isopropoxy, butoxy, isobutoxy, tert-butoxy, pentyloxy, and hexyloxy, in which preferable one is methoxy.

Suitable "halogen" may be fluorine, chlorine, bromine and iodine, in which preferable one is fluorine or chlorine.

Suitable "aryl" may be phenyl, naphthyl, phenyl substituted with $C_1$-$C_6$ alkyl (e.g. tolyl, xylyl, mesityl, cumenyl, and diisopropylphenyl), in which preferable one is phenyl or phenyl substituted with $C_1$-$C_6$ alkyl.

Suitable "$C_1$-$C_6$ alkylamino" may be mono or di($C_1$-$C_6$ alkyl)amino such as methylamino, ethylamino, dimethylamino, and diethylamino, in which preferable one is dimethylamino.

Suitable "phenyl($C_1$-$C_6$)alkyl" may be benzyl, phenethyl, and phenylpropyl, in which preferable one is benzyl.

Suitable "$C_1$-$C_6$ alkylene" may be a straight or branched one such as methylene, ethylene, trimethylene, propylene, tetramethylene, pentamethylene, hexamethylene, and ethylethylene.

The phenyl groups for $R^1$, $R^3$ and $R^4$ may be substituted with 1 to 5 substituent(s) as mentioned above, wherein the preferable number of the substituent(s) is 1, 2 or 3.

The phenyl group as substituent of lower alkyl for $R^2$ may be substituted with 1 to 5 substituent(s) as stated above, wherein the preferable number of the substituent(s) is 1, 2 or 3.

Preferable "phenyl substituted with halogen" is chlorophenyl, dichlorophenyl, difluorophenyl, trichlorophenyl or trifluorophenyl.

Suitable "heterocyclic group" may include saturated or unsaturated, monocyclic or polycyclic one containing at least one hetero atom such as nitrogen atom, oxygen atom or sulfur atom.

The preferred examples of thus defined "heterocyclic group" may be unsaturated, 3 to 8-membered, more preferably 5 or 6-membered heteromonocyclic group containing 1 to 4-nitrogen atom(s), for example, pyrrolyl, imidazolyl, pyrazolyl, pyridyl, pyridyl N-oxide, dihydropyridyl, tetrahydropyridyl, pyrimidyl, pyrazinyl, pyridazinyl, triazinyl, triazolyl, tetrazinyl, and tetrazolyl;

saturated, 3 to 8-membered, more preferably 5 or 6-membered heteromonocyclic group containing 1 to 4 nitrogen atom(s), for example, pyrrolidinyl, imidazolidinyl, piperidino, and piperazinyl;

unsaturated, condensed heterocyclic group containing 1 to 5 nitrogen atom(s), for example, indolyl, isoindolyl, indolizinyl, benzimidazolyl, quinolyl, isoquinolyl, indazolyl, and benzotriazolyl;

unsaturated, 3 to 8-membered heteromonocyclic group containing 1 to 2 oxygen atom(s) and 1 to 3 nitrogen atom(s), for example, oxazolyl, isoxazolyl, and oxadiazolyl;

saturated, 3 to 8-membered heteromonocyclic group containing 1 to 2 oxygen atom(s) and 1 to 3 nitrogen atom(s), for example, morpholino, and sydnonyl;

unsaturated, condensed heterocyclic group containing 1 to 2 oxygen atom(s) and 1 to 3 nitrogen atom(s), for example, benzoxazolyl, and benzoxadiazolyl;

unsaturated, 3 to 8-membered heteromonocyclic group containing 1 to 2 sulfur atom(s) and 1 to 3 nitrogen atom(s), for example thiazolyl, isothiazolyl, and thiadiazolyl;

unsaturated, 3 to 8-membered heteromonocyclic group containing 1 to 2 sulfur atom(s), for example, thienyl;

unsaturated condensed heterocyclic group containing 1 to 2 sulfur atom(s) and 1 to 3 nitrogen atom(s), for example, benzothiazolyl, and benzothiadiazolyl;

unsaturated, 3 to 8-membered heteromonocyclic group containing an oxygen atom, for example, furyl;

unsaturated, condensed heterocyclic group containing 1 to 2 sulfur atom(s), for example, benzothienyl;

unsaturated, condensed heterocyclic group containing 1 to 2 oxygen atom(s) for example, benzofuranyl.

Preferable one in a heterocyclic group is pyridyl or furyl.

Suitable acyl moiety in the term "acylamino" may be carboxy; esterified carboxy; carbamoyl optionally substituted with substituent(s) selected from the group consisting of $C_1$-$C_6$ alkyl, cyclo($C_3$-$C_6$)alkyl, aryl and hydroxy; $C_1$-$C_6$ alkanoyl; a heterocycliccarbonyl; and $C_1$-$C_6$ alkylsulfonyl.

The esterified carboxy may be substituted or unsubstituted $C_1$-$C_6$ alkoxycarbonyl [e.g. methoxycarbonyl, ethoxycarbonyl, propoxycarbonyl, butoxycarbonyl, hexyloxycarbonyl, 2-iodoethoxycarbonyl, and 2,2,2-trichloroethoxycarbonyl], substituted or unsubstituted aryloxycarbonyl [e.g. phenoxycarbonyl, 4-nitrophenoxycarbonyl, and 2-naphthyloxycarbonyl], substituted or unsubstituted ar($C_1$-$C_6$)alkoxycarbonyl [e.g. benzyloxycarbonyl, phenethyloxycarbonyl, benzhydryloxycarbonyl, and 4-nitrobenzyloxycarbonyl].

The $C_1$-$C_6$ alkanoyl may be formyl, acetyl, propionyl, butyryl, isobutyryl, valeryl, isovaleryl, pivaloyl, and hexanoyl, in which preferable one is acetyl.

The heterocyclic moiety in the term "heterocycliccarbonyl" may be the same as those exemplified for "heterocyclic group".

The $C_1$-$C_6$ alkylsulfonyl may be methylsulfonyl, ethylsulfonyl, and propylsulfonyl, in which the preferable one is methylsulfonyl.

Suitable "acylamino" may be $C_1$-$C_6$ alkanoylamino and $C_1$-$C_6$ alkylsulfonylamino, in which preferable one is acetylamino or methylsulfonylamino.

Preferable compound (I) is one which has phenyl optionally substituted with $C_1$-$C_6$ alkyl, halogen, nitro, amino, $C_1$-$C_6$ alkylamino, $C_1$-$C_6$ alkoxy, $C_1$-$C_6$ alkanoylamino or $C_1$-$C_6$ alkylsulfonylamino for $R^1$, hydrogen; $C_1$-$C_{20}$ alkyl; cyclo($C_3$-$C_{20}$)alkyl; or $C_1$-$C_6$ alkyl substituted with cyclo($C_3$-$C_6$)alkyl, cyclo($C_3$-$C_6$)alkenyl, furyl or phenyl optionally substituted with $C_1$-$C_6$ alkyl, halogen, hydroxy or $C_1$-$C_6$ alkoxy for $R^2$, hydrogen, $C_1$-$C_6$ alkyl or phenyl optionally substituted with $C_1$-$C_6$ alkyl or halogen for $R^3$, hydrogen, halogen, $C_1$-$C_6$ alkyl or phenyl for $R^4$, hydrogen or $C_1$-$C_6$ alkyl for $R^5$, a single bond or $C_1$-$C_6$ alkylene for A, and O or S for X; or

phenyl optionally substituted with $C_1$-$C_6$ alkyl, halogen, nitro, amino, $C_1$-$C_6$ alkylamino or $C_1$-$C_6$ alkoxy for R1, $C_1$-$C_{20}$ alkyl, cyclo($C_3$-$C_{20}$)alkyl or phenyl($C_1$-$C_6$)alkyl for $R^2$, hydrogen, $C_1$-$C_6$ alkyl or phenyl optionally substituted with halogen, nitro, amino or $C_1$-$C_6$ alkylamino for $R^3$, hydrogen, halogen, $C_1$-$C_6$ alkyl, $C_1$-$C_6$ alkoxy or phenyl optionally substituted with halogen for $R^4$, hydrogen for $R^5$, a single bond or $C_1$-$C_6$ alkylene for A, and O or S for X.

More preferable compound (I) is one which has phenyl substituted with halogen for $R^1$, $C_1$-$C_{20}$ alkyl or phenyl($C_1$-$C_6$)alkyl for $R^2$, phenyl substituted with halogen for $R^3$, $C_1$-$C_6$ alkyl for $R^4$, hydrogen for $R^5$, a single bond or $C_1$-$C_6$ alkylene for A, and O for X.

Suitable pharmaceutically acceptable salts of the object compound (I) are conventional non-toxic salts such as an inorganic acid addition salt [e.g. hydrochloride, hydrobromide, sulfate, and phosphate], an organic acid addition salt [e.g. formate, acetate, trifluoroacetate, maleate, tartrate, methanesulfonate, benzenesulfonate, and toluenesulfonate], and an alkali metal salt [e.g. sodium salt, and potassium salt].

The processes for preparing the object compound (I) are explained in detail in the following.

## Process 1

The object compound (I) or its salt can be prepared by reacting a compound (II) with a compound (III) or its salt.

Suitable salt of the compound (III) may include an acid addition salt such as an inorganic acid addition salt [e.g. hydrochloride, hydrobromide, sulfate, and phosphate], an organic acid addition salt [e.g. formate, acetate, trifluoroacetate, maleate, tartrate, methanesulfonate, benzenesulfonate, and toluenesulfonate], and an inorganic base salt [e.g. sodium salt, and potassium salt].

The reaction is usually carried out in a conventional solvent such as dioxane, chloroform, methylene chloride, ethylene chloride, tetrahydrofuran, ethyl acetate, or any other organic solvent which does not adversely influence the reaction.

The reaction may also be carried out in the presence of an inorganic or organic base such as an alkali metal bicarbonate, tri($C_1$-$C_6$)alkylamine, pyridine, N-($C_1$-$C_6$)alkylmorpholine, and N-N-di($C_1$-$C_6$)-alkylbenzylamine.

The reaction temperature is not critical, and the reaction is preferably carried out under cooling or at ambient temperature.

## Process 2

The object compound (I) or its salt can be prepared by subjecting a compound (IV) or its salt and a compound (III) or its salt to formation reaction of ureido group.

Suitable salts of the compounds (III) and (IV) may be the same as those exemplified for the compound (I).

This reaction is carried out in the presence of reagent which introduces carbonyl group such as phosgene, haloformate compound [e.g. ethyl chloroformate, and trichloromethyl chloroformate], N,N'-carbonyldiimidazole, metal carbonyl compounds [e.g. cobalt carbonyl, and manganese carbonyl], a combination of carbon monoxide and catalysts such as palladium chloride.

This reaction is usually carried out in a solvent such as dioxane, tetrahydrofuran, benzene, toluene, chloroform, methylene chloride, N,N-dimethylformamide, ethyl acetate or any other organic solvent which does not adversely influence the reaction.

The reaction temperature is not critical and the reaction is usually carried out under cooling to heating.

## Process 3

The object compound (Ib) or its salt can be prepared by subjecting a compound (Ia) or its salt to dealkylation reaction.

Suitable salts of the compounds (Ia) and (Ib) may be acid addition salts as exemplified for the compound (I).

The reaction is carried out in the presence of an acid including Lewis acid [e.g. hydrochloric acid, hydrobromic acid, hydroiodic acid, boron tribromide, and boron trichloride] or tri($C_1$-$C_6$ alkyl)silyliodide [e.g. trimethylsilyliodide].

The reaction is usually carried out in a solvent such as water, acetic acid, methylene chloride, tetrahydrofuran, a mixture thereof or any other solvent which does not adversely influence the reaction.

The reaction temperature is not critical and the reaction is usually carried out under cooling to heating.

Process 4

The object compound (Id) or its salt can be prepared by subjecting a compound (Ic) or its salt to reduction.

Suitable salts of the compounds (Ic) and (Id) may be referred to the ones as exemplified for the compound (I).

The present reduction is carried out by chemical reduction, or catalytic reduction.

Suitable reducing agents to be used in chemical reduction are a combination of metal [e.g. tin, zinc, and iron] or metallic compound [e.g. chromium chloride, and chromium acetate] and an organic or inorganic acid [e.g. formic acid, acetic acid, propionic acid, trifluoroacetic acid, p-toluenesulfonic acid, hydrochloric acid, and hydrobromic acid].

Suitable catalysts to be used in catalytic reduction are conventional ones such as platinum catalyst [e.g. platinum plate, spongy platinum, platinum black, colloidal platinum, platinum oxide, and platinum wire], palladium catalyst [e.g. spongy palladium, palladium black, palladium oxide, palladium on carbon, colloidal palladium, palladium on barium sulfate, and palladium on barium carbonate], nickel catalyst [e.g. reduced nickel, nickel oxide, and Raney nickel], cobalt catalyst [e.g. reduced cobalt, and Raney cobalt], iron catalyst [e.g. reduced iron, and Raney iron], copper catalyst [e.g. reduced copper, Raney copper, and Ullman copper].

The reduction is usually carried out in a conventional solvent which does not adversely influence the reaction such as water, an alcohol [e.g. methanol, ethanol, and propanol], N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent and other conventional solvent such as diethyl ether, methylene chloride, dioxane, and tetrahydrofuran, or a mixture thereof.

The reaction temperature of this reduction is not critical and the reaction is usually carried out under cooling to warming.

In this reaction, in case that the compound (Ic) having phenyl substituted with nitro for $R^3$ is used as a starting compound, the compound (Id) having phenyl substituted with amino for $R^3$ may be obtained according to reaction conditions. This case is included within the scope of the present reaction.

Process 5

The object compound (Ie) or its salt can be prepared by reacting a compound (Id) or its salt with an acylating agent.

Suitable salt of the compound (Ie) may be the same as those exemplified for the compound (I).

The acylating agent may be include an organic acid represented by the formula : $R^6$-OH, in which $R^6$ is acyl as illustrated above, or its reactive derivative.

The suitable reactive derivative of organic acid may be a conventional one such as an acid halide [e.g. acid chloride, and acid bromide], an acid azide, an acid anhydride, an activated amide, and an activated ester.

When free acid is used as an acylating agent, the acylation reaction may preferably be conducted in the presence of a conventional condensing agent such as N,N'-dicyclohexylcarbodiimide.

The reaction is usually carried out in a conventional solvent such as water, acetone, dioxane, chloroform, methylene chloride, acetonitrile, ethylene chloride, tetrahydrofuran, ethyl acetate, N,N-dimethylformamide, pyridine or any other organic solvent which does not adversely influence the reaction, or a mixture thereof.

The reaction is also preferably carried out in the presence of a conventional base such as triethylamine, pyridine, and sodium hydroxide.

The reaction temperature is not critical, and the reaction can be carried out under cooling to heating.

Process 6

The object compound (If) or its salt can be prepared by reacting a compound (Id) or its salt with an alkylating agent.

Suitable salts of the compound (If) may be the same as those exemplified for the compound (I).

Suitable alkylating agent may be $C_1$-$C_6$ alkyl halide [e.g. methyl iodide, and ethyl bromide], or a combination of a carbonyl compound such as aliphatic ketone [e.g. acetone, and ethyl methyl ketone], carbaldehyde [e.g. formaldehyde, and ethanal], orthocarboxylic acid ester [e.g. triethyl orthoformate], and a

reducing agent including chemical and catalytic ones [e.g. formic acid, sodium borohydride, sodium cyanoborohydride, palladium on carbon, etc.].

The reaction is usually carried out in a conventional solvent which does not adversely influence the reaction such as water, dioxane, an alcohol [e.g. methanol, and ethanol], acetonitrile, tetrahydrofuran, N,N-dimethylformamide, or a mixture thereof. Additionally, in case that the above-mentioned alkylating agent are in liquid, they can also be used as a solvent.

The reaction temperature is not critical and the reaction can be carried out under cooling to heating.

In this reaction, in case that the compound (Id) having phenyl substituted with amino for $R^3$ is used as a starting compound, the compound (If) having phenyl substituted with $C_1$-$C_6$ alkylamino may be obtained according to reaction conditions. This case is included within the scope of the present reaction.

Among the starting compound (III), some of them are new and can be prepared by processes as illustrated in the following reaction schemes.

Process A

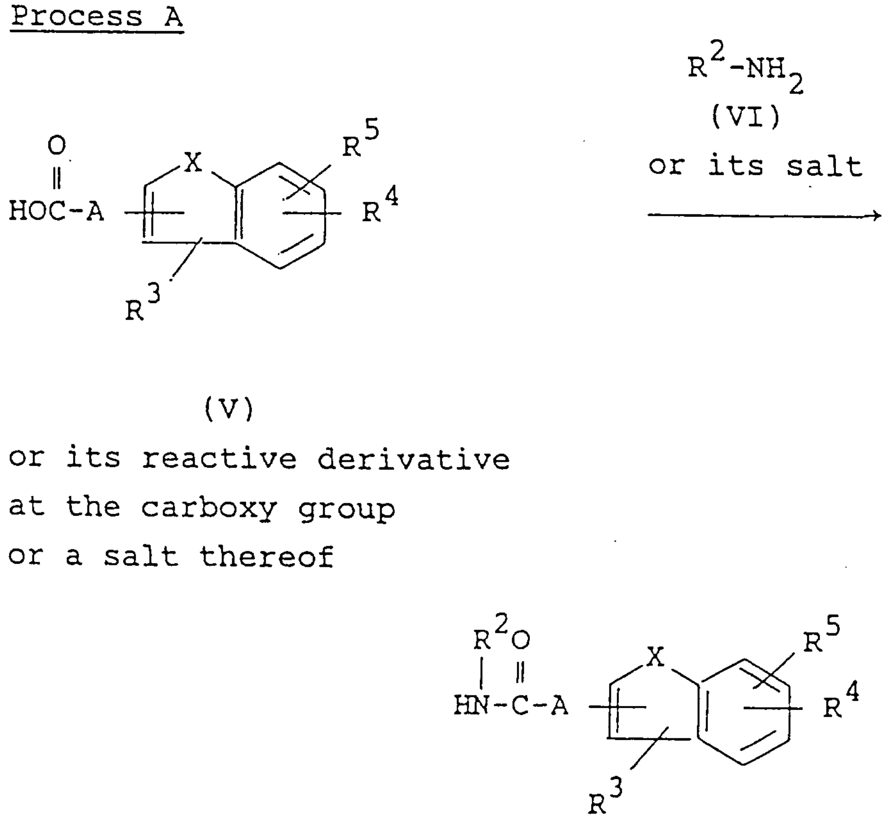


(V)
or its reactive derivative
at the carboxy group
or a salt thereof

(VII)
or its salt

Process B

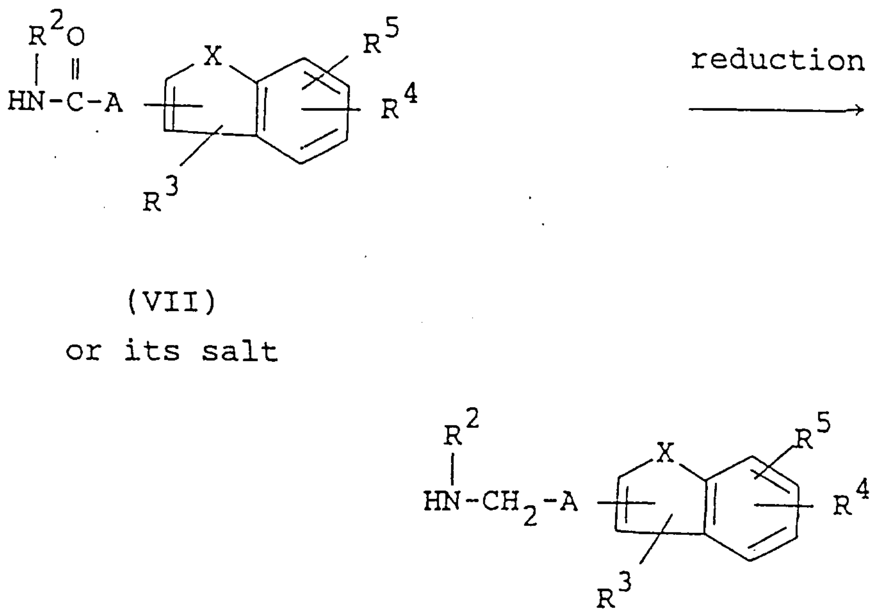


(VII)
or its salt

(III)
or its salt

wherein $R^2$, $R^3$, $R^4$, $R^5$, A and X are each as defined above.

The above-mentioned processes for preparing the starting compound are explained in detail in the following.

Process A

The compound (VII) or its salt can be prepared by reacting a compound (V) or its reactive derivative at the carboxy group or a salt thereof with a compound (VI) or its salt.

Suitable salts of the compounds (V), its reactive derivative and the compounds (VI) and (VII) may be the same as those exemplified for the compound (I).

Suitable reactive derivative of the compound (V) may include an acid halide, an acid anhydride, an activated amide, and an activated ester. The suitable example may be an acid chloride; an acid azide; a mixed acid anhydride with an acid such as substituted phosphoric acid (e.g. dialkylphosphoric acid, phenylphosphoric acid, diphenylphosphoric acid, dibenzylphosphoric acid, and halogenated phosphoric acid), dialkylphosphorous acid, sulfurous acid, thiosulfuric acid, sulfuric acid, sulfonic acid (e.g. methanesulfonic acid), alkylcarbonic acid, aliphatic carboxylic acid (e.g. pivalic acid, pentanoic acid, isopentanoic acid, 2-ethylbutyric acid, and trichloroacetic acid) or aromatic carboxylic acid (e.g. benzoic acid); a symmetrical acid anhydride; an activated amide with imidazole, 4-substituted imidazole, dimethylpyrazole, triazole or tetrazole; or an activated ester (e.g. cyanomethyl ester, methoxymethyl ester, dimethyliminomethyl $[(CH_3)_2N^+=CH-]$ ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, and 8-quinolyl thioester), or an ester with an N-hydroxy compound (e.g. N,N-dimethylhydroxylamine, 1-hydroxy-2-(1H)-pyridone, N-hydroxysuccinimide, N-hydroxyphthalimide, 1-hydroxy-1H-benzotriazole, and 1-hydroxy-6-chloro-1H-benzotriazole), These reactive derivatives can optionally be selected from them according to the kind of the compound (V) to be used.

The reaction is usually carried out in a conventional solvent such as water, an alcohol (e.g. methanol, and ethanol), acetone, dioxane, acetonitrile, chloroform, methylene chloride, ethylene chloride,

tetrahydrofuran, ethyl acetate, N,N-dimethylformamide, pyridine or any other organic solvent which does not adversely influence the reaction. These conventional solvent may also be used in a mixture with water.

When the compound (V) is used in free acid form or its salt form in the reaction, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)-carbodiimide; N,N'-diethylcarbodiimide, N,N'-diisopropylcarbodiimide; N-ethyl-N'-(3-dimethylaminopropyl)-carbodiimide; N,N-carbonylbis-(2-methylimidazole); pentamethyleneketene-N-cyclohexylimine; diphenylketene-N-cyclohexylimine; ethoxyacetylene; 1-alkoxyl-1-chloroethylene; trialkyl phosphite; ethyl polyphosphate; isopropyl polyphosphate; phosphorus oxychloride (phosphoryl chloride); phosphorus trichloride; thionyl chloride; oxalyl chloride; triphenylphosphine; 2-ethyl-7-hydroxybenzisoxazolium salt; 2-ethyl-5-(m-sulfophenyl)isoxazolium hydroxide intra-molecular salt; 1-(p-chlorobenzenesulfonyloxy)-6-chloro-1H-benzotriazole; and so-called Vilsmeier reagent prepared by the reaction of N,N-dimethylformamide with thionyl chloride, phosgene, trichloromethyl chloroformate, and phosphorus oxychloride.

The reaction may also be carried out in the presence of an inorganic or organic base such as an alkali metal bicarbonate, tri($C_1$-$C_6$)alkylamine, pyridine, N-($C_1$-$C_6$)alkylmorpholine, and N,N-di($C_1$-$C_6$)-alkylbenzylamine.

The reaction temperature is not critical and the reaction can be carried out under cooling to heating.

Process B

The compound (III) or its salt can be prepared by reacting a compound (VII) or its salt with a reducing agent.

Suitable salt of the compound (VII) may be the same as those exemplified for the compound (I).

Suitable reducing agent may be diborane, metal hydride [e.g. lithium aluminum hydride], and a combination of metal hydride [e.g. lithium aluminum hydride] and Lewis acid [e.g. aluminum chloride].

The reaction is usually carried out in a conventional solvent such as diethyl ether, tetrahydrofuran or any other organic solvent which does not adversely influence the reaction.

The reaction temperature is not critical, and the reaction can be carried out under cooling to heating.

The compounds obtained by the above process can be isolated and purified by a conventional method such as pulverization, recrystallization, column chromatography, reprecipitation, or the like.

It is to be noted that the compound (I) and the other compounds may include one or more stereoisomers due to asymmetric carbon atom(s), and all of such isomers and mixture thereof are included within the scope of this invention.

The object compounds (I) and pharmaceutically acceptable salts thereof possess a strong inhibitory activity against ACAT, and are useful for the prevention and/or treatment of hypercholesterolemia, hyperlipidemia, atherosclerosis or diseases caused thereby.

In order to illustrate the usefulness of the object compound (I), the pharmacological test data of some representative compounds of the compound (I) are shown in the following.

Test :

Acyl-CoA : cholesterol acyltransferase (ACAT) inhibitory activity

Method :

ACAT activity was measured by the method of Heider et al. described in Journal of Lipid Research, Vol. 24, page 1127 (1983). The enzyme ACAT was prepared from the mucosal microsome fraction of the small intestine of male, 18-week old Japanese white rabbits which had been fed diet containing 2% cholesterol for 8 weeks. The inhibitory activity of compounds were calculated by measuring the amount of the labeled cholesterol ester produced from [$^{14}$C]oleoyl-CoA and endogenous cholesterol as follows. [$^{14}$C]oleoyl-CoA and microsome were incubated with test compounds at 37°C for 5 minutes. The reaction was stopped by the addition of chloroform-methanol (2:1, V/V). Cholesterol ester fraction in the chloroform-methanol extracts was isolated by thin-layer chromatography and was counted their label.

Results

| Example No. (Compound No.) | IC50 (M) |
|---|---|
| 3 - (9) | 7.3 x 10-8 |
| 3 - (10) | 2.4 x 10-8 |
| 3 - (35) | 6.6 x 10-9 |
| 5 - (7) | 8.8 x 10-9 |
| 5 - (15) | 8.9 x 10-9 |
| 6 - (2) | 7.4 x 10-9 |
| 6 - (6) | 8.4 x 10-9 |
| 6 - (7) | 4.4 x 10-9 |

For therapeutic purpose, the compound (I) of the present invention can be used in a form of pharmaceutical preparation containing one of said compounds, as an active ingredient, in admixture with a pharmaceutically acceptable carrier such as an organic or inorganic solid or liquid excipient suitable for oral, parenteral or external (topical) administration. The pharmaceutical preparations may be capsules, tablets, dragees, granules, suppositories, solution, lotion, suspension, emulsion, ointment, and gel. If desired, there may be included in these preparations, auxiliary substances, stabilizing agents, wetting or emulsifying agents, buffers and other commonly used additives.

While the dosage of the compound (I) will vary depending upon the age and condition of the patient, an average single dose of about 0.1 mg, 1 mg, 10 mg, 50 mg, 100 mg, 250 mg, 500 mg and 1000 mg of the compound (I) may be effective for treating the above-mentioned diseases. In general, amounts between 0.1 mg/body and about 1,000 mg/body may be administered per day.

The following Preparations and Examples are given for the purpose of illustrating this invention.

Preparation 1

A mixture of 3-phenyl-2-benzofurancarboxylic acid (1 g) and thionyl chloride (2 ml) was stirred at 80°C for 30 minutes. After cooling excess thionyl chloride was removed under the reduced pressure, and then thionyl chloride was removed with benzene azeotropically to give 3-phenyl-2-benzofurancarbonyl chloride (1.08 g). To a stirred solution of 1-heptylamine (0.5 g) and triethylamine (0.8 ml) in methylene chloride (25 ml) was added a solution of 3-phenyl-2-benzofurancarbonyl chloride (1.08 g) in methylene chloride (5 ml) dropwise at 0°C and the mixture was stirred at ambient temperature for 1 hour. The reaction mixture was washed with 1N aqueous hydrochloric acid, saturated aqueous sodium bicarbonate and water, and dried. Evaporation of solvent gave the residue which was purified by column chromatography. Elution with chloroform gave N-heptyl-3-phenyl-2-benzofurancarboxamide (1.58 g).

$^1$H-NMR ($CDCl_3$, $\delta$) : 0.88 (3H, t, J=7Hz), 1.30 (8H, m), 1.55 (2H, m), 3.40 (2H, q, J=7Hz), 6.53 (1H, t, J=7Hz), 7.31-7.68 ppm (9H, m)

Preparation 2

The following compounds were obtained according to a similar manner to that of Preparation 1.

(1) N-Heptyl-3-phenyl-2-benzo[b]thiophenecarboxamide

$^1$H-NMR ($CDCl_3$, $\delta$) : 0.90 (3H, t, J=7Hz), 1.13-1.26 (10H, m), 3.21 (2H, q, J=7Hz), 5.56 (1H, t, J=7Hz), 7.32-7.61 (8H, m), 7.90 ppm (1H, dd, J=2, 8Hz)

(2) N-Heptyl-5-phenyl-2-benzofurancarboxamide

$^1$H-NMR ($CDCl_3$, $\delta$) : 0.90 (3H, t, J=7Hz), 1.32-1.40 (8H, m), 1.67 (2H, m), 3.51 (2H, q, J=7Hz), 6.65 (1H, t, J=7Hz), 7.32-7.68 (8H, m), 7.86 ppm (1H, d, J=2Hz)

(3) N-Heptyl-2-benzofurancarboxamide

$^1$H-NMR ($CDCl_3$, $\delta$) : 0.89 (3H, t, J=7Hz), 1.30 (8H, br s), 1.62-1.70 (2H, m), 3.49 (2H, q, J=7Hz), 6.64 (1H, br s), 7.27-7.54 (6H, m), 7.69 ppm (1H, d, J=8Hz)

(4) N-Cycloheptyl-3-phenyl-2-benzofurancarboxamide
$^1$H-NMR ($CDCl_3$, δ) : 1.55 (10H, br s), 1.95-2.07 (2H, m), 4.12 (1H, br s), 6.48 (1H, d, J=9Hz), 7.25-7.69 ppm (9H, m)

(5) 3-(4-Chlorophenyl)-N-heptyl-5-methyl-2-benzofurancarboxamide
$^1$H-NMR ($CDCl_3$, δ) : 0.89 (3H, t, J=7Hz), 1.30 (8H, br s), 1.55-1.65 (2H, m), 2.44 (3H, s), 3.43 (2H, q, J=7Hz), 6.62 (1H, br t, J=7Hz), 7.28-7.63 ppm (7H, m)

(6) N-Heptyl-5-isopropyl-3-phenyl-2-benzofurancarboxamide
$^1$H-NMR ($CDCl_3$, δ) : 0.89 (3H, t, J=7Hz), 1.30 (6H, d, J=7Hz), 1.32 (8H, s), 1.53-1.60 (2H, m), 3.00 (1H, sep, J=7Hz), 3.40 (2H, q, J=7Hz), 6.50 (1H, br s), 7.30-7.69 ppm (8H, m)

(7) 3,5-Diphenyl-N-heptyl-2-benzofurancarboxamide
$^1$H-NMR ($CDCl_3$, δ) : 0.90 (3H, t, J=7Hz), 1.30 (8H, br s), 1.50-1.62 (2H, m), 3.42 (2H, q, J=7Hz), 6.53 (1H, br s), 7.33-7.75 ppm (13H, m)

(8) 3-(2-Chlorophenyl)-N-heptyl-2-benzofurancarboxamide
$^1$H-NMR ($CDCl_3$, δ) : 0.88 (3H, t, J=7Hz), 1.30 (8H, br s), 1.50-1.61 (2H, m), 3.40 (2H, q, J=7Hz), 6.51 (1H, br t, J=7Hz), 7.23-7.58 ppm (8H, m)

(9) 3-(4-Chlorophenyl)-N-heptyl-2-benzofurancarboxamide
$^1$H-NMR ($CDCl_3$, δ) : 0.91 (3H, t, J=7Hz), 1.33 (8H, br s), 1.55-1.65 (2H, m), 3.43 (2H, q, J=7Hz), 6.65 (1H, br t, J=7Hz), 7.30-7.64 ppm (8H, m)

(10) N-Heptyl-3-(2-methylphenyl)-2-benzofurancarboxamide
$^1$H-NMR ($CDCl_3$, δ) : 0.88 (3H, t, J=7Hz), 1.23 (8H, br s), 1.39-1.52 (2H, m), 2.19 (3H, s), 3.34 (2H, q, J=7Hz), 6.20 (1H, br t, J=7Hz), 7.21-7.63 ppm (8H, m)

(11) N-Heptyl-3-(3-methylphenyl)-2-benzofurancarboxamide
$^1$H-NMR ($CDCl_3$, δ) : 0.89 (3H, t, J=7Hz), 1.30 (8H, br s), 1.50-1.60 (2H, m), 2.45 (3H, s), 3.40 (2H, q, J=7Hz), 6.46 (1H, br t, J=7Hz), 7.28-7.60 ppm (8H, m)

(12) N-Heptyl-5-methyl-3-(4-methylphenyl)-2-benzofurancarboxamide
$^1$H-NMR ($CDCl_3$, δ) : 0.89 (3H, t, J=7Hz), 1.29 (8H, m), 1.54 (2H, m), 2.41 (3H, s), 2.42 (3H, s), 3.38 (2H, q, J=7Hz), 6.48 (1H, br t, J=7Hz), 7.22-7.54 ppm (7H, m)

(13) N-Heptyl-3-(4-methylphenyl)-2-benzofurancarboxamide
$^1$H-NMR ($CDCl_3$, δ) : 0.88 (3H, t, J=7Hz), 1.30 (8H, m), 1.55 (2H, m), 2.42 (3H, s), 3.40 (2H, q, J=7Hz), 6.51 (1H, br t, J=7Hz), 7.29-7.62 ppm (8H, m)

(14) N-Heptyl-5-isopentyl-2-benzofurancarboxamide
$^1$H-NMR ($CDCl_3$, δ) : 0.89 (3H, t, J=7Hz), 0.94 (6H, d, J=7Hz), 1.29-1.36 (8H, m), 1.50-1.68 (5H, m), 2.71 (2H, t, J=7Hz), 3.47 (2H, q, J=7Hz), 6.62 (1H, br t, J=7Hz), 7.23 (1H, dd, J=2, 8Hz), 7.37-7.45 ppm (3H, m)

(15) N-Benzyl-3-(4-chlorophenyl)-5-methyl-2-benzofurancarboxamide
$^1$H-NMR ($CDCl_3$, δ) : 2.42 (3H, s), 4.61 (2H, d, J=5.5Hz), 6.92 (1H, br t, J=5.5Hz), 7.23-7.40 (8H, m), 7.45 (2H, d, J=8Hz), 7.61 ppm (2H, d, J=8Hz)

(16) 3-(4-Chlorophenyl)-N-cycloheptyl-5-methyl-2-benzofurancarboxamide
$^1$H-NMR ($CDCl_3$, δ) : 1.62 (10H, br s), 1.97-2.08 (2H, m), 2.43 (3H, s), 4.05-4.18 (1H, m), 6.58 (1H, d, J=8Hz), 7.23-7.61 ppm (7H, m)

(17) 3-(4-Chlorophenyl)-5-methyl-N-pentyl-2-benzofurancarboxamide
$^1$H-NMR ($CDCl_3$, δ) : 0.90 (3H, t, J=7Hz), 1.35 (4H, br s), 1.54-1.68 (2H, m), 2.44 (3H, s), 3.42 (2H, q, J=7Hz), 6.63 (1H, br t, J=7Hz), 7.25-7.35 (2H, m), 7.40-7.50 (3H, m), 7.61 ppm (2H, dd, J=9, 2Hz)

(18) 3-(4-Chlorophenyl)-5-methyl-N-nonyl-2-benzofurancarboxamide
$^1$H-NMR ($CDCl_3$, δ) : 0.89 (3H, t, J=7Hz), 1.29 (12H, br s), 1.55-1.65 (2H, m), 2.43 (3H, s), 3.40 (2H, q, J=7Hz), 6.62 (1H, br t, J=7Hz), 7.23-7.63 ppm (7H, m)

(19) 3-(4-Chlorophenyl)-5,7-dimethyl-N-heptyl-2-benzofurancarboxamide
$^1$H-NMR ($CDCl_3$, δ) : 0.86 (3H, t, J=7Hz), 1.32 (8H, br s), 1.58-1.67 (2H, m), 2.39 (3H, s), 2.55 (3H, s), 3.43 (2H, q, J=7Hz), 6.63 (1H, br t, J=7Hz), 7.09 (1H, s), 7.18 (1H, s), 7.45 (2H, d, J=7Hz), 7.60 ppm (2H, d, J=7Hz)

(20) 5-Chloro-N-heptyl-3-phenyl-2-benzofurancarboxamide
$^1$H-NMR ($CDCl_3$, δ) : 0.87 (3H, t, J=7Hz), 1.28 (8H, br s), 1.55 (2H, m), 3.39 (2H, dt, J=5, 7Hz), 6.49 (1H, t, J=5Hz), 7.37-7.63 ppm (8H, m)

(21) N-Heptyl-5-methyl-3-phenyl-2-benzofurancarboxamide
$^1$H-NMR ($CDCl_3$, δ) : 0.90 (3H, t, J=7Hz), 1.28 (8H, br s), 1.52-1.65 (2H, m), 2.44 (3H, s), 3.40 (2H, q, J=7Hz), 6.50 (1H, br s), 7.22-7.68 ppm (8H, m)

Preparation 3

To a suspension of lithium aluminum hydride (0.32 g) in tetrahydrofuran (20 ml) was added aluminum chloride (0.37 g) in some portions at 0 ° C with stirring, and the mixture was stirred at 0 ° C for 15 minutes. To this mixture was added a solution of N-heptyl-3-phenyl-2-benzo[b]thiophenecarboxamide (2.9 g) in tetrahydrofuran (10 ml) dropwise at 0 ° C and the mixture was stirred for 3 hours under reflux. After cooling excess aluminum hydride was destroyed with diethyl ether saturated with water. Inorganic material was filtered off. Solvent was evaporated to leave the residue which was taken up in diethyl ether. The organic solution was washed with 1N aqueous sodium hydroxide, dried and evaporated to afford N-heptyl-(3-phenylbenzo[b]thiophen-2-ylmethyl)amine (2.63 g) as an oil.

$^1$H-NMR ($CDCl_3$, $\delta$) : 0.87 (3H, t, J = 7Hz), 1.24 (8H, s), 1.44 (2H, m), 2.59 (2H, t, J = 7Hz), 4.02 (2H, s), 7.28-7.86 ppm (9H, m)

Preparation 4

A mixture of 4'-chloro-2-hydroxy-5-methylbenzophenone (2.8 g), ethyl bromoacetate (2.1 g) and potassium carbonate (3.5 g) in acetone (30 ml) was stirred for 4 hours under reflux. After cooling potassium carbonate was filtered off. Evaporation of solvent gave the residue which was dissolved in chloroform. The chloroform solution was washed with water and dried. Evaporation of solvent gave the residue which was recrystallized from ethanol to afford ethyl [2-(4-chlorobenzoyl)-4-methylphenoxy]acetate (3.5 g).

$^1$H-NMR ($CDCl_3$, $\delta$) : 1.22 (3H, t, J = 7Hz), 2.32 (3H, s), 4.16 (2H, q, J = 7Hz), 4.49 (2H, s), 6.73 (1H, d, J = 8Hz), 7.25 (2H, d, J = 8Hz), 7.40 (2H, d, J = 8Hz), 7.81 ppm (2H, d, J = 8Hz)

Preparation 5

A mixture of ethyl [2-(4-chlorobenzoyl)-4-methylphenoxy]acetate (3.0 g) and sodium ethoxide (0.64 g) in ethanol (30 ml) was reflXed for 1 hour. To this mixture was added 1N aqueous sodium hydroxide (12 ml) at ambient temperature and the mixture was refluxed for 30 minutes. After cooling ethanol was evaporated to give a residue which was acidified with concentrated hydrochloric acid (6 ml). The resulting crystal was collected by filtration and washed with water. Recrystallization from ethanol gave 3-(4-chlorophenyl)-5-methyl-2-benzofurancarboxylic acid (1.27 g).

$^1$H-NMR ($CDCl_3$, $\delta$) : 7.58-7.30 (7H, m), 2.45 ppm (3H, s)

Preparation 6

To a suspension of lithium aluminum hydride (0.57 g) in tetrahydrofuran (30 ml) was added aluminum chloride (0.67 g) in some portions at 0 ° C with stirring, and the mixture was stirred at 0 ° C for 15 minutes. To this mixture was added a solution of 3-(4-chlorophenyl)-N-heptyl-5-methyl-2-benzofurancarboxamide (4.11 g) in tetrahydrofuran (10 ml) dropwise at 0 ° C and the mixture was stirred for 3 hours under reflux. After cooling excess aluminum hydride was destroyed with diethyl ether saturated with water. Inorganic material was filtered off. Solvent was evaporated to leave the residue which was taken up in diethyl ether. The organic solution was washed with 1N aqueous sodium hydroxide, dried and evaporated to afford a residue which was dissolved in methanol (5 ml). To this solution was added 10% hydrochloric acid - methanol solution (5 ml). Methanol was evaporated to give a residue. Recrystallization from diethyl ether gave N-heptyl-[3-(4-chlorophenyl)-5-methylbenzofuran-2-ylmethyl]amine hydrochloride (3.19 g).

$^1$H-NMR ($CD_3OD$, $\delta$) : 0.91 (3H, t, J = 7Hz), 1.30 (8H, br s), 1.62 (2H, m), 2.45 (3H, s), 3.00 (2H, t, J = 7Hz), 4.47 (2H, s), 7.25-7.62 ppm (7H, m)

Preparation 7

The following compounds were obtained according to a similar manner to that of Preparation 3.

(1) N-Benzyl-[5-chloro-3-(4-chlorophenyl)benzofuran-2-ylmethyl]amine

$^1$H-NMR ($CDCl_3$, $\delta$) : 3.79 (2H, s), 3.95 (2H, s), 7.28-7.51 ppm (12H, m)

(2) N-Benzyl-(3-phenylindol-2-ylmethyl)amine

$^1$H-NMR ($CDCl_3$, $\delta$) : 3.78 (2H, s), 4.11 (2H, s), 7.08-7.45 (13H, m), 7.69 ppm (1H, d, J = 8Hz)

(3) N-Benzyl-[3-(4-chlorophenyl)-6-methylbenzofuran-2-ylmethyl]amine

$^1$H-NMR ($CDCl_3$, $\delta$) : 2.50 (3H, s), 3.79 (2H, s), 3.98 (2H, s), 7.07-7.48 ppm (12H, m)

(4) N-(2-Fluorobenzyl)-[3-(4-chlorophenyl)-5-methylbenzofuran-2-ylmethyl]amine
$^1$H-NMR ($CDCl_3$, δ) : 2.43 (3H, s), 3.85 (2H, s), 3.96 (2H, s), 6.95-7.36 (7H, m), 7.42 ppm (4H, s)
(5) N-(3-Fluorobenzyl)-[3-(4-chlorophenyl)-5-methylbenzofuran-2-ylmethyl]amine
$^1$H-NMR ($CDCl_3$, δ) : 2.43 (3H, s), 3.76 (2H, s), 3.95 (2H, s), 6.88-7.37 (7H, m), 7.42 ppm (4H, s)
(6) N-(4-Fluorobenzyl)-[3-(4-chlorophenyl)-5-methylbenzofuran-2-ylmethyl]amine
$^1$H-NMR ($CDCl_3$, δ) : 2.38 (3H, s), 4.02-4.11 (4H, m), 6.92 (2H, t, J = 8Hz), 7.12 (1H, dd, J = 2, 8Hz), 7.34-7.43 ppm (8H, m).
(7) N-Cyclopropyl-[3-(4-chlorophenyl)-5-methylbenzofuran-2-ylmethyl]amine
$^1$H-NMR ($CDCl_3$, δ) : 0.37-0.42 (4H, m), 2.15 (1H, m), 2.43 (3H, s), 4.00 (2H, s), 7.12 (1H, dd, J = 2, 8Hz), 7.32 (1H, d, J = 2Hz), 7.37 (1H, d, J = 8Hz), 7.46 ppm (4H, s)
(8) N-Furfuryl-[3-(4-bromophenyl)-5-methylbenzofuran-2-ylmethyl]amine
$^1$H-NMR ($CDCl_3$, δ) : 2.43 (3H, s), 3.77 (2H, s), 3.95 (2H, s), 6.04 (1H, d, J = 3Hz), 6.27 (1H, m), 7.13 (1H, dd, J = 2, 8Hz), 7.33-7.42 (3H, m), 7.35 (2H, d, J = 8Hz), 7.60 ppm (2H, d, J = 8Hz)
(9) N-Furfuryl-[3-(4-chlorophenyl)-5-methylbenzofuran-2-ylmethyl]amine
$^1$H-NMR ($CDCl_3$, δ) : 2.43 (3H, s), 3.77 (2H, s), 3.95 (2H, s), 6.03 (1H, d, J = 3Hz), 6.26 (1H, dd, J = 2, 8Hz), 7.32-7.47 ppm (7H, m)
(10) N-Furfuryl-[5-methyl-3-(4-methylphenyl)benzofuran-2-ylmethyl]amine
$^1$H-NMR ($CDCl_3$, δ) : 2.43 (6H, s), 3.77 (2H, s), 3.98 (2H, s), 6.01 (1H, d, J = 3Hz), 6.25 (1H, m), 7.11 (1H, dd, J = 2, 8Hz), 7.29-7.40 ppm (7H, m)
(11) N-Heptyl-[5-methyl-3-(4-propylphenyl)benzofuran-2-ylmethyl]amine
$^1$H-NMR ($CDCl_3$, δ) : 0.87 (3H, t, J = 7Hz), 1.00 (3H, t, J = 7Hz), 1.25 (8H, br s), 1.43 (2H, m), 1.72 (2H, tq, J = 7, 7Hz), 2.42 (3H, s), 2.59 (2H, t, J = 7Hz), 2.65 (2H, t, J = 7Hz), 3.98 (2H, s), 7.09 (1H, dd, J = 2, 8Hz), 7.30 (2H, d, J = 8Hz), 7.38 (1H, d, J = 2Hz), 7.38 (1H, d, J = 8Hz), 7.41 ppm (2H, d, J = 8Hz)
(12) N-Heptyl-(6-phenylbenzofuran-2-ylmethyl)amine
$^1$H-NMR ($CDCl_3$, δ) : 0.90 (3H, t, J = 7Hz), 1.30 (8H, br s), 1.48-1.60 (2H,m), 2.69 (2H, t, J = 7Hz), 3.96 (2H, s), 6.59 (1H, s), 7.3-7.68 ppm (8H, m)
(13) N-Heptyl-(7-phenylbenzofuran-2-ylmethyl)amine
$^1$H-NMR ($CDCl_3$, δ) : 0.89 (3H, t, J = 7Hz), 1.29 (8H, br s), 1.49-1.60 (2H, m), 2.69 (2H, t, J = 7Hz), 3.96 (2H, s), 6.63 (1H, s), 7.24-7.54 (6H, m), 7.84 ppm (2H, dd, J = 2, 8Hz)
(14) N-Heptyl-(3-phenylindol-2-ylmethyl)amine
$^1$H-NMR ($CDCl_3$, δ) : 0.88 (3H, t, J = 7Hz), 1.27 (8H, br s), 1.48 (2H, br t, J = 7Hz), 2.62 (2H, t, J = 7Hz), 4.06 (2H, s), 7.06-7.48 (8H, m), 7.69 (1H, d, J = 8Hz), 8.93 ppm (1H, br s)
(15) N-Phenyl-[3-(4-chlorophenyl)-5-methylbenzofuran-2-ylmethyl]amine
$^1$H-NMR ($CDCl_3$, δ) : 2.42 (3H, s), 4.45 (2H, s), 6.60 (2H, d, J = 8Hz), 6.73 (1H, dd, J = 8, 8Hz), 7.12-7.18 (3H, m), 7.32 (1H, d, J = 2Hz), 7.37 (1H, d, J = 8Hz), 7.43 (2H, d, J = 8Hz), 7.47 ppm (2H, d, J = 8Hz)

Preparation 8

The following compounds were obtained according to a similar manner to that of Preparation 4.
(1) Ethyl [2-(4-bromobenzoyl)-4-methylphenoxy]acetate
$^1$H-NMR ($CDCl_3$, δ) : 1.23 (3H, t, J = 7Hz), 2.33 (3H, s), 4.17 (2H, q, J = 7Hz), 4.49 (2H, s), 6.74 (1H, d, J = 8Hz), 7.24 (2H, d, J = 8Hz), 7.56 (2H, d, J = 8Hz), 7.74 ppm (2H, d, J = 8Hz)
(2) Ethyl [2-(4-chlorobenzoyl)-4-chlorophenoxy]acetate
$^1$H-NMR ($CDCl_3$, δ) : 1.23 (3H, t, J = 7Hz), 4.19 (2H, q, J = 7Hz), 4.52 (2H, s), 6.78 (1H, d, J = 8Hz), 7.40-7.45 (4H, m), 7.81 ppm (2H, d, J = 8Hz)
(3) Ethyl [4-chloro-2-(4-methylbenzoyl)phenoxy]acetate
$^1$H-NMR ($CDCl_3$, δ) : 1.24 (3H, t, J = 7Hz), 2.43 (3H, s), 4.19 (2H, q, J = 7Hz), 4.55 (2H, s), 6.79 (1H, d, J = 7Hz), 7.30-7.39 (4H, m), 7.78 ppm (2H, d, J = 7Hz)
(4) Ethyl [2-(4-chlorobenzoyl)-4,6-dimethylphenoxy]acetate
$^1$H-NMR ($CDCl_3$, δ) : 1.23 (3H, t, J = 7Hz), 2.30 (3H, s), 2.35 (3H, s), 4.16 (2H, q, J = 7Hz), 4.38 (2H, s), 6.97 (1H, s), 7.18 (1H, s), 7.42 (2H, d, J = 7Hz), 7.78 ppm (2H, d, J = 7Hz)
(5) Ethyl [2-(4-chlorobenzoyl)-4-ethylphenoxy]acetate
$^1$H-NMR ($CDCl_3$, δ) : 1.25 (6H, t, J = 7Hz), 2.65 (2H, q, J = 7Hz), 4.18 (2H, q, J = 7Hz), 4.52 (2H, s),

6.78 (1H, d, J=7Hz), 7.25-7.30 (2H, m), 7.40 (2H, d, J=7Hz), 7.83 ppm (2H, d, J=7Hz)

(6) Ethyl [2-(4-chlorobenzoyl)-5-methylphenoxy]acetate

$^1$H-NMR ($CDCl_3$, δ) : 1.25 (3H, t, J=7Hz), 2.42 (3H, s), 4.19 (2H, q, J=7Hz), 4.52 (2H, s), 6.67 (1H, s), 6.93 (1H, d, J=8Hz), 7.35 (1H, d, J=8Hz), 7.40 (2H, d, J=7Hz), 7.82 ppm (2H, d, J=7Hz)

(7) Ethyl [2-(4-chlorobenzoyl)-6-methylphenoxy]acetate

$^1$H-NMR ($CDCl_3$, δ) : 1.22 (3H, t, J=7Hz), 2.40 (3H, s), 4.28 (2H, q, J=7Hz), 4.40 (2H, s), 6.99-7.23 (2H, m), 7.34 - 7.43 (3H, m), 7.78 ppm (2H, d, J=7Hz)

(8) Ethyl [2-(4-fluorobenzoyl)-4-methylphenoxy]acetate

$^1$H-NMR ($CDCl_3$, δ) : 1.23 (3H, t, J=7Hz), 2.33 (3H, s), 4.16 (2H, q, J=7Hz), 4.51 (2H, s), 6.74 (1H, d, J=8Hz), 7.10 (2H, t, J=8Hz), 7.22 (1H, s), 7.23 (1H, d, J=8Hz), 7.90 ppm (2H, dd, J=5, 8Hz)

(9) Ethyl (2-formyl-5-phenylphenoxy)acetate

$^1$H-NMR ($CDCl_3$, δ) : 1.32 (3H, t, J=7Hz), 4.30 (2H, q, J=7Hz), 4.83 (2H, s), 7.05 (1H, d, J=2Hz), 7.39-7.60 (6H, m), 7.95 (1H, d, J=8Hz), 10.59 ppm (1H, s)

(10) Ethyl (2-formyl-6-phenylphenoxy)acetate

$^1$H-NMR ($CDCl_3$, δ) : 1.20 (3H, t, J=7Hz), 4.13 (2H, q, J=7Hz), 4.15 (2H, s), 7.31-7.60 (7H, m), 7.89 (1H, dd, J=2, 8Hz), 10.72 ppm (1H, d, J=2Hz)

Preparation 9

The following compounds were obtained according to a similar manner to that of Preparation 5.

(1) 3-(4-Bromophenyl)-5-methyl-2-benzofurancarboxylic acid

$^1$H-NMR (DMSO-$d_6$, δ) : 2.39 (3H, s), 7.25 (1H, d, J=8Hz), 7.31 (1H, s), 7.55 (3H, d, J=8Hz), 7.64 ppm (2H, d, J=8Hz)

(2) 3-(4-Chlorophenyl)-6-methyl-2-benzofurancarboxylic acid

$^1$H-NMR ($CD_3OD$, δ) : 2.50 (3H, s), 7.10-7.17 (1H, m), 7.49 - 7.60 ppm (6H, m)

(3) 5-Chloro-3-(4-chlorophenyl)-2-benzofurancarboxylic acid

$^1$H-NMR ($CD_3OD$, δ) : 7.40-7.57 ppm (7H, m)

(4) 5-Chloro-3-(4-methylphenyl)-2-benzofurancarboxylic acid

$^1$H-NMR ($CDCl_3$, δ) : 2.20 (3H, s), 7.00-7.38 ppm (7H, m)

(5) 5-Chloro-3-phenyl-2-benzofurancarboxylic acid

$^1$H-NMR ($CD_3OD$, δ) : 7.35-7.53 ppm (8H, m)

(6) 3-(4-Chlorophenyl)-5,7-dimethyl-2-benzofurancarboxylic acid

$^1$H-NMR ($CD_3OD$, δ) : 2.38 (3H, s), 2.53 (3H, s), 7.14 (2H, d, J=7Hz), 7.45-7.57 ppm (4H, m)

(7) 3-(4-Chlorophenyl)-5-ethyl-2-benzofurancarboxylic acid

$^1$H-NMR ($CD_3OD$, δ) : 1.25 (3H, t, J=7Hz), 2.74 (2H, q, J=7Hz), 7.33-7.59 ppm (7H, m)

(8) 3-(4-Chlorophenyl)-7-methyl-2-benzofurancarboxylic acid

$^1$H-NMR ($CD_3OD$, δ) : 2.60 (3H, s), 7.20-7.58 ppm (7H, m)

(9) 3-(4-Fluorophenyl)-5-methyl-2-benzofurancarboxylic acid

$^1$H-NMR ($CD_3OD$, δ) : 2.10 (3H, s), 6.85-7.37 ppm (7H, s)

(10) 6-Phenyl-2-benzofurancarboxylic acid

$^1$H-NMR ($CD_3OD$, δ) : 7.31-7.83 ppm (9H, m)

(11) 7-Phenyl-2-benzofurancarboxylic acid

$^1$H-NMR ($CDCl_3$-$CD_3OD$, δ) : 7.38-7.70 (7H, m), 7.89-7.93 ppm (2H, m)

Preparation 10

The following compounds were obtained according to similar manners those of Preparations 1, 4 and 5.

(1) 3-(3,4-Dichlorophenyl)-N-heptyl-2-benzofurancarboxamide

$^1$H-NMR ($CDCl_3$, δ) : 0.89 (3H, t, J=7Hz), 1.28 (8H, br s), 1.58-1.70 (2H, m), 3.43 (2H, q, J=7Hz), 6.69 (1H, br t, J=7Hz), 7.30-7.38 (1H, m), 7.44-7.60 (5H, m), 7.75 ppm (1H, s)

(2) 3-(3-Chlorophenyl)-N-heptyl-2-benzofurancarboxamide

$^1$H-NMR ($CDCl_3$, δ) : 0.89 (3H, t, J=7Hz), 1.30 (8H, br s), 1.57-1.65 (2H, m), 3.43 (2H, q, J=7Hz), 6.63 (1H, br t, J=7Hz), 7.28-7.66 ppm (8H, m)

(3) 3-(2-Chlorophenyl)-N-heptyl-5-methyl-2-benzofurancarboxamide

$^1$H-NMR ($CDCl_3$, δ) : 0.88 (3H, t, J = 7Hz), 1.30 (8H, br s), 1.50-1.60 (2H, m), 2.52 (3H, s), 3.49 (2H, q, J = 7Hz), 6.49 (1H, br s), 7.13-7.58 ppm (7H, m)

(4) N-Heptyl-3-isopentyl-2-benzofurancarboxamide

$^1$H-NMR ($CDCl_3$, δ) : 0.90 (3H, t, J = 7Hz), 0.99 (6H, d, J = 7Hz), 1.27-1.42 (8H, m), 1.53-1.72 (5H, m), 3.15 (2H, t, J = 7Hz), 3.46 (2H, q, J = 7Hz), 6.65 (1H, br t, J = 7Hz), 7.23-7.47 (3H, m), 7.63 ppm (1H, d, J = 8Hz)

Preparation 11

The following compounds were obtained according to similar manners those of Preparations 4 and 5.

(1) 5-Isopropyl-3-phenyl-2-benzofurancarboxylic acid

$^1$H-NMR ($CDCl_3$, δ) : 1.28 (6H, d, J = 7Hz), 3.00 (1H, sep, J = 7Hz), 7.38-7.62 ppm (8H, m)

(2) 3,5-Diphenyl-2-benzofurancarboxylic acid

$^1$H-NMR ($CDCl_3$, δ) : 7.30-7.79 ppm (13H, m)

(3) 3-(2-Chlorophenyl)-2-benzofurancarboxylic acid

$^1$H-NMR ($CDCl_3$, δ) : 7.30-7.70 ppm (8H, m)

(4) 3-(4-Chlorophenyl)-2-benzofurancarboxylic acid

$^1$H-NMR ($CDCl_3$, δ) : 7.25-7.70 ppm (8H, m)

(5) 3-(2-Methylphenyl)-2-benzofurancarboxylic acid

$^1$H-NMR ($CDCl_3$, δ) : 2.20 (3H, s), 7.27-7.68 ppm (8H, m)

(6) 3-(3-Methylphenyl)-2-benzofurancarboxylic acid

$^1$H-NMR ($CDCl_3$, δ) : 2.47 (3H, s), 7.28-7.67 ppm (8H, m)

(7) 5-Methyl-3-(4-methylphenyl)-2-benzofurancarboxylic acid

$^1$H-NMR ($CDCl_3$, δ) : 2.43 (3H, s), 2.47 (3H, s), 7.28-7.37 (4H, m), 7.45-7.52 ppm (3H, m)

(8) 3-(4-Methylphenyl)-2-benzofurancarboxylic acid

$^1$H-NMR ($CD_3OD$, δ) : 2.32 (3H, s), 7.17-7.52 ppm (8H, m)

(9) 5-Methyl-3-phenyl-2-benzofurancarboxylic acid

$^1$H-NMR ($CDCl_3$, δ) : 2.45 (3H, s), 7.40-7.62 ppm (8H, m)

(10) 5-Methyl-3-(4-propylphenyl)-2-benzofurancarboxylic acid

$^1$H-NMR ($CDCl_3$, δ) : 1.02 (3H, t, J = 7Hz), 1.73 (2H, tq, J = 7, 7Hz), 2.43 (3H, s), 2.68 (2H, t, J = 7Hz), 7.30 (2H, d, J = 8Hz), 7.32 (1H, dd, J = 2, 8Hz), 7.40 (2H, d, J = 1Hz), 7.51 (1H, d, J = 8Hz), 7.51 ppm (2H, d, J = 8Hz)

Preparation 12

The following compounds were obtained according to a similar manner to that of Preparation 6.

(1) N-Benzyl-[3-(4-bromophenyl)-5-methylbenzofuran-2-ylmethyl]aminehydrochloride

$^1$H-NMR ($CDCl_3$, δ) : 2.43 (3H, s), 3.77 (2H, s), 3.95 (2H, s), 7.13 (1H, dd, J = 2, 8Hz), 7.22-7.41 (9H, m), 7.56 ppm (2H, d, J = 8Hz)

(2) N-Benzyl-[3-(4-chlorophenyl)-5-methylbenzofuran-2-ylmethyl]amine hydrochloride

$^1$H-NMR ($CD_3OD$, δ) : 2.43 (3H, s), 4.23 (2H, s), 4.45 (2H, s), 7.26-7.55 ppm (12H, m)

(3) N-Benzyl-[3-(4-fluorophenyl)-5-methylbenzofuran-2-ylmethyl]amine hydrochloride

$^1$H-NMR ($CDCl_3$-$CD_3OD$, δ) : 2.46 (3H, s), 4.12 (2H, s), 4.32 (2H, s), 7.17-7.48 ppm (12H, m)

(4) N-Benzyl-[5-methyl-3-(4-methylphenyl)benzofuran-2-ylmethyl]amine hydrochloride

$^1$H-NMR ($CD_3OD$, δ) : 2.42 (6H, s), 4.22 (2H, s), 4.45 (2H, s), 7.23-7.51 ppm (12H, m)

(5) N-Benzyl-[5-chloro-3-(4-methylphenyl)benzofuran-2-ylmethyl]amine hydrochloride

$^1$H-NMR ($CD_3OD$, δ) : 2.44 (3H, s), 4.24 (2H, s), 4.50 (2H, s), 7.35-7.65 ppm (12H, m)

(6) N-(4-Methylbenzyl)-[3-(4-chlorophenyl)-5-methylbenzofuran-2-ylmethyl]amine hydrochloride

$^1$H-NMR ($CD_3OD$, δ) : 2.37 (3H, s), 2.45 (3H, s), 4.20 (2H, s), 4.43 (2H, s), 7.19-7.30 (5H, m), 7.40-7.55 ppm (6H, m)

(7) N-(2-Phenylethyl)-[3-(4-chlorophenyl)-5-methylbenzofuran-2-ylmethyl]amine hydrochloride

$^1$H-NMR ($CD_3OD$, δ) : 2.43 (3H, s), 3.02 (2H, t, J = 7Hz), 3.20 (2H, t, J = 7Hz), 4.41 (2H, s), 7.16-7.56 ppm (12H, m)

(8) N-(3,4-Dichlorobenzyl)-[3-(4-chlorophenyl)-5-methylbenzofuran-2-ylmethyl]amine hydrochloride

$^1$H-NMR ($CD_3OD$, δ) : 2.45 (3H, s), 4.15 (2H, s), 4.42 (2H, s), 7.24-7.60 ppm (10H, m)

(9) N-Benzyl-(5-chloro-3-phenylbenzofuran-2-ylmethyl)-amine hydrochloride

$^1$H-NMR ($CD_3OD$, δ) : 4.28 (2H, s), 4.50 (2H, s), 7.40-7.67 ppm (13H, m)

(10) N-Cyclobutyl-(5-chloro-3-phenylbenzofuran-2-ylmethyl)amine hydrochloride

$^1$H-NMR ($CD_3OD$, $\delta$) : 1.80-1.93 (2H, m), 2.06-2.21 (4H, m), 3.76 (1H, m), 4.38 (2H, s), 7.42-7.65 ppm (8H, m)

(11) N-Cyclobutyl-[3-(4-chlorophenyl)-5-methylbenzofuran-2-ylmethyl]amine hydrochloride

$^1$H-NMR ($CD_3OD$, $\delta$) : 1.77-1.94 (2H, m), 2.08-2.22 (4H, m), 2.43 (3H, s), 3.75 (1H, m), 4.34 (2H, s), 7.27 (1H, dd, J=2, 8Hz), 7.39 (1H, d, J=2Hz), 7.50 (1H, d, J=8Hz), 7.53-7.62 ppm (4H, m)

(12) N-Cyclohexyl-[3-(4-chlorophenyl)-5-methylbenzofuran-2-ylmethyl]amine hydrochloride

$^1$H-NMR ($CD_3OD$, $\delta$) : 1.22-1.37 (6H, m), 1.80-1.87 (2H, m), 2.00-2.05 (2H, m), 2.43 (3H, s), 3.04 (1H, m), 4.47 (2H, s), 7.28 (1H, dd, J=2, 8Hz), 7.40 (1H, d, J=2Hz), 7.50 (1H, d, J=8Hz), 7.54 (2H, d, J=8Hz), 7.62 ppm (2H, d, J=8Hz)

(13) N-Cyclopentyl-[3-(4-chlorophenyl)-5-methylbenzofuran-2-ylmethyl]amine hydrochloride

$^1$H-NMR ($CDCl_3$, $\delta$) : 1.43 (2H, m), 1.67-1.90 (6H, m), 2.39 (3H, s), 3.35 (1H, m), 4.23 (2H, t, J=5Hz), 7.16 (1H, dd, J=2, 8Hz), 7.26 (1H, d, J=2Hz), 7.42-7.57 ppm (5H, m)

(14) N-Heptyl-(5-chloro-3-phenylbenzofuran-2-ylmethyl)-amine

$^1$H-NMR ($CD_3OD$, $\delta$) : 0.90 (3H, t, J=7Hz), 1.30 (8H, br s), 1.62 (2H, m), 3.02 (2H, t, J=7Hz), 4.51 (2H, s), 7.42-7.66 ppm (8H, m)

## Preparation 13

To a mixture of 4-isopentylanisole (1.0 g) and tin(IV) chloride (1.3 ml) in methylene chloride (15 ml) was added 1,1-dichloromethyl ether (0.97 g) dropwise at 0°C and the mixture was stirred for 2 hours under reflux. After cooling the reaction mixture was poured into ice-cold water. The organic layer was washed with water and dried. Evaporation of solvent gave a residue which was purified by column chromatography on silica gel. Elution with chloroform gave 5-isopentyl-2-methoxybenzaldehyde (0.96 g).

$^1$H-NMR ($CDCl_3$, $\delta$) : 0.92 (6H, d, J=7Hz), 1.42-1.61 (3H, m), 2.58 (2H, t, J=7Hz), 3.91 (3H, s), 6.90 (1H, d, J=8Hz), 7.37 (1H, dd, J=2, 8Hz), 7.63 (1H, d, J=2Hz), 10.45 ppm (1H, s)

## Preparation 14

To a solution of 5-isopentyl-2-methoxybenzaldehyde (0.96 g) in methylene chloride (15 ml) was added boron tribromide (0.9 ml) dropwise at 0°C and the mixture was stirred at ambient temperature for 2 hours. The reaction mixture was poured into water. The organic layer was washed with water and dried. Evaporation of solvent gave a residue which was purified by column chromatography on silica gel. Elution with chloroform gave 2-hydroxy-5-isopentylbenzaldehyde (0.72 g).

$^1$H-NMR ($CDCl_3$, $\delta$) : 0.94 (6H, d, J=7Hz), 1.43-1.67 (3H, m), 2.60 (2H, t, J=7Hz), 6.92 (1H, d, J=8Hz), 7.34-7.38 (2H, m), 9.88 (1H, s), 10.85 ppm (1H, s)

## Preparation 15

A mixture of 2-hydroxy-5-isopentylbenzaldehyde (0.72 g), diethyl bromomalonate (0.95 g) and potassium carbonate (1.1 g) in 2-butanone (10 ml) was stirred under reflux for 10 hours. After cooling potassium carbonate was filtered off, solvent was evaporated to give a residue which was taken up in diethyl ether and water. The organic layer was washed with water and dried. Evaporation of solvent gave a residue which was chromatographed on silica gel. Elution with chloroform gave ethyl 5-isopentyl-2-benzofurancarboxylate (0.46 g).

$^1$H-NMR ($CDCl_3$, $\delta$) : 0.94 (6H, d, J=7Hz), 1.42 (3H, t, J=7Hz), 1.51-1.64 (3H, m), 2.71 (2H, t, J=7Hz), 4.44 (2H, q, J=7Hz), 7.27 (1H, dd, J=2, 8Hz), 7.46-7.51 ppm (3H, m)

## Preparation 16

A mixture of ethyl 5-isopentyl-2-benzofurancarboxylate (0.46 g) and 1N aqueous sodium hydroxide in methanol (5 ml) was stirred at 60°C for 1 hour. After cooling methanol was evaporated to leave a residue which was acidified with 1N hydrochloric acid and extracted with ethyl acetate, the extract was washed with water and dried. Evaporation of solvent gave 5-isopentyl-2-benzofurancarboxylic acid (0.36 g).

$^1$H-NMR ($CD_3OD$, $\delta$) : 0.95 (6H, d, J=7Hz), 1.49-1.65 (3H, m), 2.70 (2H, t, J=7Hz), 7.19 (1H, dd,

J = 2, 8Hz), 7.25 (1H, s), 7.39-7.42 (2H, m), 7.54 ppm (1H, s)

Preparation 17

To a stirred mixture of 5-methyl-3-(4-methylphenyl)-2-benzofurancarboxylic acid (456 mg) and N,N-dimethylformamide (1 drop) in methylene chloride (10 ml) was added oxalyl chloride (0.3 ml) at ambient temperature and the mixture was stirred at the same temperature for 2 hours. Oxalyl chloride was removed under reduced pressure to give 5-methyl-3-(4-methylphenyl)-2-benzofurancarbonyl chloride, which was dissolved in methylene chloride (10 ml). To this stirred solution was added triethylamine (0.5 ml) and then benzylamine (220 mg) dropwise at 0°C and the mixture was stirred at the same temperature for 15 minutes. The reaction mixture was washed with dilute hydrochloric acid and dilute aqueous sodium bicarbonate, and dried. Evaporation of solvent followed by recrystallization from n-hexane-ethyl acetate gave N-benzyl-5-methyl-3-(4-methylphenyl)-2-benzofurancarboxamide (0.55 g).

$^1$H-NMR ($CDCl_3$, $\delta$) : 2.42 (6H, s), 4.60 (2H, d, J = 5Hz), 6.80 (1H, br t, J = 5Hz), 7.34-7.55 ppm (12H, m)

Preparation 18

The following compounds were obtained according to a similar manner to that of Preparation 17.

(1) N-Benzyl-3-(4-bromophenyl)-5-methyl-2-benzofurancarboxamide

$^1$H-NMR ($CDCl_3$, $\delta$) : 2.43 (3H, s), 4.62 (2H, d, J = 5Hz), 6.93 (1H, t, J = 5Hz), 7.24-7.40 (8H, m), 7.54 (2H, d, J = 8Hz), 7.62 ppm (2H, d, J = 8Hz)

(2) N-Benzyl-5-chloro-3-(4-chlorophenyl)-2-benzofurancarboxamide

$^1$H-NMR ($CDCl_3$, $\delta$) : 4.61 (2H, d, J = 5Hz), 6.90 (1H, br t, J = 5Hz), 7.45-7.61 ppm (12H, m)

(3) N-Benzyl-3-(4-chlorophenyl)-6-methyl-2-benzofurancarboxamide

$^1$H-NMR ($CDCl_3$, $\delta$) : 2.50 (3H, s), 4.63 (2H, d, J = 7Hz), 6.93 (1H, br t, J = 7Hz), 7.12-7.50 (10H, m), 7.64 ppm (2H, d, J = 8Hz)

(4) N-Benzyl-3-(4-chlorophenyl)-7-methyl-2-benzofurancarboxamide

$^1$H-NMR ($CDCl_3$, $\delta$) : 2.47 (3H, s), 4.66(2H, d, J = 7Hz), 6.95 (1H, br t, J = 7Hz), 7.21-7.39 (8H, m), 7.45 (2H, d, J = 8Hz), 7.64 ppm (2H, d, J = 8Hz)

(5) N-Benzyl-5-chloro-3-(4-methylphenyl)-2-benzofurancarboxamide

$^1$H-NMR ($CDCl_3$, $\delta$) : 2.43 (3H, s), 4.60 (2H, d, J = 7Hz), 6.79 (1H, br t, J = 7Hz), 7.28-7.58 ppm (12H, m)

(6) N-Benzyl-3-(4-fluorophenyl)-5-methyl-2-benzofurancarboxamide

$^1$H-NMR ($CDCl_3$, $\delta$) : 2.43 (3H, s), 4.61 (2H, d, J = 5Hz), 6.90 (1H, br t, J = 5Hz), 7.13-7.41 (10H, m), 7.65 ppm (2H, dd, J = 5, 8Hz)

(7) 5-Chloro-N-furfuryl-3-(4-methylphenyl)-2-benzofurancarboxamide

$^1$H-NMR ($CDCl_3$, $\delta$) : 2.45 (3H, s), 4.61 (2H, d, J = 7Hz), 6.26-6.29 (1H, m), 6.32-6.35 (1H, m), 6.82 (1H, br t, J = 7Hz), 7.28-7.59 ppm (8H, m)

(8) 3-(4-Chlorophenyl)-N-(3-fluorobenzyl)-5-methyl-2-benzofurancarboxamide

$^1$H-NMR($CDCl_3$, $\delta$) : 2.44 (3H, s), 4.62 (2H, d, J = 5Hz), 6.95-7.42 (8H, m), 7.45 (2H, d, J = 8Hz), 7.62 ppm (2H, d, J = 8Hz)

(9) 3-(4-Chlorophenyl)-N-(4-methoxybenzyl)-5-methyl-2-benzofurancarboxamide

$^1$H-NMR ($CDCl_3$, $\delta$) : 2.45 (3H, s), 3.80 (3H, s), 4.55 (2H, d, J = 7Hz), 6.83-6.90 (3H, m), 7.25-7.40 (4H, m), 7.45 (2H, d, J = 7Hz), 7.61 ppm (2H, d, J = 7Hz)

(10) 3-(4-Chlorophenyl)-5-methyl-N-(4-methylbenzyl)-2-benzofurancarboxamide

$^1$H-NMR ($CDCl_3$, $\delta$) : 2.38 (3H, s), 2.44 (3H, s), 4.58 (2H, d, J = 5Hz), 6.88 (1H, br t, J = 7Hz), 7.15-7.63 ppm (11H, m)

(11) N-(4-Chlorobenzyl)-3-(4-chlorophenyl)-5-methyl-2-benzofurancarboxamide

$^1$H-NMR ($CDCl_3$, $\delta$) : 2.47 (3H, s), 4.60 (2H, d, J = 7Hz), 6.95 (1H, br t, J = 7Hz), 7.30-7.65 ppm (11H, m)

(12) 3-(4-Chlorophenyl)-N-[2-(1-cyclohexenyl)ethyl]-5-methyl-2-benzofurancarboxamide

$^1$H-NMR ($CDCl_3$, $\delta$) : 1.59 (6H, s), 1.95 (2H, br s), 2.22 (2H, t, J = 7Hz), 2.45 (3H, s), 3.49 (2H, q, J = 7 Hz), 5.50 (1H, s), 6.59 (1H, t, J = 7Hz), 7.28-7.60 ppm (7H, m)

(13) 3-(4-Chlorophenyl)-N-(4-fluorobenzyl)-5-methyl-2-benzofurancarboxamide

$^1$H-NMR ($CDCl_3$, $\delta$) : 2.43 (3H, s), 4.58 (2H, d, J = 5Hz), 6.93 (1H, t, J = 5Hz), 7.03 (2H, t, J = 8Hz), 7.29-7.42 (5H, m), 7.45 (2H, d, J = 8Hz), 7.62 ppm (2H, d, J = 8Hz)

(14) 3-(4-Chlorophenyl)-N-(3-furylmethyl)-5-methyl-2-benzofurancarboxamide
$^1$H-NMR ($CDCl_3$, $\delta$) : 2.45 (3H, s), 4.47 (2H, d, J = 5Hz), 6.43 (1H, s), 6.79 (1H, br t, J = 5Hz), 7.28-7.48 (7H, m), 7.61 ppm (2H, d, J = 8Hz)

(15) 3-(4-Chlorophenyl)-5-methyl-N-phenyl-2-benzofurancarboxamide
$^1$H-NMR ($CDCl_3$, $\delta$) : 2.45 (3H, s), 7.14 (1H, dd, J = 8, 8Hz), 7.32-7.66 (11H, m), 8.37 ppm (1H, s)

(16) 3-(4-Chlorophenyl)-5-methyl-N-(2-phenylethyl)-2-benzofurancarboxamide
$^1$H-NMR ($CDCl_3$, $\delta$) : 2.42 (3H, s), 2.92 (2H, t, J = 7Hz), 3.68 (2H, dt, J = 7, 5Hz), 6.67 (1H, t, J = 5Hz), 7.20-7.41 (8H, m), 7.43 (2H, d, J = 8Hz), 7.57 ppm (2H, d, J = 8Hz)

(17) 3-(4-Chlorophenyl)-5-methyl-N-(3-phenylpropyl)-2-benzofurancarboxamide
$^1$H-NMR ($CDCl_3$, $\delta$) : 1.95 (2H, q, J = 7Hz), 2.43 (3H, s), 2.70 (2H, t, J = 7Hz), 3.48 (2H, q, J = 7Hz), 6.63 (1H, br t, J = 7Hz), 7.21-7.62 ppm (12H, m)

(18) 3-(4-Chlorophenyl)-N-(3,4-dichlorobenzyl)-5-methyl-2-benzofurancarboxamide
$^1$H-NMR ($CDCl_3$, $\delta$) : 2.43 (3H, s), 4.55 (2H, d, J = 5Hz), 7.00 (1H, t, J = 5Hz), 7.19 (1H, dd, J = 2, 8Hz), 7.31 (1H, d, J = 2Hz), 7.35-7.44 (4H, m), 7.47 (2H, d, J = 8Hz), 7.61 ppm (2H, d, J = 8Hz)

(19) N-Benzyl-5-chloro-3-phenyl-2-benzofurancarboxamide
$^1$H-NMR ($CDCl_3$, $\delta$) : 4.62 (2H, d, J = 7Hz), 6.82 (1H, br t, J = 7Hz), 7.33-7.65 ppm (13H, m)

(20) 5-Chloro-N-cyclobutyl-3-phenyl-2-benzofurancarboxamide
$^1$H-NMR ($CDCl_3$, $\delta$) : 1.68-2.04 (4H, m), 2.32-2.46 (2H, m), 4.55 (1H, m), 7.37-7.62 ppm (8H, m)

(21) 3-(4-Chlorophenyl)-5-methyl-2-benzofurancarboxamide
$^1$H-NMR ($CDCl_3$, $\delta$) : 5.72 (1H, br s), 6.45 (1H, br s), 7.27-7.47 (3H, m), 7.48 (2H, d, J = 8Hz), 7.60 ppm (2H, d, J = 8Hz)

(22) 3-(4-Chlorophenyl)-N-cyclobutyl-5-methyl-2-benzofurancarboxamide
$^1$H-NMR ($CDCl_3$, $\delta$) : 1.67-1.84 (2H, m), 1.90-2.10 (2H, m), 2.33-2.48 (2H, m), 2.42 (3H, s), 4.53 (1H, m), 6.75 (1H, d, J = 8Hz), 7.30-7.45 (3H, m), 7.44 (2H, d, J = 9Hz), 7.59 ppm (2H, d, J = 9Hz)

(23) 3-(4-chlorophenyl)-N-cyclopropyl-5-methyl-2-benzofurancarboxamide
$^1$H-NMR ($CDCl_3$, $\delta$) : 0.60-0.68 (2H, m), 0.82-0.92 (2H, m), 2.43 (3H, s), 2.86 (1H, m), 6.73 (1H, d, J = 3Hz), 7.28-7.42 (3H, m), 7.46 (2H, d, J = 8Hz), 7.62 ppm (2H, d, J = 8Hz)

(24) 3-(4-chlorophenyl)-N-cyclohexyl-5-methyl-2-benzofurancarboxamide
$^1$H-NMR ($CDCl_3$, $\delta$) : 1.16-1.50 (6H, m), 1.62-1.80 (2H, m), 1.97-2.03 (2H, m), 2.43 (3H, s), 3.93 (1H, m), 6.50 (1H, d, J = 8Hz), 7.24-7.41 (3H, m), 7.44 (2H, d, J = 8Hz), 7.60 ppm (2H, d, J = 8Hz)

(25) 3-(4-chlorophenyl)-N-cyclopentyl-5-methyl-2-benzofurancarboxamide
$^1$H-NMR ($CDCl_3$, $\delta$) : 1.46-1.77 (6H, m), 1.98-2.14 (2H, m), 2.43 (3H, s), 4.35 (1H, m), 6.54 (1H, d, J = 7Hz), 7.27 (1H, dd, J = 2, 8Hz), 7.33 (1H, d, J = 2Hz), 7.42 (1H, d, J = 8Hz), 7.45 (2H, d, J = 8Hz), 7.61 ppm (2H, d, J = 8Hz)

(26) 3-(4-Chlorophenyl)-N-cyclohexylmethyl-5-methyl-2-benzofurancarboxamide
$^1$H-NMR ($CDCl_3$, $\delta$) : 0.85-1.05 (2H, m), 1.10-1.28 (3H, m), 1.65-1.80 (6H, m), 2.48 (3H, s), 3.28 (2H, t, J = 7Hz), 6.69 (1H, br t, J = 7Hz), 7.30-7.50 (5H, m), 7.60 ppm (2H, d, J = 7Hz)

(27) 5-Chloro-N-cyclopentyl-3-phenyl-2-benzofurancarboxamide
$^1$H-NMR ($CDCl_3$, $\delta$) : 1.38-1.52 (2H, m), 1.58-1.70 (4H, m), 1.94-2.09 (2H, m), 4.37 (1H, sext, J = 7Hz), 6.49 (1H, br d, J = 7Hz), 7.38-7.62 ppm (8H, m)

(28) 3-(4-Chlorophenyl)-5-methyl-N-propyl-2-benzofurancarboxamide
$^1$H-NMR ($CDCl_3$, $\delta$) : 0.98 (3H, t, J = 7Hz), 1.62 (2H, sext, J = 7Hz), 2.43 (3H, s), 3.39 (2H, q, J = 7Hz), 6.67 (1H, br t, J = 7Hz), 7.27-7.40 (3H, m), 7.47 (2H, d, J = 7Hz), 7.60 ppm (2H, d, J = 7Hz)

(29) 3-(4-Chlorophenyl)-5-ethyl-N-heptyl-2-benzofurancarboxamide
$^1$H-NMR ($CDCl_3$, $\delta$) : 0.89 (3H, t, J = 7Hz), 1.30 (11H, br s), 1.55-1.65 (2H, m), 2.74 (2H, q, J = 7Hz), 3.42 (2H, q, J = 7Hz), 6.62 (1H, br t, J = 7Hz), 7.28-7.63 ppm (7H, m)

(30) N-(2-Fluorobenzyl)-3-(4-chlorophenyl)-5-methyl-2-benzofurancarboxamide
$^1$H-NMR ($CDCl_3$, $\delta$) : 2.43 (3H, s), 4.67 (2H, d, J = 5Hz), 6.93 (1H, t, J = 5Hz), 7.12 (2H, t, J = 8Hz), 7.24-7.40 (5H, m), 7.46 (2H, d, J = 8Hz), 7.60 ppm (2H, d, J = 8Hz)

(31) N-Furfuryl-3-(4-bromophenyl)-5-methyl-2-benzofurancarboxamide
$^1$H-NMR ($CDCl_3$, $\delta$) : 2.43 (3H, s), 4.61 (2H, d, J = 5Hz), 6.28-6.35 (2H, m), 6.92 (1H, t, J = 5Hz), 7.29-7.43 (4H, m), 7.53 (2H, d, J = 8Hz), 7.62 ppm (2H, d, J = 8Hz)

(32) 3-(4-Fluorophenyl)-N-furfuryl-5-methyl-2-benzofurancarboxamide
$^1$H-NMR ($CDCl_3$, δ) : 2.42 (3H, s), 4.60 (2H, d, J = 7Hz), 6.28-6.36 (2H, m), 6.91 (1H, br t, J = 7Hz), 7.12-7.42 (6H, m), 7.58-7.69 ppm (2H, m)

(33) N-Furfuryl-3-(4-chlorophenyl)-5-methyl-2-benzofurancarboxamide
$^1$H-NMR ($CDCl_3$, δ) : 2.43 (3H, s), 4.62 (2H, d, J = 6Hz), 6.32 (2H, m), 6.92 (1H, t, J = 6Hz), 7.28-7.43 (4H, m), 7.45 (2H, d, J = 8Hz), 7.60 ppm (2H, d, J = 8Hz)

(34) N-Furfuryl-5-methyl-3-(4-methylphenyl)-2-benzofurancarboxamide
$^1$H-NMR ($CDCl_3$, δ) : 2.42 (6H, s), 4.59 (2H, d, J = 5Hz), 6.27 (1H, d, J = 3Hz), 6.33 (1H, m), 6.83 (1H, t, J = 5Hz), 7.23-7.43 (6H, m), 7.52 ppm (2H, d, J = 8Hz)

(35) N-Heptyl-6-phenylbenzofuran-2-carboxamide
$^1$H-NMR ($CDCl_3$, δ) : 0.90 (3H, t, J = 7Hz), 1.30 (8H, br s) 1.60-1.75 (2H, m), 3.50 (2H, q, J = 7Hz), 6.65 (1H, br t, J = 7Hz), 7.33-7.77 ppm (9H, m)

(36) N-Heptyl-7-phenyl-2-benzofurancarboxamide
$^1$H-NMR ($CDCl_3$, δ) : 0.90 (3H, t, J = 7Hz), 1.28-1.42 (8H, m), 1.55-1.67 (2H, m), 3.47 (2H, g, J = 7Hz), 6.55 (1H, br t, J = 7Hz), 7.35-7.83 ppm (9H, m)

(37) N-Heptyl-3-phenylindole-2-carboxamide
$^1$H-NMR ($CDCl_3$, δ) : 0.88 (3H, t, J = 7Hz), 1.06-1.37 (10H, m), 3.29 (2H, q, J = 7Hz), 5.94 (1H, t, J = 7Hz), 7.12 (1H, t, J = 8Hz), 7.33 (1H, t, J = 8Hz), 7.43-7.55 (7H, m), 9.45 ppm (1H, s)

(38) 5-Methyl-N-heptyl-3-(4-propylphenyl)-2-benzofurancarboxamide
$^1$H-NMR ($CDCl_3$, δ) : 0.89 (3H, t, J = 7Hz), 1.00 (3H, t, J = 7Hz), 1.29 (8H, br s), 1.54 (2H, m), 1.72 (2H, tq, J = 7, 7Hz), 2.42 (3H, s), 2.64 (2H, t, J = 7Hz), 3.38 (2H, dt, J = 7, 5Hz), 6.46 (1H, t, J = 5Hz), 7.24 (1H, dd, J = 2, 8Hz), 7.31 (2H, d, J = 8Hz), 7.35 (1H, d, J = 2Hz), 7.42 (1H, d, J = 8Hz), 7.54 ppm (2H, d, J = 8Hz)

Preparation 19

To a stirred solution of 3-phenylindole-2-carboxylic acid (0.5 g) and benzylamine (0.23 g) in methylene chloride (10 ml) was added N-ethyl-N'-(3-dimethylaminopropyl)-carbodiimide hydrochloride (0.45 g) in some portions at 0 °C and the mixture was stirred at ambient temperature for 2 hours. The reaction mixture was washed with 3% aqueous hydrochloric acid (10 ml) and 3% aqueous sodium bicarbonate (10 ml), and dried. Evaporation of solvent gave a residue which was recrystallized from n-hexane-ethyl acetate to provide N-benzyl-3-phenylindole-2-carboxamide (0.52 g).

$^1$H-NMR ($CDCl_3$, δ) : 4.51 (2H, d, J = 5.5Hz), 6.30 (1H, t, J = 5.5Hz), 7.10-7.48 ppm (14H m)

Example 1

To a stirred solution of N-heptyl-(3-phenyl-2-benzo[b]thiophen-2-ylmethyl)amine (2.60 g) in chloroform (30 ml) was added 2,4-difluorophenyl isocyanate (1.25 g) dropwise at 0 °C and the mixture was stirred at ambient temperature for 1 hour. The solvent was evaporated to leave the residue which was chromatographed on silica gel. Elution with chloroform gave N-(2,4-difluorophenyl)-N'-heptyl-N'-(3-phenylbenzo[b]-thiophen-2-ylmethyl)urea (4.10 g) as an oil.

$^1$H-NMR ($CDCl_3$, δ) : 0.85 (3H, t, J = 7Hz), 1.22 (8H, m), 1.48 (2H, m), 3.20 (2H, t, J = 7Hz), 4.79 (2H, s), 6.40 (1H, d, J = 3Hz), 6.74-6.88 (2H, m), 7.31-7.53 (8H, m), 7.83 (1H, m), 8.05 ppm (1H, m)

Example 2

To a stirred suspension of lithium aluminum hydride (0.27 g) and aluminum chloride (0.32 g) in tetrahydrofuran (20 ml) was added a solution of N-heptyl-3-phenyl-2-benzofurancarboxamide (1.98 g) in tetrahydrofuran (10 ml) dropwise at 0 °C and the mixture was refluxed for 3 hours. After cooling excess aluminum hydride was destroyed with diethyl ether saturated with water and filtered off. The organic solution was condensed to afford the residue which was taken up in diethyl ether and 1N aqueous sodium hydroxide. The organic solution was dried and evaporated to give N-heptyl-(3-phenylbenzofuran-2-ylmethyl)amine (1.89 g).

To a solution of N-heptyl-(3-phenylbenzofuran-2-ylmethyl)amine (1.89 g) in chloroform (30 ml) was added 2,4-difluorophenyl isocyanate (0.92 g) dropwise at 0 °C and the mixture was stirred at ambient temperature for 1 hour. Evaporation of solvent gave a residue which was chromatographed on silica gel.

Elution with chloroform provided N-(2,4-difluorophenyl)-N'-heptyl-N'-(3-phenylbenzofuran-2-ylmethyl)urea (2.4 g).

$^1$H-NMR ($CDCl_3$, $\delta$) : 0.84 (3H, t, J = 7Hz), 1.15 (8H, m), 1.43 (2H, m), 3.22 (2H, t, J = 7Hz), 4.77 (2H, s), 6.80-6.89 (2H, m), 6.98 (1H, d, J = 3Hz), 7.23-7.60 (9H, m), 7.99 ppm (1H, m)

Example 3

The following compounds were obtained according to a similar manner to that of Example 2.

(1) N-Benzyl-N-[3-(4-chlorophenyl)-7-methylbenzofuran-2-ylmethyl]-N'-(2,4,6-trifluorophenyl)urea mp : 164-165.5 ° C

$^1$H-NMR ($CDCl_3$, $\delta$) : 2.58 (3H, s), 4.42 (2H, s), 4.70 (2H, s), 6.48 (1H, br s), 6.75 (2H, t, J = 7Hz), 6.99-7.04 (2H, m), 7.19-7.27 (6H, m), 7.37 (2H, d, J = 8Hz), 7.48 ppm (2H, d, J = 8Hz)

(2) N-[3-(4-Chlorophenyl)-5-methylbenzofuran-2-ylmethyl]-N-(3-furylmethyl)-N'-(2,4,6-trifluorophenyl)urea mp : 173-175 ° C

$^1$H-NMR ($CDCl_3$, $\delta$) : 2.46 (3H, s), 4.27 (2H, s), 4.69 (2H, s), 6.25 (1H, s), 6.59 (1H, s), 6.74 (2H, t, J = 8Hz), 6.99 (1H, s), 7.18-7.52 ppm (8H, m)

(3) N-[3-(4-Chlorophenyl)-5-methylbenzofuran-2-ylmethyl]-N-[2-(1-cyclohexenyl)ethyl]-N'-(2,4,6-trifluorophenyl)urea mp : 169.5-170.5 ° C

$^1$H-NMR ($CDCl_3$, $\delta$) : 1.50 (4H, br s), 1.75 (2H, br s), 1.90 (2H, br s), 2.08 (2H, t, J = 7Hz), 2.45 (3H, s), 3.29 (2H, t, J = 7Hz), 4.75 (2H, s), 5.30 (1H, s), 6.45 (1H, br s), 6.72 (2H, t, J = 7Hz), 7.28-7.51 ppm (7H, m)

(4) N-[5-Chloro-3-(4-methylphenyl)benzofuran-2-ylmethyl]-N-furfuryl-N'-(2,4,6-trifluorophenyl)urea mp : 151-152.5 ° C

$^1$H-NMR ($CDCl_3$, $\delta$) : 2.44 (3H, s), 4.43 (2H, s), 4.80 (2H, s), 5.85 (1H, d, J = 2Hz), 6.20-6.24 (1H, m), 6.62 (1H, s), 6.72 (2H, t, J = 8Hz), 7.28-7.46 (7H, m), 7.51 ppm (1H, d, J = 2Hz)

(5) N-[3-(4-Fluorophenyl)-5-methylbenzofuran-2-ylmethyl]-N-furfuryl-N'-(2,4,6-trifluorophenyl)urea mp : 158.5-161 ° C

$^1$H-NMR ($CDCl_3$, $\delta$) : 2.44 (3H, s), 4.42 (2H, s), 4.78 (2H, s), 5.90 (1H, d, J = 3Hz), 6.23 (1H, dd, J = 3, 1Hz), 6.72 (1H, s), 6.73 (2H, d, J = 7Hz), 7.16-7.50 ppm (8H, m)

(6) N-[3-(4-Chlorophenyl)-5-methylbenzofuran-2-ylmethyl]-N-(3-phenylpropyl)-N'-(2,4,6-trifluorophenyl)-urea mp : 84-86 ° C

$^1$H-NMR ($CDCl_3$, $\delta$) : 1.79 (2H, qui, J = 7Hz), 2.47 (3H, s), 2.50 (2H, t, J = 7Hz), 3.30 (2H, t, J = 7Hz), 4.73 (2H, s), 6.39 (1H, br s), 6.73 (2H, t, J = 7Hz), 6.99-7.06 (2H, m), 7.15-7.50 ppm (10H, m)

(7) N-Heptyl-N-(3-phenylbenzofuran-2-ylmethyl)-N'-(2,4,6-trifluorophenyl)urea

$^1$H-NMR ($CDCl_3$, $\delta$) : 0.83 (3H, t, J = 7Hz), 1.17 (8H, s), 1.43 (2H, m), 3.23 (2H, t, J = 7Hz), 4.77 (2H, s), 6.45 (1H, s), 6.72 (2H, t, J = 8Hz), 7.28-7.62 ppm (9H, m)

(8) N-Heptyl-N-(5-phenylbenzofuran-2-ylmethyl)-N'-(2,4,6-trifluorophenyl)urea

$^1$H-NMR ($CDCl_3$, $\delta$) : 0.87 (3H, t, J = 7Hz), 1.28-1.35 (8H, m), 1.67 (2H, m), 3.44 (2H, t, J = 7Hz), 4.69 (2H, s), 6.32 (1H, s), 6.73 (2H, t, J = 8Hz), 7.32-7.75 ppm (9H, m)

(9) N-(Benzofuran-2-ylmethyl)-N-heptyl-N'-(2,4,6-trifluorophenyl)urea

$^1$H-NMR ($CDCl_3$, $\delta$) : 0.87 (3H, t, J = 7Hz), 1.30 (8H, br s), 1.58-1.70 (2H, m), 3.44 (2H, t, J = 7Hz), 4.68 (2H, s), 6.34 (1H, s), 6.71 (2H, t, J = 7Hz), 7.20-7.34 (3H, m), 7.45-7.58 ppm (2H, m)

MASS (m/z) : 419 (M +)

(10) N-Cycloheptyl-N-(3-phenylbenzofuran-2-ylmethyl)-N'-(2,4,6-trifluorophenyl)urea

$^1$H-NMR ($CDCl_3$, $\delta$) : 1.39-1.50 (10H, m), 1.67-1.78 (2H, m), 4.10 (1H, br s), 4.75 (2H, s), 6.72 (2H, t, J = 7Hz), 6.90 (1H, s), 7.29-7.63 ppm (9H, m)

(11) N-[3-(3,4-Dichlorophenyl)benzofuran-2-ylmethyl]-N-heptyl-N'-(2,4,6-trifluorophenyl)urea

$^1$H-NMR ($CDCl_3$, $\delta$) : 0.85 (3H, t, J = 7Hz), 1.19 (8H, br s), 1.45-1.54 (2H, m), 3.28 (2H, t, J = 7Hz), 4.78 (2H, s), 6.33 (1H, s), 6.74 (2H, t, J = 7Hz), 7.30-7.42 (3H, m), 7.52-7.63 ppm (4H, m)

(12) N-[3-(3-Chlorophenyl)benzofuran-2-ylmethyl]-N-heptyl-N'-(2,4,6-trifluorophenyl)urea

$^1$H-NMR ($CDCl_3$, $\delta$) : 0.85 (3H, t, J = 7Hz), 1.18 (8H, br s), 1.40-1.53 (2H, m), 3.27 (2H, t, J = 7Hz), 4.30 (2H, s), 6.39 (1H, s), 6.75 (2H, t, J = 7Hz), 7.30-7.60 ppm (8H, m)

(13) N-Heptyl-N-(5-isopropyl-3-phenylbenzofuran-2-ylmethyl)-N'-(2,4,6-trifluorophenyl)urea

$^1$H-NMR ($CDCl_3$, $\delta$) : 0.83 (3H, t, J = 7Hz), 1.14 (8H, br s), 1.28 (6H, d, J = 7Hz), 1.39-1.50 (2H, m),

3.01 (1H, sep, J = 7Hz), 3.22 (2H, t, J = 7Hz), 4.75 (2H, s), 6.49 (1H, br s), 6.68-6.80 (2H, m), 7.23-7.53 ppm (8H, m)

(14) N-[3-(2-Chlorophenyl)-5-methylbenzofuran-2-ylmethyl]-N-heptyl-N'-(2,4,6-trifluorophenyl)urea

$^1$H-NMR ($CDCl_3$, $\delta$) : 0.85 (3H, t, J = 7Hz), 1.16 (8H, br s), 1.32-1.48 (2H, m), 2.40 (3H, s), 3.22 (2H, t, J = 7Hz), 4.62 (2H, ABq, J = 15, 14Hz), 6.47 (1H, s), 6.75 (2H, t, J = 7Hz), 7.11-7.20 (2H, m), 7.39-7.60 ppm (5H, m)

(15) N-Heptyl-N-(5-methyl-3-phenylbenzofuran-2-ylmethyl)-N'-(2,4,6-trifluorophenyl)urea

$^1$H-NMR ($CDCl_3$, $\delta$) : 0.83 (3H, t, J = 7Hz), 1.10 (8H, br s), 1.38-1.50 (2H, m), 2.45 (3H, s), 3.24 (2H, t, J = 7Hz), 4.74 (2H, s), 6.48 (1H, s), 6.72 (2H, t, J = 7Hz), 7.16-7.53 ppm (8H, m)

(16) N-(3,5-Diphenylbenzofuran-2-ylmethyl)-N-heptyl-N'-(2,4,6-trifluorophenyl)urea

$^1$H-NMR ($CDCl_3$, $\delta$) : 0.84 (3H, t, J = 7Hz), 1.18 (8H, br s), 1.36-1.53 (2H, m), 3.26 (2H, t, J = 7Hz), 4.83 (2H, s), 6.44 (1H, s), 6.75 (2H, t, J = 7Hz), 7.40-7.78 ppm (13H, m)

(17) N-[3-(2-Chlorophenyl)benzofuran-2-ylmethyl]-N-heptyl-N'-(2,4,6-trifluorophenyl)urea

$^1$H-NMR ($CDCl_3$, $\delta$) : 0.85 (3H, t, J = 7Hz), 1.17 (8H, s), 1.38-1.47 (2H, m), 3.22 (2H, t, J = 7Hz), 4.64 (2H, ABq, J = 15Hz), 6.44 (1H, s), 6.73 (2H, t, J = 7Hz), 7.28-7.40 (6H, m), 7.52-7.60 ppm (2H, m)

(18) N-[3-(4-Chlorophenyl)benzofuran-2-ylmethyl]-N-heptyl-N'-(2,4,6-trifluorophenyl)urea

$^1$H-NMR ($CDCl_3$, $\delta$) : 0.83 (3H, t, J = 7Hz), 1.15 (8H, br s), 1.40-1.50 (2H, m), 3.24 (2H, t, J = 7Hz), 4.79 (2H, s), 6.37 (1H, s), 6.70 (2H, t, J = 7Hz), 7.28-7.58 ppm (8H, m)

(19) N-Heptyl-N-[3-(2-methylphenyl)benzofuran-2-ylmethyl]-N'-(2,4,6-trifluorophenyl)urea

$^1$H-NMR ($CDCl_3$, $\delta$) : 0.85 (3H, t, J = 7Hz), 1.18 (8H, br s), 1.35-1.48 (2H, m), 2.20 (3H, s), 3.11-3.30 (2H, m), 4.59 (2H, ABq, J = 15Hz), 6.48 (1H, s), 6.75 (2H, t, J = 7Hz), 7.20-7.58 ppm (8H, m)

(20) N-Heptyl-N-[3-(3-methylphenyl)benzofuran-2-ylmethyl]-N'-(2,4,6-trifluorophenyl)urea

$^1$H-NMR ($CDCl_3$, $\delta$) : 0.84 (3H, t, J = 7Hz), 1.18 (8H, br s), 1.48-1.50 (2H, m), 2.45 (3H, s), 3.28 (2H, t, J = 7Hz), 4.79 (2H, s), 6.45 (1H, s), 6.73 (2H, t, J = 7Hz), 7.20-7.62 ppm (8H, m)

(21) N-Heptyl-N-[5-methyl-3-(4-methylphenyl)benzofuran-2-ylmethyl]-N'-(2,4,6-trifluorophenyl)urea

$^1$H-NMR ($CDCl_3$, $\delta$) : 0.83 (3H, t, J = 7Hz), 1.14 (8H, br s), 1.41 (2H, m), 2.42 (6H, s), 3.24 (2H, t, J = 7Hz), 4.73 (2H, s), 6.50 (1H, s), 6.71 (2H, t, J = 8Hz), 7.15 (1H, dd, J = 2, 8Hz), 7.28-7.42 ppm (6H, m)

(22) N-Heptyl-N-[3-(4-methylphenyl)benzofuran-2-ylmethyl]-N'-(2,4,6-trifluorophenyl)urea

$^1$H-NMR ($CDCl_3$, $\delta$) : 0.84 (3H, t, J = 7Hz), 1.13 (8H, br s), 1.42 (2H, m), 2.43 (3H, s), 3.24 (2H, t, J = 7Hz), 4.77 (2H, s), 6.47 (1H, br s), 6.72 (2H, t, J = 8Hz), 7.23-7.60 ppm (8H, m)

(23) N-Heptyl-N-(3-isopentylbenzofuran-2-ylmethyl)-N'-(2,4,6-trifluorophenyl)urea

$^1$H-NMR ($CDCl_3$, $\delta$) : 0.86 (3H, t, J = 7Hz), 0.98 (6H, d, J = 7Hz), 1.23-1.32 (8H, m), 1.50-1.70 (5H, m), 2.72 (2H, t, J = 7Hz), 3.38 (2H, t, J = 7Hz), 4.65 (2H, s), 6.72 (2H, t, J = 8Hz), 7.21-7.56 ppm (4H, m)

(24) N-Heptyl-N-(5-isopentylbenzofuran-2-ylmethyl)-N'-(2,4,6-trifluorophenyl)urea

$^1$H-NMR ($CDCl_3$, $\delta$) : 0.86 (3H, t, J = 7Hz), 0.94 (6H, d, J = 7Hz), 1.24-1.34 (8H, m), 1.47-1.67 (5H, m), 2.70 (2H, t, J = 7Hz), 3.42 (2H, t, J = 7Hz), 4.63 (2H, s), 6.37 (1H, s), 6.72 (2H, t, J = 8Hz), 7.11 (1H, dd, J = 2, 8Hz), 7.33-7.38 ppm (3H, m)

(25) N-[3-(4-Chlorophenyl)-5-methylbenzofuran-2-ylmethyl]-N-(4-methoxybenzyl)-N'-(2,4,6-trifluorophenyl)urea mp : 149.5-153.5 °C

$^1$H-NMR ($CDCl_3$, $\delta$) : 2.48 (3H, s), 3.79 (3H, s), 4.39 (2H, s), 4.67 (2H, s), 6.61-6.93 (6H, m), 7.18-7.50 ppm (8H, m)

(26) N-(4-Chlorobenzyl)-N-[3-(4-chlorophenyl)-5-methylbenzofuran-2-ylmethyl]-N'-(2,4,6-trifluorophenyl)-urea

$^1$H-NMR ($CDCl_3$, $\delta$) : 2.48 (3H, s), 4.48 (2H, s), 4.63 (2H, s), 6.76 (3H, t, J = 7Hz), 6.99 (2H, d, J = 7Hz), 7.15-7.25 (3H, m), 7.33-7.50 ppm (6H, m)

(27) N-[3-(4-Chlorophenyl)-5-methylbenzofuran-2-ylmethyl]-N'-(2,4,6-trifluorophenyl)urea mp : 234 °C

$^1$H-NMR ($CDCl_3$, $\delta$) : 4.64 (2H, d, J = 5Hz), 6.72 (2H, t, J = 8Hz), 7.27-7.47 ppm (7H, m)

(28) N-[3-(4-Chlorophenyl)-5-methylbenzofuran-2-ylmethyl]-N-cycloheptyl-N'-(2,4,6-trifluorophenyl)urea mp : 54-58 °C

$^1$H-NMR ($CDCl_3$, $\delta$) : 1.30 (10H, br s), 1.66-1.79 (2H, m), 2.47 (3H, s), 4.03-4.12 (1H, m), 4.70 (2H, s), 6.86 (1H, s), 6.72 (2H, t, J = 7Hz), 7.17-7.56 ppm (7H, m)

(29) N-[3-(4-Chlorophenyl)-5-methylbenzofuran-2-ylmethyl]-N-cyclohexylmethyl-N'-(2,4,6-trifluorophenyl)-urea

$^1$H-NMR ($CDCl_3$, $\delta$) : 0.78-0.90 (3H, m), 1.03-1.13 (4H, m), 1.44-1.67 (4H, m), 2.45 (3H, s), 3.09 (2H, d, J = 7Hz), 4.74 (2H, s), 6.40 (1H, br s), 6.70 (2H, t, J = 7Hz), 7.13-7.51 ppm (7H, m)

(30) N-(5-Chloro-3-phenylbenzofuran-2-ylmethyl)-N-cyclopentyl-N'-(2,4,6-trifluorophenyl)urea

$^1$H-NMR ($CDCl_3$, $\delta$) : 1.40 (6H, br s), 1.75-1.85 (2H, m), 4.38-4.54 (1H, m), 4.78 (2H, s), 6.59 (1H, s), 6.72 (2H, t, J = 7Hz), 7.29-7.58 ppm (8H, m)

(31) N-[3-(4-Chlorophenyl)-5-methylbenzofuran-2-ylmethyl]-N-pentyl-N'-(2,4,6-trifluorophenyl)urea

$^1$H-NMR ($CDCl_3$, $\delta$) : 0.80 (3H, t, J = 7Hz), 1.17 (4H, br s), 1.48-1.51 (2H, m), 2.47 (3H, s), 3.22 (2H, t, J = 7Hz), 4.73 (2H, s), 6.40 (1H, s), 6.72 (2H, t, J = 7Hz), 7.16-7.52 ppm (7H, m)

(32) N-[3-(4-Chlorophenyl)-5-methylbenzofuran-2-ylmethyl]-N-nonyl-N'-(2,4,6-trifluorophenyl)urea

$^1$H-NMR ($CDCl_3$, $\delta$) : 0.88 (3H, t, J = 7Hz), 1.16 (12H, br s), 1.40-1.50 (2H, m), 2.44 (3H, s), 3.25 (2H, t, J = 7Hz), 4.77 (2H, s), 6.39 (1H, s), 6.72 (2H, t, J = 7Hz), 7.18-7.52 ppm (7H, m)

(33) N-[3-(4-Chlorophenyl)-5-methylbenzofuran-2-ylmethyl]-N-propyl-N'-(2,4,6-trifluorophenyl)urea

$^1$H-NMR ($CDCl_3$, $\delta$) : 0.82 (3H, t, J = 7Hz), 1.40-1.59 (2H, m), 2.44 (3H, s), 3.23 (2H, t, J = 7Hz), 4.74 (2H, s), 6.39 (1H, s), 6.72 (2H, t, J = 7Hz), 7.15-7.52 ppm (7H, m)

(34) N-[3-(4-Chlorophenyl)-5,7-dimethylbenzofuran-2-ylmethyl]-N-heptyl-N'-(2,4,6-trifluorophenyl)urea

$^1$H-NMR ($CDCl_3$, $\delta$) : 0.84 (3H, t, J = 7Hz), 1.18 (8H, br s), 1.40-1.50 (2H, m), 2.41 (3H, s), 2.54 (3H, s), 3.25 (2H, t, J = 7Hz), 4.77 (2H, s), 6.31 (1H, s), 6.72 (2H, t, J = 7Hz), 7.01 (1H, s), 7.15 (1H, s), 7.46 ppm (4H, dd, J = 7, 5Hz)

(35) N-[3-(4-Chlorophenyl)-5-ethylbenzofuran-2-ylmethyl]-N-heptyl-N'-(2,4,6-trifluorophenyl)urea

$^1$H-NMR ($CDCl_3$, $\delta$) : 0.85 (3H, t, J = 7Hz), 1.18 (11H, br s), 1.39-1.50 (2H, m), 2.66 (2H, q, J = 7Hz), 3.24 (2H, t, J = 7Hz), 4.75 (2H, s), 6.40 (1H, s), 6.73 (2H, t, J = 7Hz), 7.20-7.33 (2H, m), 7.42-7.52 ppm (5H, m)

(36) N-[3-(4-Chlorophenyl)-5-methylbenzofuran-2-ylmethyl]-N-heptyl-N'-(4-nitrophenyl)urea mp : 141-142 °C

$^1$H-NMR ($CDCl_3$, $\delta$) : 0.85 (3H, t, J = 7Hz), 1.20 (8H, s), 1.49 (2H, t, J = 7Hz), 2.43 (3H, s), 3.28 (2H, t, J = 7Hz), 4.72 (2H, s), 6.99 (1H, s), 7.19 (1H, dd, J = 2, 8Hz), 7.31 (1H, d, J = 2Hz), 7.41 (2H, d, J = 9Hz), 7.41 (1H, d, J = 8Hz), 7.45 (2H, d, J = 8Hz), 7.52 (2H, d, J = 8Hz), 8.16 ppm (2H, d, J = 9Hz)

(37) N-[3-(4-Chlorophenyl)-5-methylbenzofuran-2-ylmethyl]-N-heptyl-N'-(2,6-diisopropylphenyl)urea mp : 172-173 °C

$^1$H-NMR ($CDCl_3$, $\delta$) : 0.85 (3H, t, J = 7Hz), 1.17 (20H, br s), 1.48 (2H, t, J = 7Hz), 2.43 (3H, s), 3.08 (2H, m), 3.32 (2H, t, J = 7Hz), 4.77 (2H, s), 6.04 (1H, s), 7.17-7.45 ppm (10H, m)

## Example 4

To a stirred solution of 2,4,6-trifluoroaniline (335 mg) in ethyl acetate (10 ml) was added trichloromethyl chloroformate (258 mg) dropwise at ambient temperature and the mixture was stirred under reflux for 3 hours. To this mixture was added a solution of N-heptyl-[3-(4-chlorophenyl)-5-methylbenzofuran-2-ylmethyl]-amine (817 mg), prepared by treating the corresponding hydrochloride with 1N aqueous sodium hydroxide, in ethyl acetate (2 ml) dropwise at 0° and the mixture was stirred at ambient temperature for 1 hour. The reaction mixture was washed with 1N aqueous sodium hydroxide, 1N hydrochloric acid and saturated aqueous sodium bicarbonate, and dried. Evaporation of solvent gave a residue which was purified by column chromatography on silica gel. Elution with ethyl acetate-hexane (1:10) gave N-[3-(4-chlorophenyl)-5-methylbenzofuran-2-ylmethyl]-N-heptyl-N'-(2,4,6-trifluorophenyl)urea (724 mg).

$^1$H-NMR ($CDCl_3$, $\delta$) : 7.52-7.15 (7H, m), 6.74 (2H, t, J = 7Hz), 6.40 (1H, s), 4.77 (2H, s), 3.25 (2H, t, J = 7Hz), 2.46 (3H, s), 1.52-1.38 (2H, m), 1.18 (8H, br s), 0.85 ppm (3H, t, J = 7Hz)

Example 5

The following compounds were obtained according to a similar manner to that of Example 1.

(1) N-Benzyl-N-[5-chloro-3-(4-chlorophenyl)benzofuran-2-ylmethyl]-N'-(2,4,6-trifluorophenyl)urea mp : 166-167 °C

$^1$H-NMR ($CDCl_3$, $\delta$) : 4.46 (2H, s), 4.72 (2H, s), 6.38 (1H, br s), 6.72 (2H, t, J = 8Hz), 7.00-7.04 (2H, m), 7.23-7.48 ppm (10H, m)

(2) N-Benzyl-N-(3-phenylindol-2-ylmethyl)-N'-(2,4,6-trifluorophenyl)urea mp : 173-174.5 °C

$^1$H-NMR ($CDCl_3$, $\delta$) : 4.43 (2H, s), 4.75 (2H, s), 5.75 (1H, s), 6.72 (2H, t, J = 8Hz), 7.02-7.43 (13H, m) 7.62 (1H, d, J = 8Hz), 9.22 ppm (1H, s)

(3) N-Benzyl-N-[3-(4-chlorophenyl)-6-methylbenzofuran-2-ylmethyl]-N'-(2,4,6-trifluorophenyl)urea mp : 179.5-181 °C

$^1$H-NMR ($CDCl_3$, $\delta$) : 2.53 (3H, s), 4.44 (2H, s), 4.69 (2H, s), 6.63 (1H, br s), 6.75 (2H, t, J = 8Hz), 6.99-7.03 (2H, m), 7.11-7.48 ppm (10H, m)

(4) N-[3-(4-Chlorophenyl)-5-methylbenzofuran-2-ylmethyl]-N-furfuryl-N'-(2,4,6-trifluorophenyl)urea mp : 146-146.5 °C

$^1$H-NMR ($CDCl_3$, $\delta$) : 2.44 (3H, s), 4.43 (2H, s), 4.77 (2H, s), 5.92 (1H, d, J = 3Hz), 6.72 (1H, br s), 6.72 (2H, t, J = 8Hz), 7.17 (1H, dd, J = 2, 8Hz), 7.30 (2H, s), 7.42 (1H, d, J = 8Hz), 7.42 (2H, d, J = 8Hz), 7.48 ppm (2H, d, J = 8Hz)

(5) N-[3-(4-Chlorophenyl)-5-methylbenzofuran-2-ylmethyl]-N-(2-fluorobenzyl)-N'-(2,4,6-trifluorophenyl)urea mp : 157-158 °C

$^1$H-NMR ($CDCl_3$, $\delta$) : 2.44 (3H, s), 4.57 (2H, s), 4.73 (2H, s), 6.58 (1H, s), 6.73 (2H, t, J = 8Hz), 7.00 (2H, q, J = 8Hz), 7.12-7.27 (4H, m), 7.34 (2H, d, J = 8Hz), 7.41 (1H, d, J = 8Hz), 7.43 ppm (2H, d, J = 8Hz)

(6) N-[3-(4-Chlorophenyl)-5-methylbenzofuran-2-ylmethyl]-N-(3-fluorobenzyl)-N'-(2,4,6-trifluorophenyl)urea mp : 162-163 °C

$^1$H-NMR ($CDCl_3$, $\delta$) : 2.45 (3H, s), 4.42 (2H, s), 4.65 (2H, s), 6.69-6.78 (5H, m), 6.92 (1H, dt, J = 2, 8Hz), 7.13-7.35 (6H, m), 7.45 ppm (2H, t, J = 8Hz)

(7) N-[3-(4-Chlorophenyl)-5-methylbenzofuran-2-ylmethyl]-N-(4-fluorobenzyl)-N'-(2,4,6-trifluorophenyl)urea mp : 171-172 °C

$^1$H-NMR ($CDCl_3$, $\delta$) : 2.43 (3H, s), 4.35 (2H, s), 4.61 (2H, s), 6.71 (1H, s), 6.73 (2H, t, J = 8Hz), 6.89-6.92 (4H, m), 7.18-7.50 ppm (7H, m)

(8) N-[3-(4-Chlorophenyl)-5-methylbenzofuran-2-ylmethyl]-N-phenyl-N'-(2,4,6-trifluorophenyl)urea mp : 180-182.5 °C

$^1$H-NMR ($CDCl_3$, $\delta$) : 2.41 (3H, s), 5.12 (2H, s), 5.56 (1H, s), 6.71 (2H, t, J = 8Hz), 7.02 (2H, d, J = 8Hz), 7.11-7.43 ppm (10H, m)

(9) N-[3-(4-Chlorophenyl)-5-methylbenzofuran-2-ylmethyl]-N-cyclopropyl-N'-(2,4,6-trifluorophenyl)urea

$^1$H-NMR ($CDCl_3$, $\delta$) : 0.80-0.82 (4H, m), 2.42 (3H, s), 2.58 (1H, m), 4.82 (2H, s), 6.62 (1H, s), 6.72 (2H, t, J = 8Hz), 7.13 (1H, dd, J = 2, 8Hz), 7.30 (1H, d, J = 2Hz), 7.40 (1H, d, J = 8Hz), 7.47 ppm (4H, s)

(10) N-Furfuryl-N-[5-methyl-3-(4-methylphenyl)benzofuran-2-ylmethyl]-N'-(2,4,6-trifluorophenyl)urea mp : 154-155 °C

$^1$H-NMR ($CDCl_3$, $\delta$) : 2.43 (6H, s), 4.42 (2H, s), 4.75 (2H, s), 5.83 (1H, d, J = 3Hz), 6.21 (1H, m), 6.71 (2H, t, J = 8Hz), 7.16 (1H, dd, J = 2, 8Hz), 7.28-7.42 ppm (7H, m)

(11) N-Heptyl-N-(7-phenylbenzofuran-2-ylmethyl)-N'-(2,4,6-trifluorophenyl)urea

$^1$H-NMR ($CDCl_3$, $\delta$) : 0.88 (3H, t, J = 7Hz), 1.29 (8H, br s), 1.61-1.76 (2H, m), 3.45 (2H, t, J = 7Hz) 4.71 (2H, s), 6.00 (1H, s), 6.70 (2H, t, J = 7Hz), 6.70 (1H, s), 7.29-7.56 (6H, m) 7.81 ppm (2H, dd, J = 2, 8Hz)

(12) N-Heptyl-N-(3-phenylindol-2-ylmethyl)-N'-(2,4,6-trifluorophenyl)urea mp : 122-123.5 °C

$^1$H-NMR ($CDCl_3$, $\delta$) : 0.84 (3H, t, J = 7Hz), 1.13-1.23 (8H, m), 1.42 (2H, br t, J = 7Hz), 3.08 (2H, t, J = 7Hz) 4.69 (2H, s), 5.72 (1H, s), 6.77 (2H, t, J = 8Hz) 7.12 (1H, td, J = 2, 8Hz), 7.33-7.49 (6H, m) 7.63 (1H, d, J = 8Hz), 9.34 ppm (1H, s)

(13) N-Heptyl-N-(6-phenylbenzofuran-2-ylmethyl)-N'-(2,4,6-trifluorophenyl)urea

$^1$H-NMR ($CDCl_3$, $\delta$) : 0.89 (3H, t, J = 7Hz), 1.25 (8H, br s), 1.60-1.75 (2H, m), 3.45 (2H, t, J = 7Hz), 4.70 (2H, s), 6.34 (1H, s), 6.72 (1H, s), 6.75 (2H, t, J = 8Hz), 7.33-7.70 ppm (8H, m)

(14) N-Heptyl-N-[5-methyl-3-(4-propylphenyl)benzofuran-2-ylmethyl]-N'-(2,4,6-trifluorophenyl)urea

$^1$H-NMR ($CDCl_3$, $\delta$) : 0.83 (3H, t, J = 7Hz), 0.98 (3H, t, J = 7Hz), 1.13 (8H, br s), 1,42 (2H, m), 1.70

(2H, tq, J = 7, 7Hz), 2.43 (3H, s), 2.65 (2H, t, J = 7Hz), 3.23 (2H, t, J = 7Hz), 4.75 (2H, s), 6.47 (1H, s), 6.72 (2H, t, J = 8Hz), 7.17 (1H, dd, J = 2, 8Hz), 7.32 (2H, d, J = 8Hz), 7.37-7.42 ppm (4H, m)

(15) N-[3-(4-Bromophenyl)-5-methylbenzofuran-2-ylmethyl]-N-furfuryl-N'-(2,4,6-trifluorophenyl)urea mp : 144 °C

$^1$H-NMR ($CDCl_3$, δ) : 2.44 (3H, s), 4.43 (2H, s), 4.77 (2H, s), 5.92 (1H, d, J = 4Hz), 6.23 (1H, m), 6.72 (1H, s), 6.73 (2H, t, J = 8Hz), 7.17 (1H, dd, J = 2, 8Hz), 7.31 (1H, d, J = 2Hz), 7.36 (2H, d, J = 8Hz), 7.40 (1H, d, J = 8Hz), 7.64 ppm (2H, d, J = 8Hz)

Example 6

The following compounds were obtained according to a similar manner to that of Example 4.

(1) N-Benzyl-N-[3-(4-bromophenyl)-5-methylbenzofuran-2-ylmethyl]-N'-(2,4,6-trifluorophenyl)urea mp : 172-174 °C

$^1$H-NMR ($CDCl_3$, δ) : 2.43 (3H, s), 4.43 (2H, s), 4.66 (2H, s), 6.73 (2H, t, J = 8Hz), 6.98 (2H, m), 7.16-7.31 (7H, m), 7.42 (1H, d, J = 8Hz), 7.61 ppm (2H, d, J = 8Hz)

(2) N-Benzyl-N-[3-(4-chlorophenyl)-5-methylbenzofuran-2-ylmethyl]-N'-(2,4,6-trifluorophenyl)urea mp : 161-162 °C

$^1$H-NMR ($CDCl_3$, δ) : 2.43 (3H, s), 4.42 (2H, s), 4.66 (2H, s), 6.62 (1H, br s), 6.72 (2H, t, J = 8Hz), 6.96-7.02 (2H, s), 7.16-7.47 ppm (10H, m)

(3) N-Benzyl-N-[3-(4-chlorophenyl)-5-methylbenzofuran-2-ylmethyl]-N'-(2,4,6-trifluorophenyl)urea

$^1$H-NMR ($CDCl_3$, δ) : 2.18 (6H, s), 2.28 (3H, s), 2.47 (3H, s), 4.48 (2H, s), 4.75 (2H, s), 6.20 (1H, s), 6.88 (2H, s), 7.04-7.48 ppm (12H, m)

(4) N-Benzyl-N-[3-(4-fluorophenyl)-5-methylbenzofuran-2-ylmethyl]-N'-(2,4,6-trifluorophenyl)urea mp : 149-150 °C

$^1$H-NMR ($CDCl_3$, δ) : 2.43 (3H, s), 4.43 (2H, s), 4.65 (2H, s), 6.63 (1H, br s), 6.73 (2H, t, J = 8Hz), 7.02 (2H, m), 7.14 (1H, d, J = 8Hz), 7.26-7.43 ppm (9H, m)

(5) N-Benzyl-N-[5-methyl-3-(4-methylphenyl)benzofuran-2-ylmethyl]-N'-(2,4,6-trifluorophenyl)urea mp : 158-159 °C

$^1$H-NMR ($CDCl_3$, δ) : 2.44 (3H, s), 2.45 (3H, s), 4.41 (2H, s), 4.63 (2H, s), 6.66 (1H, br s), 6.72 (2H, t, J = 8Hz), 6.98-7.43 ppm (12H, m)

(6) N-Benzyl-N-[5-chloro-3-(4-methylphenyl)benzofuran-2-ylmethyl]-N'-(2,4,6-trifluorophenyl)urea

$^1$H-NMR ($CDCl_3$, δ) : 2.48 (3H, s), 4.46 (2H, s), 4.70 (2H, s), 6.46 (1H, s), 6.74 (2H, t, J = 7Hz), 7.00-7.05 (2H, m), 7.20-7.53 ppm (10H, m)

(7) N-[3-(4-Chlorophenyl)-5-methylbenzofuran-2-ylmethyl]-N-(4-methylbenzyl)-N'-(2,4,6-trifluorophenyl)-urea mp : 166.5-167.5 °C

$^1$H-NMR ($CDCl_3$, δ) : 2.33 (3H, s), 2.47 (3H, s), 4.40 (2H, s), 4.69 (2H, s), 6.58 (1H, s), 6.75 (2H, t, J = 7Hz), 6.90 (2H, d, J = 7Hz), 7.05 (2H, d, J = 7Hz), 7.18-7.49 ppm (7H, m)

(8) N-[3-(4-Chlorophenyl)-5-methylbenzofuran-2-ylmethyl]-N-(2-phenylethyl)-N'-(2,4,6-trifluorophenyl)urea mp : 159-161 °C

$^1$H-NMR ($CDCl_3$, δ) : 2.43 (3H, s), 2.77 (2H, t, J = 7Hz), 3.42 (2H, t, J = 7Hz), 4.57 (2H, s), 6.42 (1H, s), 6.73 (2H, t, J = 8Hz), 6.96 (2H, dd, J = 2, 8Hz), 7.17-7.30 (5H, m), 7.37 (2H, d, J = 8Hz), 7.40 (1H, d, J = 8Hz), 7.50 ppm (2H, d, J = 8Hz)

(9) N-[3-(4-Chlorophenyl)-5-methylbenzofuran-2-ylmethyl]-N-(3,4-dichlorobenzyl)-N'-(2,4,6-trifluorophenyl)urea mp : 193-195 °C

$^1$H-NMR ($CDCl_3$, δ) ; 2.45 (3H, s), 4.32 (2H, s), 4.60 (2H, s), 6.76 (2H, t, J = 8Hz), 6.82 (1H, d, J = 2Hz), 6.88 (1H, dd, J = 2, 8Hz), 7.19-7.32 (3H, m), 7.33 (2H, d, J = 8Hz), 7.42 (1H, d, J = 8Hz), 7.51 ppm (2H, d, J = 8Hz)

(10) N-Benzyl-N-[(3-(4-chlorophenyl)-5-methylbenzofuran-2-ylmethyl]-N'-(2,4,6-trichlorophenyl)urea mp : 181-182 °C

$^1$H-NMR ($CDCl_3$, δ) : 2.47 (3H, s), 4.43 (2H, s), 4.69 (2H, s), 6.98-7.04 (3H, m), 7.17-7.48 ppm (12H, m)

(11) N-Benzyl-N-(5-chloro-3-phenylbenzofuran-2-ylmethyl)-N'-(2,4,6-trifluorophenyl)urea mp : 179-180 °C

$^1$H-NMR ($CDCl_3$, δ) : 4.43 (2H, s), 4.70 (2H, s), 6.48 (1H, s), 6.73 (2H, t, J = 7Hz), 6.94-7.01 (2H, m), 7.19-7.25 (3H, m), 7.30-7.54 ppm (8H, m)

(12) N-(5-Chloro-3-phenylbenzofuran-2-ylmethyl)-N-cyclobutyl-N'-(2,4,6-trifluorophenyl)urea mp : 142-143 °C

$^1$H-NMR ($CDCl_3$, δ) : 1.46-1.60 (2H, m), 1.87-2.08 (4H, m), 4.28 (1H, m), 4.83 (2H, s), 6.15 (1H, s), 6.72 (2H, t, J=8Hz), 7.27-7.54 ppm (8H, m)

(13) N-[3-(4-Chlorophenyl)-5-methylbenzofuran-2-ylmethyl]-N-cyclobutyl-N'-(2,4,6-trifluorophenyl)urea mp : 111°-112°C

$^1$H-NMR ($CDCl_3$, δ) : 1.45-1.61 (2H, m), 1.88-2.11 (4H, m), 2.43 (3H, s), 4.27 (1H, m), 4.82 (2H, s), 6.25 (1H, s), 6.72 (2H, t, J=8Hz), 7.16 (1H, dd, J=2, 8Hz), 7.31 (1H, d, J=2Hz), 7.41 (1H, d, J=8Hz), 7.41-7.52 ppm (4H, m)

(14) N-[3-(4-Chlorophenyl)-5-methylbenzofuran-2-ylmethyl]-N-cyclohexyl-N'-(2,4,6-trifluorophenyl)urea mp : 170-173°C

$^1$H-NMR ($CDCl_3$, δ) : 0.70-1.32 (6H, m), 1.58-1.67 (4H, m), 2.43 (3H, s), 4.02 (1H, m), 4.68 (2H, s), 6.74 (2H, t, J=8Hz), 6.82 (1H, s), 7.18 (1H, dd, J=2, 8Hz), 7.31 (1H, d, J=2Hz), 7.42 (1H, d, J=8Hz), 7.41 (2H, d, J=8Hz), 7.52 ppm (2H, d, J=8Hz)

(15) N-[3-(4-Chlorophenyl)-5-methylbenzofuran-2-ylmethyl]-N-cyclopentyl-N'-(2,4,6-trifluorophenyl)urea mp : 151-152°C

$^1$H-NMR ($CDCl_3$, δ) : 1.27-1.45 (6H, m), 1.73-1.83 (2H, m), 2.44 (3H, s), 4.43 (1H, m), 4.70 (2H, s), 6.74 (2H, t, J=8Hz), 6.75 (1H, s), 7.18 (1H, dd, J=2, 8Hz), 7.31 (1H, s), 7.41 (2H, d, J=8Hz), 7.42 (1H, d, J=8Hz), 7.51 ppm (2H, d, J=8Hz)

(16) N-[3-(4-Chlorophenyl)-5-methylbenzofuran-2-ylmethyl]-N-heptyl-N'-(2,4,6-trimethoxyphenyl)urea

$^1$H-NMR ($CDCl_3$, δ) : 0.88 (3H, t, J=7Hz), 1.18 (8H, br s), 1.45-1.55 (2H, m), 2.45 (3H, s), 3.27 (2H, t, J=7Hz), 3.75 (6H, s), 3.80 (3H, s), 4.78 (2H, s), 5.89 (1H, s), 6.16 (2H, s), 7.12-7.19 (1H, m), 7.32-7.48 ppm (6H, m)

(17) N-(5-chloro-3-phenylbenzofuran-2-ylmethyl)-N-heptyl-N'-(2,4,6-trifluorophenyl)urea

$^1$H-NMR ($CDCl_3$, δ) : 0.83 (3H, t, J=7Hz), 1.15 (8H, br s), 1.43 (2H, m), 3.22 (2H, t, J=7Hz), 4.78 (2H, s), 6.27 (1H, s), 6.72 (2H, t, J=8Hz), 7.32 (1H, dd, J=2, 8Hz), 7.43-7.53 ppm (7H, m)

Example 7

To a solution of N-[3-(4-chlorophenyl)-5-methylbenzofuran-2-ylmethyl]-N-(4-methoxybenzyl)-N'-(2,4,6-trifluorophenyl)urea (701 mg) in methylene chloride (15 ml) was added boron tribromide (0.7 ml) dropwise at 0°C and the mixture was stirred at the same temperature for 1 hours. The reaction mixture was poured into ice cold water and washed with water, and dried. Evaporation of solvent followed by recrystallization from ethyl acetate-n-hexane gave N-[3-(4-chlorophenyl)-5-methylbenzofuran-2-ylmethyl]-N-(4-hydroxybenzyl)-N'-(2,4,6-trifluorophenyl)urea (238 mg).

mp : 175-178°C

$^1$H-NMR ($CD_3OD$, δ) : 2.43 (3H, s), 4.34 (2H, s), 4.72 (2H, s), 6.56-7.00 (6H, m), 7.12-7.58 ppm (8H, m)

Example 8

A solution of N-[3-(4-chlorophenyl)-5-methylbenzofuran-2-ylmethyl]-N-heptyl-N'-(4-nitrophenyl)-urea (2.2 g) in methanol (30 ml) was hydrogenated over 10% palladium on carbon at 4 kg/cm2 pressure at ambient temperature for 7 hours. 10% Palladium on carbon was filtered off and washed with methanol. The combined filtrate and washing were concentrated under the reduced pressure to leave N-(4-aminophenyl)-N'-[3-(4-chlorophenyl)-5-methylbenzofuran-2-ylmethyl]-N'-heptylurea (2.0 g).

$^1$H-NMR ($CDCl_3$, δ) : 0.85 (3H, t, J=7Hz), 1.19 (8H, br s), 1.45 (2H, m), 2.43 (3H, s), 3.22 (2H, t, J=7Hz), 4.72 (2H, s), 6.30 (1H, s), 6.63 (2H, d, J=8Hz), 7.04 (2H, d, J=8Hz), 7.15 (1H, dd, J=2, 8Hz), 7.30 (1H, d, J=2Hz), 7.40 (1H, d, J=8Hz), 7.45 (2H, d, J=8Hz), 7.48 ppm (2H, d, J=8Hz)

Example 9

A mixture of N-(4-aminophenyl)-N'-[3-(4-chlorophenyl)-5-methylbenzofuran-2-ylmethyl]-N-heptylurea (0.3 g), acetic anhydride (0.3 ml) and pyridine (0.3 ml) was allowed to stand at ambient temperature overnight. The reaction mixture was poured into ice cold water and extracted with chloroform. The extract was washed with 3% hydrochloric acid and dilute aqueous sodium bicarbonate, and dried. Evaporation of solvent gave a residue which was chromatographed on silica gel. Elution with chloroform gave N-(4-acetylaminophenyl)-N'-[3-(4-chlorophenyl)-5-methylbenzofuran-2-ylmethyl]-N'-heptylurea (0.28 g).

$^1$H-NMR ($CDCl_3$, δ) : 0.84 (3H, t, J = 7Hz), 1.15 (8H, br s), 1.43 (2H, m), 2.04 (3H, s), 2.42 (3H, s), 3.22 (2H, t, J = 7Hz), 4.72 (2H, s), 6.72 (1H, s), 7.01-7.28 (6H, m), 7.37 (1H, d, J = 8Hz), 7.42 (2H, d, J = 8Hz), 7.46 (2H, d, J = 8Hz), 8.20 ppm (1H, s)

Example 10

A mixture of N-(4-aminophenyl)-N'-[3-(4-chlorophenyl)-5-methylbenzofuran-2-ylmethyl]-N'-heptylurea (315 mg), methanesulfonyl chloride (90 mg) and pyridine (0.5 ml) was allowed to stand at ambient temperature overnight. The reaction mixture was poured into ice cold water and extracted with chloroform. The extract was washed with dilute hydrochloric acid and dilute aqueous sodium bicarbonate, and dried. Evaporation of solvent gave a residue which was chromatographed on silica gel. Elution with chloroform gave N-[3-(4-chlorophenyl)-5-methylbenzofuran-2-ylmethyl]-N-heptyl-N'-(4-methylsulfonylaminophenyl)urea (0.15 g).

$^1$H-NMR ($CDCl_3$, δ) : 0.85 (3H, t, J = 7Hz), 1.16 (8H, br s), 1.45 (2H, m), 2.43 (3H, s), 2.90 (3H, s), 3.23 (2H, t, J = 7Hz), 4.73 (2H, s), 6.65 (1H, s), 7.02-7.22 (5H, m), 7.29 (1H, d, J = 2Hz), 7.39 (1H, d, J = 8Hz), 7.43 (2H, d, J = 8Hz), 7.49 ppm (2H, d, J = 8Hz)

Example 11

A mixture of N-(4-aminophenyl)-N'-[3-(4-chlorophenyl)-5-methylbenzofuran-2-ylmethyl]-N'-heptylurea (0.8 g), 37% formaldehyde (0.3 g) and 10% palladium on carbon (0.13 g) in methanol (20 ml) was hydrogenated at ambient temperature at 4 kg/cm2 pressure for 20 hours. 10% Palladium on carbon was filtered off and washed with methanol. The combined filtrate and washings were concentrated under the reduced pressure to leave a residue which was chromatographed on silica gel. Elution with chloroform gave N-[3-(4-chlorophenyl)-5-methylbenzofuran-2-ylmethyl]-N'-(4-dimethylaminophenyl)-N-heptylurea (0.5 g).

$^1$H-NMR ($CDCl_3$, δ) : 0.86 (3H, t, J = 7Hz), 1.18 (8H, br s), 1.46 (2H, m), 2.43 (3H, s), 2.39 (6H, s), 3.22 (2H, t, J = 7Hz), 4.73 (2H, s), 6.32 (1H, s), 6.70 (2H, d, J = 8Hz), 7.13 (2H, d, J = 8Hz), 7.15 (1H, dd, J = 2, 8Hz), 7.30 (1H, d, J = 2Hz), 7.40 (1H, d, J = 8Hz), 7.46 ppm (4H, s)

**Claims**

**Claims for the following Contracting States : AT, BE, CH, LI, DE, DK, FR, GB, IT, LU, NL, PT, SE**

**1.** A compound of the formula :

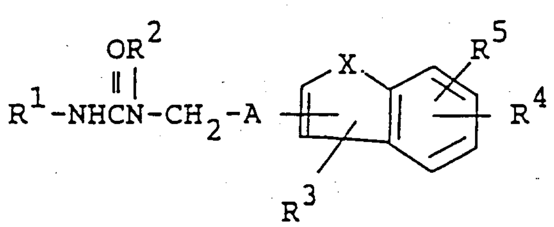

(I)

wherein

$R^1$ is phenyl which may be substitited with $C_1$-$C_6$ alkyl, halogen, nitro, amino, $C_1$-$C_6$ alkylamino, $C_1$-$C_6$ alkoxy or acylamino,

$R^2$ is hydrogen; $C_1$-$C_{20}$ alkyl; cyclo($C_3$-$C_{20}$)alkyl; or $C_1$-$C_6$ alkyl which is substituted with cyclo-($C_3$-$C_6$)alkyl, cyclo($C_3$-$C_6$)alkenyl, furyl or phenyl optionally substituted with substituent(s) selected from the group consisting of $C_1$-$C_6$ alkyl, halogen, hydroxy and $C_1$-$C_6$ alkoxy;

$R^3$ is hydrogen, $C_1$-$C_6$ alkyl or phenyl which may be substituted with $C_1$-$C_6$ alkyl, halogen, nitro, amino or $C_1$-$C_6$ alkylamino,

$R^4$ is hydrogen, halogen, $C_1$-$C_6$ alkyl, $C_1$-$C_6$ alkoxy or phenyl which may be substituted with $C_1$-$C_6$ alkyl or halogen,

$R^5$ is hydrogen, halogen, $C_1$-$C_6$ alkyl or phenyl which may be substituted with $C_1$-$C_6$ alkyl,

A is a single bond or $C_1$-$C_6$ alkylene, and

X is O, S or NH,

in which at least one of $R^3$, $R^4$ and $R^5$ is not hydrogen, provided that $R^3$ is phenyl which may be

substituted with $C_1$-$C_6$ alkyl, halogen, nitro, amino or $C_1$-$C_6$ alkyamino, or
$R^4$ is halogen, $C_1$-$C_6$ alkoxy or phenyl which may be substituted with $C_1$-$C_6$ alkyl or halogen, when $R^2$ is cyclo($C_3$-$C_{20}$)alkyl,
and pharmaceutically acceptable salts thereof.

**2.** A compound according to claim 1,
wherein
$R^2$ is hydrogen; $C_1$-$C_{20}$ alkyl; cyclo($C_3$-$C_{20}$)alkyl; or $C_1$-$C_6$ alkyl which is substituted with cyclo-($C_3$-$C_6$)alkyl, furyl or phenyl optionally substituted with substituent(s) selected from the group consisting of $C_1$-$C_6$ alkyl, halogen, hydroxy and $C_1$-$C_6$ alkoxy.

**3.** A compound according to claim 2,
wherein
$R^1$ is phenyl which may be substituted with $C_1$-$C_6$ alkyl, halogen, nitro, amino, $C_1$-$C_6$ alkyamino or $C_1$-$C_6$ alkoxy,
$R^2$ is $C_1$-$C_{20}$ alkyl; cyclo($C_3$-$C_{20}$)alkyl; or $C_1$-$C_6$ alkyl which is substituted with cyclo($C_3$-$C_6$)alkyl, furyl or phenyl optionally substituted with substituent(s) selected from the group consisting of $C_1$-$C_6$ alkyl, halogen, hydroxy and $C_1$-$C_6$ alkoxy, and
$R^5$ is hydrogen.

**4.** A compound according to claim 3,
wherein
$R^2$ is $C_1$-$C_{20}$ alkyl; cyclo($C_3$-$C_{20}$)alkyl; or $C_1$-$C_6$ alkyl which is substituted with phenyl optionally substituted with $C_1$-$C_6$ alkyl,

**5.** A compound according to claim 3,
wherein
$R^1$ is phenyl which may be substituted with $C_1$-$C_6$ alkyl, halogen or $C_1$-$C_6$ alkoxy, and
X is O or S.

**6.** A compound according to claim 5,
wherein
$R^3$ is hydrogen or phenyl which may be substituted with $C_1$-$C_6$ alkyl or halogen, and
$R^4$ is hydrogen, halogen, $C_1$-$C_6$ alkyl or phenyl which may be substituted with $C_1$-$C_6$ alkyl or halogen.

**7.** A compound according to claim 6, wherein
$R^2$ is $C_1$-$C_{20}$ alkyl; cyclo($C_3$-$C_{20}$)alkyl; or $C_1$-$C_6$ alkyl which is substituted with phenyl optionally substituted with $C_1$-$C_6$ alkyl.

**8.** A compound according to claim 6,
wherein
$R^1$ is phenyl which is substituted with 1 to 3 $C_1$-$C_6$ alkyl, halogen or $C_1$-$C_6$ alkoxy substituent(s).
$R^2$ is $C_1$-$C_{20}$ alkyl; cyclo($C_3$-$C_{20}$)alkyl; or $C_1$-$C_6$ alkyl which is substituted with cyclo($C_3$-$C_6$)alkyl, furyl or phenyl optionally substituted with substituent(s) selected from the group consisting of $C_1$-$C_6$ alkyl, halogen, hydroxy and $C_1$-$C_6$ alkoxy; and
$R^3$ is phenyl which may be substituted with $C_1$-$C_6$ alkyl or halogen; or
$R^1$ and $R^2$ are each defined above, and
$R^4$ is halogen or phenyl which may be substituted with $C_1$-$C_6$ alkyl or halogen.

**9.** A compound according to claim 8,
wherein
$R^1$ is phenyl substituted with 1 to 3 halogen,
$R^2$ is $C_1$-$C_6$ alkyl which may be substituted with furyl or phenyl optionally substituted with substituent(s) selected from the group consisting of $C_1$-$C_6$ alkyl and halogen,
$R^3$ is phenyl substituted with $C_1$-$C_6$ alkyl or halogen, and
$R^4$ is halogen or $C_1$-$C_6$ alkyl.

**10.** A compound according to claim 9,
wherein

$R^1$ is phenyl substituted with 3 halogen,
$R^2$ is $C_1$-$C_6$ alkyl substituted with furyl or phenyl,
$R^3$ is phenyl substituted with halogen,
$R^4$ is $C_1$-$C_6$ alkyl, and
X is $\theta$.

**11.** N-Benzyl-N-[3-(4-chlorophenyl)-5-methylbenzofuran-2-ylmethyl]-N'-(2,4,6-trifluorophenyl)urea.

**12.** N-[3-(4-Bromophenyl)-5-methylbenzofuran-2-ylmethyl]-N-furfuryl-N'-(2,4,6-trifluorophenyl)urea.

**13.** A process for preparing a compound of the formula:

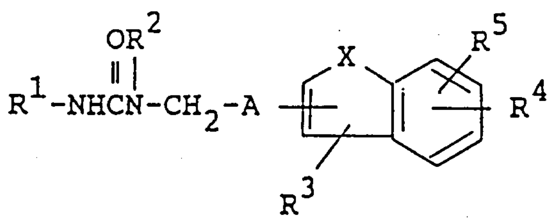

(I)

wherein

$R^1$ is phenyl which may be substituted with $C_1$-$C_6$ alkyl, halogen, nitro, amino, $C_1$-$C_6$ alkylamino, $C_1$-$C_6$ alkoxy or acylamino,
$R^2$ is hydrogen; $C_1$-$C_{20}$ alkyl; cyclo($C_3$-$C_{20}$)alkyl; or $C_1$-$C_6$ alkyl which is substituted with cyclo($C_3$-$C_6$)alkyl, cyclo($C_3$-$C_6$)alkenyl, furyl or phenyl optionally substituted with substituent(s) selected from the group consisting of $C_1$-$C_6$ alkyl, halogen, hydroxy and $C_1$-$C_6$ alkoxy;
$R^3$ is hydrogen, $C_1$-$C_6$ alkyl or phenyl which may be substituted with $C_1$-$C_6$ alkyl, halogen, nitro, amino or $C_1$-$C_6$ alkylamino,
$R^4$ is hydrogen, halogen, $C_1$-$C_6$ alkyl, $C_1$-$C_6$ alkoxy or phenyl which may be substituted with $C_1$-$C_6$ alkyl or halogen,
$R^5$ is hydrogen, halogen, $C_1$-$C_6$ alkyl or phenyl which may be substituted with $C_1$-$C_6$ alkyl,
A is a single bond or $C_1$-$C_6$ alkylene, and
X is O, S or NH,

in which at least one of $R^3$, $R^4$ and $R^5$ is not hydrogen, provided that $R^3$ is phenyl which may be substituted with $C_1$-$C_6$ alkyl, halogen, nitro, amino or $C_1$-$C_6$ alkylamino, or
$R^4$ is halogen, $C_1$-$C_6$ alkoxy or phenyl which may be substituted with $C_1$-$C_6$ alkyl or halogen, when $R^2$ is cyclo($C_3$-$C_{20}$)alkyl,

or pharmaceutically acceptable salts therof, which comprises,
(a) reacting a compound of the formula :

$R^1$-NCO (II)

with a compound of the formula :

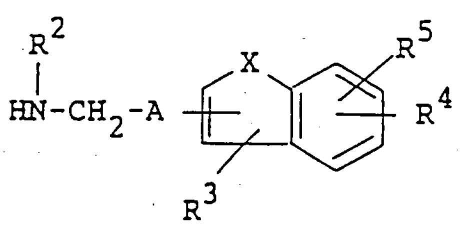

(III)

or its salt to provide a compound of the formula :

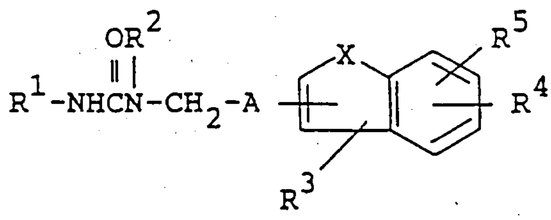

(I)

or its salt, in the above formulas,
$R^1$, $R^2$, $R^3$, $R^4$, $R^5$, A and X are each as defined above, or
(b) subjecting a compound of the formula :

$R^1$-$NH_2$ (IV)

or its salt and a compound of the formula :

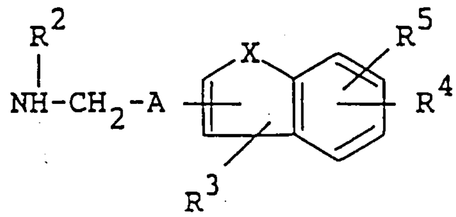

(III)

or its salt to formation reaction of ureido group to provide a compound of the formula :

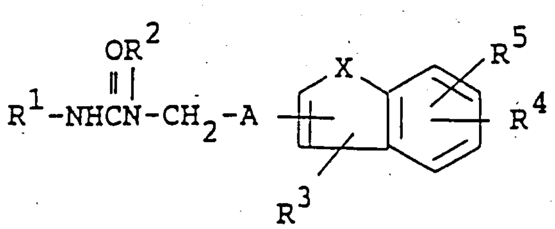

(I)

or its salt, in the above formulas,
$R^1$, $R^2$, $R^3$, $R^4$, $R^5$, A and X are each as defined above, or
(c) subjecting a compound of the formula :

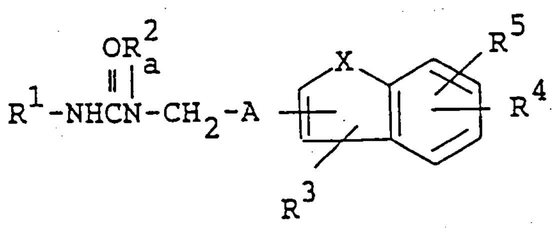

(Ia)

or its salt to dealkylation reaction to provide a compound of the formula :

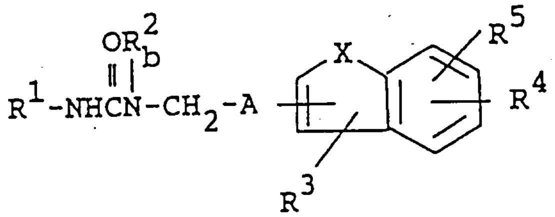

(Ib)

or its salt, in the above formulas,

| | |
|---|---|
| $R^1$, $R^3$, $R^4$, $R^5$, A and X | are each as defined above, |
| $R^2_a$ | is $C_1$-$C_6$ alkyl substituted with phenyl or $C_1$-$C_6$ alkyl-substituted phenyl, each of which is substituted with $C_1$-$C_6$ alkoxy, and |
| $R^2_b$ | is $C_1$-$C_6$ alkyl substituted with phenyl or $C_1$-$C_6$ alkyl-substituted phenyl, each of which is substituted with hydroxy, or |

(d) subjecting a compound of the formula :

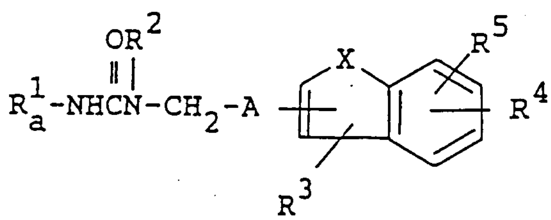

(Ic)

or its salt to reduction
to provide a compound of the formula :

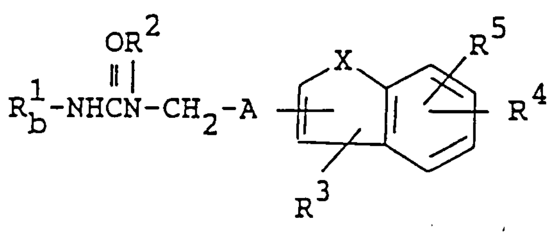

(Id)

or its salt, in the above formulas,

| | |
|---|---|
| $R^2$, $R^3$, $R^4$, $R^5$, A and X | are each as defined above, |
| $R^1_a$ | is phenyl or $C_1$-$C_6$ alkyl-substituted phenyl, each of which is substituted with nitro, and |
| $R^1_b$ | is phenyl or $C_1$-$C_6$ alkyl-substituted phenyl, each of which is substituted with amino, or |

(e) reacting a compound of the formula :

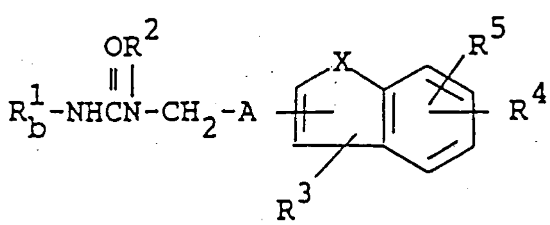

(Id)

or its salt with an acylating agent
to provide a compound of the formula :

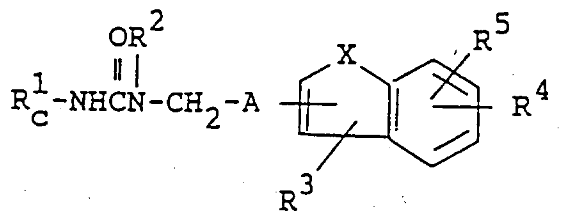 (Ie)

or its salt, in the above formulas,

$R^1_b$, $R^2$, $R^3$, $R^4$, $R^5$, A and X are each as defined above, and

$R^1_c$ is phenyl or $C_1$-$C_6$ alkyl-substituted phenyl, each of which is substituted with acylamino, or

(f) reacting a compound of the formula :

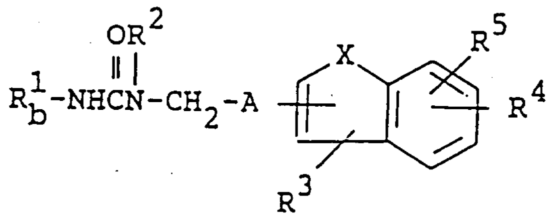 (Id)

or its salt with an alkylating agent

to provide a compound of the formula :

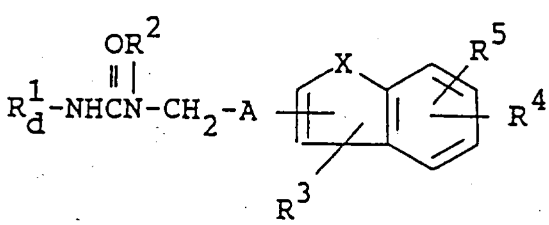 (If)

or its salt, in the above formulas,

$R^1_b$, $R^2$, $R^3$, $R^4$, $R^5$, A and X are each as defined above, and

$R^1_d$ is phenyl or $C_1$-$C_6$ alkyl-substituted phenyl, each of which is substituted with $C_1$-$C_6$ alkylamino.

**14.** A pharmaceutical composition comprising a compound of claim 1, as an active ingredient, in association with a pharmaceutically acceptable, substantially non-toxic carrier or excipient.

**15.** A compound of claim 1 for use as a medicament.

**16.** A compound of claim 1 for use in therapeutic treatment of hypercholesterolemia, hyperlipidemia, atherosclerosis or diseases caused thereby.

**17.** Use of a compound of claim 1 for the manufacture of a medicament for treating hypercholesterolemia, hyperlipidemia, atherosclerosis or diseases caused thereby.

**Claims for the following Contracting States : ES, GR**

**1.** A process for preparing a compound of the formula:

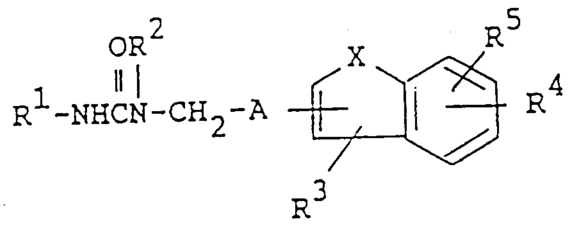

(I)

wherein

$R^1$ is phenyl which may be substituted with $C_1$-$C_6$ alkyl, halogen, nitro, amino, $C_1$-$C_6$ alkylamino, $C_1$-$C_6$ alkoxy or acylamino,

$R^2$ is hydrogen; $C_1$-$C_{20}$ alkyl; cyclo($C_3$-$C_{20}$)alkyl; or $C_1$-$C_6$ alkyl which is substituted with cyclo($C_3$-$C_6$)alkyl, cyclo($C_3$-$C_6$)alkenyl, furyl or phenyl optionally substituted with substituent(s) selected from the group consisting of $C_1$-$C_6$ alkyl, halogen, hydroxy and $C_1$-$C_6$ alkoxy;

$R^3$ is hydrogen, $C_1$-$C_6$ alkyl or phenyl which may be substituted with $C_1$-$C_6$ alkyl, halogen, nitro amino or $C_1$-$C_6$ alkylamino,

$R^4$ is hydrogen, halogen, $C_1$-$C_6$ alkyl, $C_1$-$C_6$ alkoxy or phenyl which may be substituted with $C_1$-$C_6$ alkyl or halogen,

$R^5$ is hydrogen, halogen, $C_1$-$C_6$ alkyl or phenyl which may be substituted with $C_1$-$C_6$ alkyl,

A is a single bond or $C_1$-$C_6$ alkylene, and

X is O, S or NH,

in which at least one of $R^3$, $R^4$ and $R^5$ is not hydrogen, provided that $R^3$ is phenyl which may be substituted with $C_1$-$C_6$ alkyl, halogen, nitro, amino or $C_1$-$C_6$ alkylamino, or

$R^4$ is halogen, $C_1$-$C_6$ alkoxy or phenyl which may be substituted with $C_1$-$C_6$ alkyl or halogen, when $R^2$ is cyclo($C_3$-$C_{20}$)alkyl,

or pharmaceutically acceptable salts therof, which comprises,

(a) reacting a compound of the formula :

$R^1$-NCO (II)

with a compound of the formula :

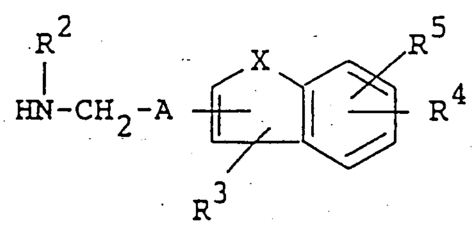

(III)

or its salt to provide a compound of the formula :

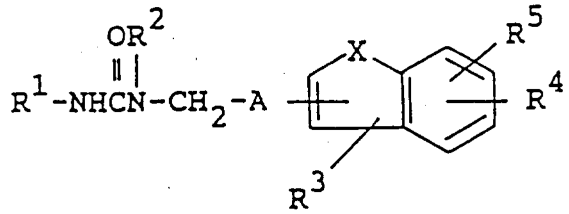

(I)

or its salt, in the above formulas,

$R^1$, $R^2$, $R^3$, $R^4$, $R^5$, A and X are each as defined above, or
(b) subjecting a compound of the formula :

$R^1$-$NH_2$ (IV)

or its salt and a compound of the formula :

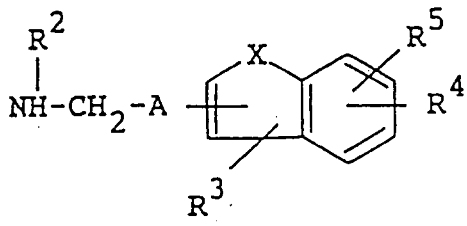

(III)

or its salt to formation reaction of ureido group to provide a compound of the formula :

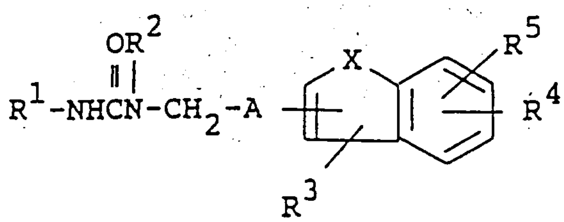

(I)

or its salt, in the above formulas,
$R^1$, $R^2$, $R^3$, $R^4$, $R^5$, A and X are each as defined above, or
(c) subjecting a compound of the formula :

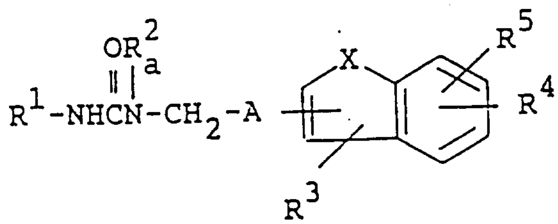

(Ia)

or its salt to dealkylation reaction to provide a compound of the formula :

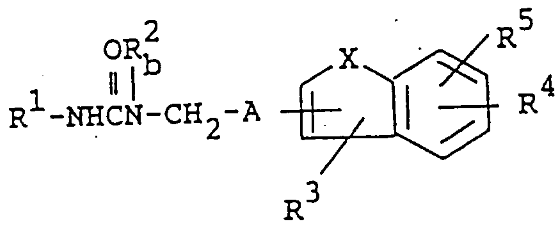

(Ib)

or its salt, in the above formulas,

| | |
|---|---|
| $R^1$, $R^3$, $R^4$, $R^5$, A and X | are each as defined above, |
| $R^2_a$ is | $C_1$-$C_6$ alkyl substituted with phenyl or $C_1$-$C_6$ alkyl-substituted phenyl, each of which is substituted with $C_1$-$C_6$ alkoxy, and |
| $R^2_b$ is | $C_1$-$C_6$ alkyl substituted with phenyl or $C_1$-$C_6$ alkyl-substituted phenyl, each of which is substituted with hydroxy, or |

(d) subjecting a compound of the formula :

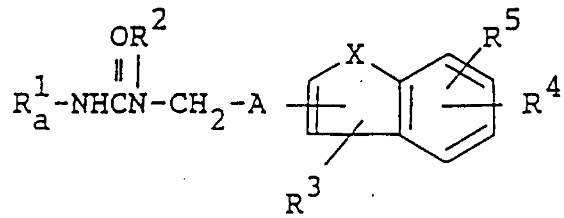

(Ic)

or its salt to reduction
to provide a compound of the formula :

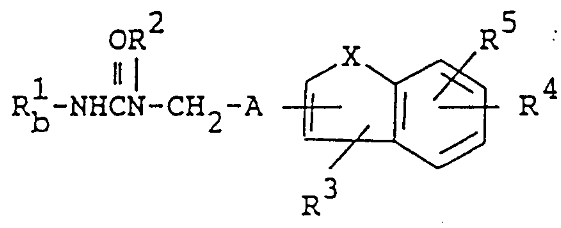

(Id)

or its salt, in the above formulas,

| | |
|---|---|
| $R^2$, $R^3$, $R^4$, $R^5$, A and X | are each as defined above, |
| $R^1_a$ is | phenyl on $C_1$-$C_6$ alkyl-substituted phenyl, each of which is substituted with nitro, and |
| $R^1_b$ is | phenyl or $C_1$-$C_6$ alkyl-substituted phenyl, each of which is substituted with amino, or |

(e) reacting a compound of the formula :

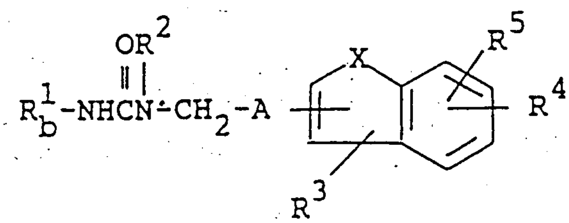

(Id)

or its salt with an acylating agent
to provide a compound of the formula :

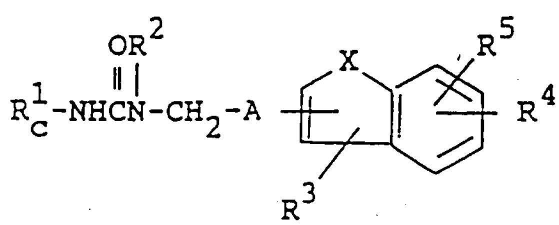

(Ie)

or its salt, in the above formulas,

| | |
|---|---|
| $R^1_b$ , $R^2$, $R^3$, $R^4$, $R^5$, A and X | are each as defined above, and |
| $R^1_c$ is | phenyl or $C_1$-$C_6$ alkyl-substituted phenyl, each of which is substituted with acylamino, or |

(f) reacting a compound of the formula :

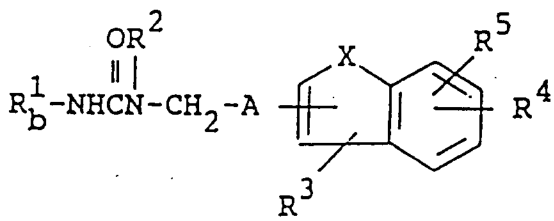

(Id)

or its salt with an alkylating agent
to provide a compound of the formula :

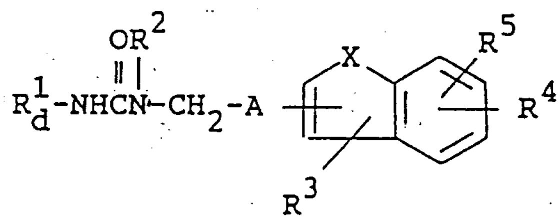

(If)

or its salt, in the above formulas,

| | |
|---|---|
| $R_b^1$ , $R^2$, $R^3$ $R^4$, $R^5$, A and X | are each as defined above, and |
| $R_d^1$ is | phenyl or $C_1$-$C_6$ alkyl-substituted phenyl, each of which is substituted with $C_1$-$C_6$ alkylamino. |

**2.** A process according to claim 1,
wherein
$R^2$ is hydrogen; $C_1$-$C_{20}$ alkyl; cyclo($C_3$-$C_{20}$)alkyl; or $C_1$-$C_6$ alkyl which is substituted with cyclo($C_3$-$C_6$)alkyl, furyl or phenyl optionally substituted with substituent(s) selected from the group consisting of $C_1$-$C_6$ alkyl, halogen, hydroxy and $C_1$-$C_6$ alkoxy.

**3.** A process according to claim 2,
wherein
$R^1$ is phenyl which may be substituted with $C_1$-$C_6$ alkyl, halogen, nitro, amino, $C_1$-$C_6$ alkylamino or $C_1$-$C_6$ alkoxy,
$R^2$ is $C_1$-$C_{20}$ alkyl; cyclo($C_3$-$C_{20}$)alkyl; or $C_1$-$C_6$ alkyl which is substituted with cyclo($C_3$-$C_6$)alkyl, furyl or phenyl optionally substituted with substituent(s) selected from the group consisting of $C_1$-$C_6$ alkyl, halogen, hydroxy and $C_1$-$C_6$ alkoxy, and
$R^5$ is hydrogen.

**4.** A process according to claim 3,
wherein
$R^2$ is $C_1$-$C_{20}$ alkyl; cyclo($C_3$-$C_{20}$)alkyl; or $C_1$-$C_6$ alkyl which is substituted with phenyl optionally substituted with $C_1$-$C_6$ alkyl.

**5.** A process according to claim 3,
wherein
$R^1$ is phenyl which may be substituted with $C_1$-$C_6$ alkyl, halogen or $C_1$-$C_6$ alkoxy, and
X is O or S.

**6.** A process according to claim 5,
wherein
$R^3$ is hydrogen or phenyl which may be substituted with $C_1$-$C_6$ alkyl or halogen, and
$R^4$ is hydrogen, halogen, $C_1$-$C_6$ alkyl or phenyl which may be substitued with $C_1$-$C_6$ alkyl or halogen.

**7.** A process according to claim 6, wherein
$R^2$ is $C_1$-$C_{20}$ alkyl; cyclo($C_3$-$C_{20}$)alkyl; or $C_1$-$C_6$ alkyl which is substituted with phenyl optionally substituted with $C_1$-$C_6$ alkyl.

**8.** A process according to claim 6, wherein
$R^1$ is phenyl which is substituted with 1 to 3 $C_1$-$C_6$ alkyl, halogen or $C_1$-$C_6$ alkoxy substituent(s).
$R^2$ is $C_1$-$C_{20}$ alkyl; cyclo($C_3$-$C_{20}$)alkyl; or $C_1$-$C_6$ alkyl which is substituted with cyclo($C_3$-$C_6$)alkyl, furyl or phenyl optionally substituted with substituent(s) selected from the group consisting of $C_1$-$C_6$ alkyl, halogen, hydroxy and $C_1$-$C_6$ alkoxy; and
$R^3$ is phenyl which may be substituted with $C_1$-$C_6$ alkyl or halogen; or
$R^1$ and $R^2$ are each defined above, and
$R^4$ is halogen or phenyl which may be substituted with $C_1$-$C_6$ alkyl or halogen.

**9.** A process according to claim 8, wherein
$R^1$ is phenyl substituted with 1 to 3 halogen,
$R^2$ is $C_1$-$C_6$ alkyl which may be substituted with furyl or phenyl optionally substituted with substituent(s) selected from the group consisting of $C_1$-$C_6$ alkyl and halogen,
$R^3$ is phenyl substituted with $C_1$-$C_6$ alkyl or halogen, and
$R^4$ is halogen or $C_1$-$C_6$ alkyl.

**10.** A process according to claim 9, wherein
$R^1$ is phenyl substituted with 3 halogen,
$R^2$ is $C_1$-$C_6$ alkyl substituted with furyl or phenyl,
$R^3$ is phenyl substituted with halogen,
$R^4$ is $C_1$-$C_6$ alkyl, and
X is *θ*.

**11.** A process according to claim 1 for preparing N-Benzyl-N-[3-(4-chlorophenyl)-5-methylbenzofuran-2-ylmethyl]-N'-(2,4,6-trifluorophenyl)urea.

**12.** A process according to claim 1 for preparing N-[3-(4-Bromophenyl)-5-methylbenzofuran-2-ylmethyl]-N-furfuryl-N'-(2,4,6-trifluorophenyl)urea.

**13.** Modification of the process according to claim 1, wherein a compound of the formula (I) or a pharmaceutical acceptable salt thereof is brought into a pharmaceutical acceptable form, by admixture or dilution of said compound (I) with a pharmaceutical acceptable carrier or diluent.

## Patentansprüche

## Patentansprüche für folgende Vertragsstaaten : AT, BE, CH, LI, DE, DK, FR, GB, IT, LU, NL, PT, SE

**1.** Verbindung der Formel:

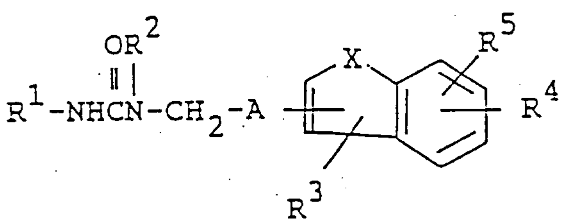

(I)

worin $R^1$ Phenyl ist, das mit ($C_1$-$C_6$)Alkyl, Halogen, Nitro, Amino, ($C_1$-$C_6$)Alkylamino, ($C_1$-$C_6$)Alkoxy oder Acylamino substituiert sein kann,
$R^2$ Wasserstoff; ($C_1$-$C_{20}$)Alkyl; Cyclo($C_3$-$C_{20}$)alkyl; oder ($C_1$-$C_6$)Alkyl ist, das substituiert ist, mit Cyclo-($C_3$-$C_6$)-alkyl, Cyclo($C_3$-$C_6$)alkenyl, Furyl oder Phenyl, das wahlweise mit Substituent(en) substituiert ist, ausgewählt aus der Gruppe, bestehend aus ($C_1$-$C_6$)Alkyl, Halogen, Hydroxy und ($C_1$-$C_6$)-Alkoxy;
$R^3$ Wasserstoff, ($C_1$-$C_6$)Alkyl oder Phenyl ist, das mit ($C_1$-$C_6$)Alkyl, Halogen, Nitro, Amino oder ($C_1$-$C_6$)-

Alkylamino substituiert sein kann,
$R^4$ Wasserstoff, Halogen, $(C_1-C_6)$Alkyl, $(C_1-C_6)$Alkoxy oder Phenyl ist, das mit $(C_1-C_6)$Alkyl oder Halogen substituiert sein kann,
$R^5$ Wasserstoff, Halogen, $(C_1-C_6)$Alkyl oder Phenyl ist, das mit $(C_1-C_6)$Alkyl substituiert sein kann,
A eine Einfachbindung oder $(C_1-C_6)$Alkylen ist, und X ist O, S oder NH,
worin mindestens eines von $R^3$, $R^4$ und $R^5$ nicht Wasserstoff ist,
mit der Maßgabe, daß
$R^3$ Phenyl ist, das mit $(C_1-C_6)$Alkyl, Halogen, Nitro, Amino oder $(C_1-C_6)$Alkylamino substituiert sein kann, oder
$R^4$ Halogen, $(C_1-C_6)$Alkoxy oder Phenyl ist, das mit $(C_1-C_6)$-Alkyl oder Halogen substituiert sein kann,
wenn $R^2$ Cyclo$(C_3-C_{20})$alkyl ist,
und pharmazeutisch verträgliche Salze davon.

**2.** Verbindung nach Anspruch 1, worin $R^2$ Wasserstoff; $(C_1-C_{20})$-Alkyl; Cyclo$(C_3-C_{20})$alkyl oder $(C_1-C_6)$-Alkyl ist, das substituiert ist, mit Cyclo$(C_3-C_6)$alkyl, Furyl oder Phenyl, das wahlweise mit Substituent(en) substituiert ist, ausgewählt aus der Gruppe, bestehend aus $(C_1-C_6)$Alkyl,Halogen, Hydroxy und $(C_1-C_6)$Alkoxy.

**3.** Verbindung nach Anspruch 2, worin $R^1$ Phenyl ist, das mit $(C_1-C_6)$Alkyl, Halogen, Nitro, Amino, $(C_1-C_6)$-alkylamino oder $(C_1-C_6)$Alkoxy substituiert sein kann,
$R^2$ $(C_1-C_{20})$Alkyl; Cyclo$(C_3-C_{20})$alkyl; oder $(C_1-C_6)$Alkyl ist, das substituiert ist, mit Cyclo$(C_3-C_6)$alkyl, Furyl oder Phenyl, das wahlweise mit Substituent(en) substituiert ist, die aus der Gruppe ausgewählt sind, die aus $(C_1-C_6)$-Alkyl, Halogen, Hydroxy und $(C_1-C_6)$Alkoxy besteht, und
$R^5$ Wasserstoff ist

**4.** Verbindung nach Anspruch 3, worin $R^2$ $(C_1-C_{20})$Alkyl; Cyclo$(C_3-C_{20})$alkyl; oder $(C_1-C_6)$Alkyl ist, das mit Phenyl substituiert ist, das wahlweise mit $(C_1-C_6)$Alkyl substituiert ist.

**5.** Verbindung nach Anspruch 3, worin $R_1$ Phenyl ist, das mit $(C_1-C_6)$Alkyl, Halogen oder $(C_1-C_6)$Alkoxy substituiert sein kann, und X ist O oder S.

**6.** Verbindung nach Anspruch 5, worin $R^3$ Wasserstoff oder Phenyl ist, das mit $(C_1-C_6)$Alkyl oder Halogen substituiert sein kann, und $R^4$ Wasserstoff, Halogen, $(C_1-C_6)$Alkyl oder Phenyl ist, das mit $(C_1-C_6)$Alkyl oder Halogen substituiert ist.

**7.** Verbindung nach Anspruch 6, worin $R^2$ $(C_1-C_{20})$Alkyl; Cyclo$(C_3-C_{20})$alkyl; oder $(C_1-C_6)$Alkyl ist, das mit Phenyl substituiert ist, das wahlweise mit $(C_1-C_6)$Alkyl substituiert ist.

**8.** Verbindung nach Anspruch 6, worin $R^1$ Phenyl ist, das mit 1 bis 3 $(C_1-C_6)$Alkyl-, Halogen- oder $(C_1-C_6)$-Alkoxy-Substituent(en) substituiert ist,
$R^2$ $(C_1-C_{20})$Alkyl; Cyclo$(C_3-C_{20})$alkyl; oder $(C_1-C_6)$Alkyl ist, das substitutiert ist, mit Cyclo$(C_3-C_6)$alkyl, Furyl oder Phenyl, das wahlweise mit Substituent(en) substituiert ist, die aus der Gruppe ausgewählt sind, die aus $(C_1-C_6)$-Alkyl, Halogen, Hydroxy und $(C_1-C_6)$Alkoxy besteht; und
$R^3$ Phenyl ist, das mit $(C_1-C_6)$Alkyl oder Halogen substituiert sein kann;
oder $R^1$ und $R^2$ wie oben definiert sind, und
$R^4$ Halogen oder Phenyl ist, das mit $(C_1-C_6)$Alkyl oder Halogen substituiert sein kann.

**9.** Verbindung nach Anspruch 8, worin $R^1$ Phenyl, substituiert mit 1 bis 3 Halogen(en) ist,
$R^2$ $(C_1-C_6)$Alkyl ist, das substituiert sein kann, mit Furyl oder Phenyl, das wahlweise mit Substituent(en) substituiert ist, die aus der Gruppe ausgewählt sind, die aus $(C_1-C_6)$-Alkyl und Halogen besteht,
$R^3$ Phenyl ist, das mit $(C_1-C_6)$Alkyl oder Halogen substituiert ist, oder
$R^4$ Halogen oder $(C_1-C_6)$Alkyl ist.

**10.** Verbindung nach Anspruch 9, worin $R^1$ Phenyl, substituiert mit 3 Halogenen, ist,
$R^2$ $(C_1-C_6)$Alkyl ist, das mit Furyl oder Phenyl substituiert ist,
$R^3$ Phenyl, substituiert mit Halogen, ist,
$R^4$ $(C_1-C_6)$Alkyl ist,
und X ist O.

**11.** N-Benzyl-N-[3-(4-chlorphenyl)-5-methylbenzofuran-2-ylmethyl]-N'-(2,4,6-trifluorphenyl)harnstoff.

**12.** N-[3-(4-Bromphenyl)-5-methylbenzofuran-2-ylmethyl]-N-furfuryl-N'-(2,4,6-trifluorphenyl)harnstoff.

**13.** Verfahren zur Herstellung einer Verbindung der Formel:

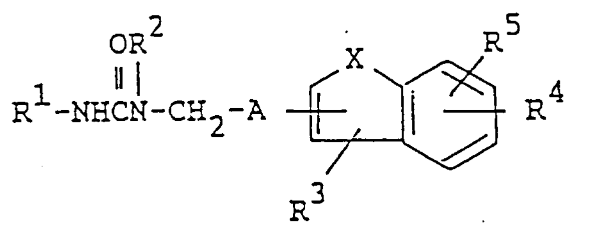

(I)

worin $R^1$ Phenyl ist, das mit $(C_1\text{-}C_6)$Alkyl, Halogen, Nitro, Amino, $(C_1\text{-}C_6)$Alkylamino, $(C_1\text{-}C_6)$Alkoxy oder Acylamino substituiert sein kann,
$R^2$ Wasserstoff; $(C_1\text{-}C_{20})$Alkyl; Cyclo$(C_3\text{-}C_{20})$alkyl; oder $(C_1\text{-}C_6)$Alkyl ist, das substituiert ist, mit Cyclo-$(C_3\text{-}C_6)$-alkyl, Cyclo$(C_3\text{-}C_6)$alkenyl, Furyl oder Phenyl, das wahlweise mit Substituent(en) substituiert ist, ausgewählt aus der Gruppe, bestehend aus $(C_1\text{-}C_6)$Alkyl, Halogen, Hydroxy und $(C_1\text{-}C_6)$-Alkoxy;
$R^3$ Wasserstoff, $(C_1\text{-}C_6)$Alkyl oder Phenyl ist, das mit $(C_1\text{-}C_6)$Alkyl, Halogen, Nitro, Amino oder $(C_1\text{-}C_6)$-Alkylamino substituiert sein kann,
$R^4$ Wasserstoff, Halogen, $(C_1\text{-}C_6)$Alkyl, $(C_1\text{-}C_6)$Alkoxy oder Phenyl ist, das mit $(C_1\text{-}C_6)$Alkyl oder Halogen substituiert sein kann,
$R^5$ Wasserstoff, Halogen, $(C_1\text{-}C_6)$Alkyl oder Phenyl ist, das mit $(C_1\text{-}C_6)$Alkyl substituiert sein kann,
A eine Einfachbindung oder $(C_1\text{-}C_6)$Alkylen ist und
X ist O, S oder NH,
worin mindestens eines von $R^3$, $R^4$ und $R^5$ nicht Wasserstoff ist,
mit der Maßgabe, daß
$R^3$ Phenyl ist, das mit $(C_1\text{-}C_6)$Alkyl, Halogen, Nitro, Amino oder $(C_1\text{-}C_6)$Alkylamino substituiert sein kann, oder
$R^4$ Halogen, $(C_1\text{-}C_6)$Alkoxy oder Phenyl ist, das mit $(C_1\text{-}C_6)$-Alkyl oder Halogen substituiert sein kann, wenn $R^2$ Cyclo$(C_3\text{-}C_{20})$alkyl ist,
oder pharmazeutisch verträglichen Salzen davon, das umfaßt,

(a) Reagieren einer Verbindung der Formel:

$R^1$-NCO (II)

mit einer Verbindung der Formel:

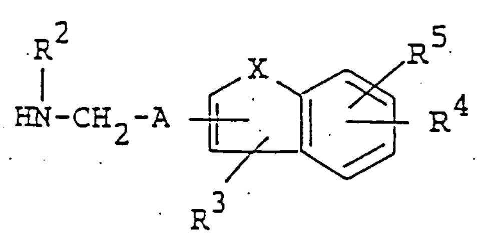

(III)

oder ihres Salzes, um eine Verbindung der Formel:

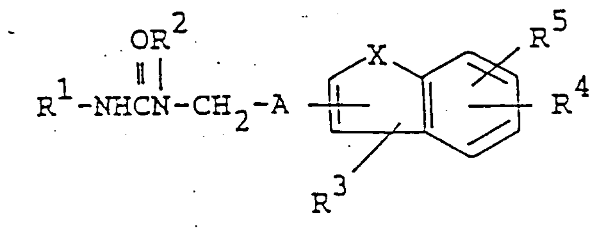

(I)

oder ihr Salz bereitzustellen, wobei in den obigen Formeln $R^1$, $R^2$, $R^3$, $R^4$ und $R^5$ und X jeweils die oben definiert sind, oder

(b) Unterwerfen einer Verbindung der Formel:

$R^1$-$NH_2$ (IV)

oder ihres Salzes und eine Verbindung der Formel:

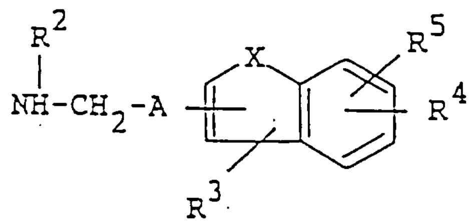

(III)

oder ihr Salz der Bildungsreaktion einer Ureidogruppe, um eine Verbindung der Formel:

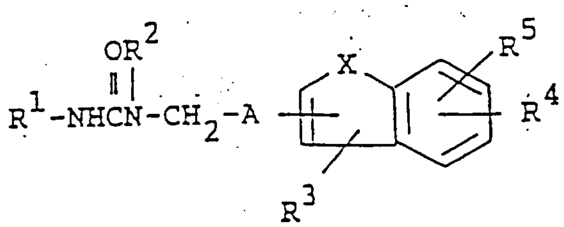

(I)

oder ihr Salz bereitzustellen, wobei in den obigen Formeln $R^1$, $R^2$, $R^3$, $R^4$, $R^5$, A und X jeweils wie oben definiert sind, oder

(c) Unterwerfen einer Verbindung der Formel:

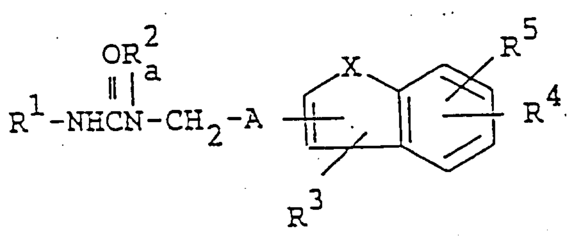

(Ia)

oder ihres Salzes der Dealkylierungsreaktion, um eine Verbindung der Formel:

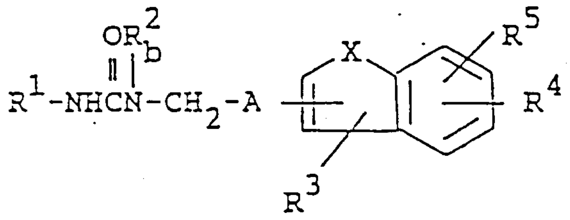

(Ib)

oder ihr Salz bereitzustellen, wobei in den obigen Formeln $R^1$, $R^3$, $R^4$, $R^5$, A und X jeweils wie oben definiert sind,

$R^2{}_a$ $(C_1\text{-}C_6)$Alkyl ist, das substituiert ist, mit Phenyl oder $(C_1\text{-}C_6)$alkylsubstituiertem Phenyl, wovon jedes mit $(C_1\text{-}C_6)$Alkoxy substituiert ist, und

$R^2{}_b$ $(C_1\text{-}C_6)$Alkyl ist, das substituiert ist, mit Phenyl oder $(C_1\text{-}C_6)$alkylsubstituiertem Phenyl, wovon jedes mit Hydroxy substituiert ist, oder

(d) Unterwerfen einer Verbindung der Formel:

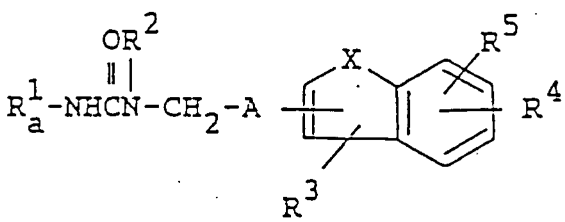

(Ic)

oder Ihres Salzes der Reduktion, um eine Verbindung der Formel:

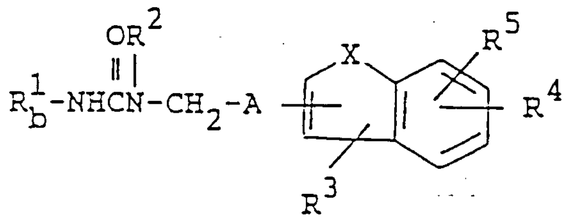

(Id)

oder ihr Salz bereitzustellen, wobei in den obigen Formeln $R^2$, $R^3$, $R^4$, $R^5$, A und X jeweils wie oben definiert sind,

$R^1{}_a$ Phenyl oder $(C_1\text{-}C_6)$alkyl-substituiertes Phenyl ist, wovon jedes mit Nitro substituiert ist, und

$R^1{}_b$ Phenyl oder $(C_1\text{-}C_6)$alkyl-substituiertes Phenyl ist, wovon jedes mit Amino substituiert ist, oder

(e) Reagieren einer Verbindung der Formel:

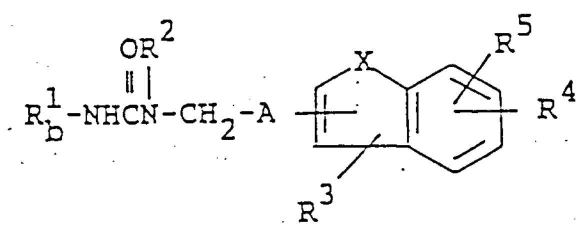

(Id)

oder ihres Salzes mit einem Acylierungsmittel, um eine Verbindung der Formel:

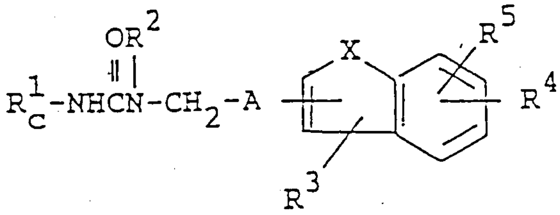

(Ie)

oder ihr Salz bereitzustellen, wobei in den obigen Formeln $R^1_b$ $R^2$, $R^3$, $R^4$, $R^5$, A und X jeweils wie oben definiert sind, und

$R^1_c$ Phenyl oder $(C_1\text{-}C_6)$alkylsubstituiertes Phenyl ist, wovon jedes mit Acylamino substituiert ist, oder

(f) Reagieren einer Verbindung der Formel:

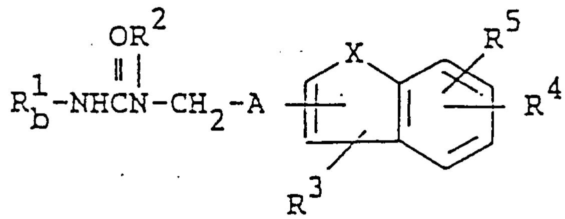

(Id)

oder ihres Salzes mit einem Alkylierungsmittel, um eine Verbindung der Formel:

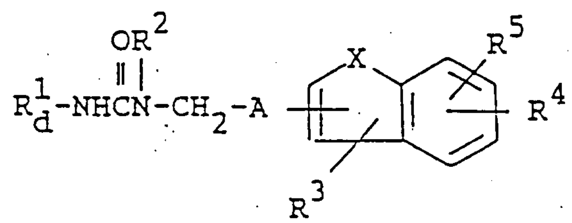

(If)

oder ihr Salz bereitzustellen, wobei in den obigen Formeln $R^1_b$, $R^2$, $R^3$, $R^4$, $R^5$, A und X jeweils wie oben definiert sind, und

$R^1_d$ Phenyl oder $(C_1\text{-}C_6)$alkylsubstituiertes Phenyl ist, wovon jedes mit $(C_1\text{-}C_6)$Alkylamino substituiert ist.

**14.** Pharmazeutische Zusammensetzung, die eine Verbindung nach Anspruch 1 als aktiven Bestandteil in Assoziierung mit einem pharmazeutisch verträglichen - im wesentlichen nicht giftigen - Träger oder Exzipienten umfaßt.

**15.** Verbindung nach Anspruch 1 zur Verwendung als Medikament.

**16.** Verbindung nach Anspruch 1 zur Verwendung in der therapeutischen Behandlung von Hypercholesterinämie, Hyperlipidämie, Atherosklerose oder dadurch verursachte Krankheiten.

**17.** Verwendung einer Verbindung nach Anspruch 1 zur Herstellung eines Medikamentes zur Behandlung von Hypercholesterinämie, Hyperlipidämie, Atherosklerose oder dadurch verursachte Krankheiten.

**Patentansprüche für folgende Vertragsstaaten : ES, GR**

**1.** Verfahren zur Herstellung einer Verbindung der Formel:

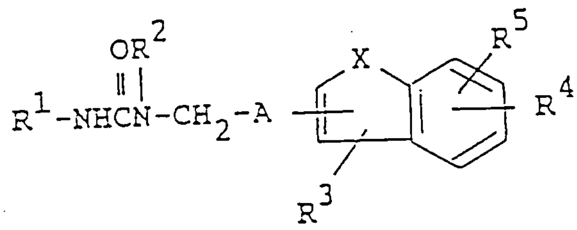

(I)

worin $R^1$ Phenyl ist, das mit $(C_1\text{-}C_6)$Alkyl, Halogen, Nitro, Amino, $(C_1\text{-}C_6)$Alkylamino, $(C_1\text{-}C_6)$Alkoxy oder Acylamino substituiert sein kann,
$R^2$ Wasserstoff; $(C_1\text{-}C_{20})$Alkyl; Cyclo$(C_3\text{-}C_{20})$alkyl; oder $(C_1\text{-}C_6)$Alkyl ist, das substituiert ist, mit Cyclo-$(C_3\text{-}C_6)$-alkyl, Cyclo$(C_3\text{-}C_6)$alkenyl, Furyl oder Phenyl, das wahlweise mit Substituent(en) substituiert ist, ausgewählt aus der Gruppe, bestehend aus $(C_1\text{-}C_6)$Alkyl, Halogen, Hydroxy und $(C_1\text{-}C_6)$-Alkoxy;
$R^3$ Wasserstoff, $(C_1\text{-}C_6)$Alkyl oder Phenyl ist, das mit $(C_1\text{-}C_6)$Alkyl, Halogen, Nitro, Amino oder $(C_1\text{-}C_6)$-Alkylamino substituiert sein kann,
$R^4$ Wasserstoff, Halogen, $(C_1\text{-}C_6)$Alkyl, $(C_1\text{-}C_6)$Alkoxy oder Phenyl ist, das mit $(C_1\text{-}C_6)$Alkyl oder Halogen substituiert sein kann,
$R^5$ Wasserstoff, Halogen, $(C_1\text{-}C_6)$Alkyl oder Phenyl ist, das mit $(C_1\text{-}C_6)$Alkyl substituiert sein kann,
A eine Einfachbindung oder $(C_1\text{-}C_6)$Alkylen ist und X ist O, S oder NH,
worin mindestens eines von $R^3$, $R^4$ und $R^5$ nicht Wasserstoff ist,
mit der Maßgabe, daß
$R^3$ Phenyl ist, das mit $(C_1\text{-}C_6)$Alkyl, Halogen, Nitro, Amino oder $(C_1\text{-}C_6)$Alkylamino substituiert sein kann, oder
$R^4$ Halogen, $(C_1\text{-}C_6)$Alkoxy oder Phenyl ist, das mit $(C_1\text{-}C_6)$-Alkyl oder Halogen substituiert sein kann,
wenn $R^2$ Cyclo$(C_3\text{-}C_{20})$alkyl ist,
oder ihren pharmazeutisch verträglichen Salze, das umfaßt,
(a) Reagieren einer Verbindung der Formel:

$R^1$-NCO (II)

mit einer Verbindung der Formel:

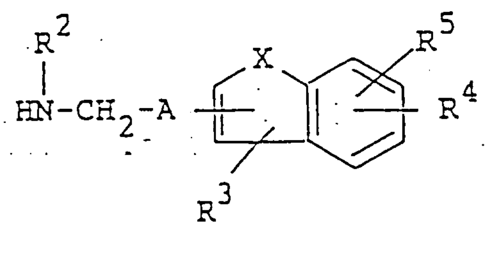

(III)

oder ihres Salzes, um eine Verbindung der Formel:

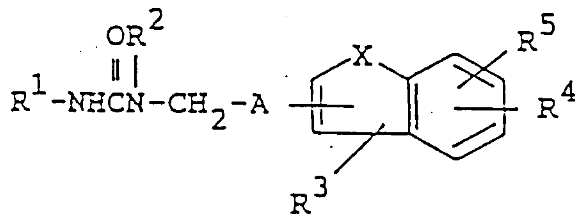

(I)

oder ihr Salz bereitzustellen, wobei in den obigen Formeln $R^1$, $R^2$, $R^3$, $R^4$ und $R^5$ und X jeweils die

oben definiert sind, oder

(b) Unterwerfen einer Verbindung der Formel:

$R^1$-$NH_2$ (IV)

oder ihres Salzes und eine Verbindung der Formel:

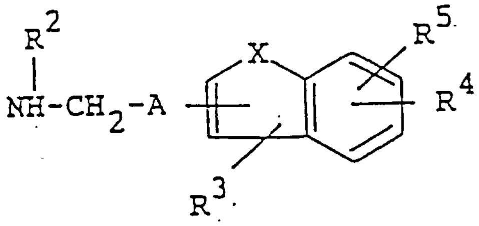

(III)

oder ihr Salz der Bildungsreaktion einer Ureidogruppe, um eine Verbindung der Formel:

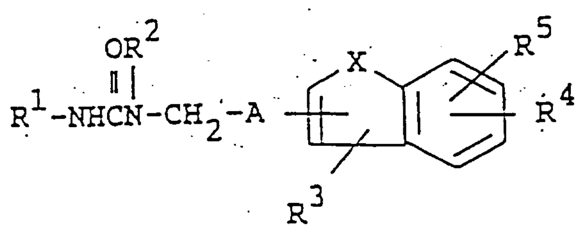

(I)

oder ihr Salz bereitzustellen, wobei in den obigen Formeln $R^1$, $R^2$, $R^3$, $R^4$, $R^5$, A und X jeweils wie oben definiert sind, oder

(c) Unterwerfen einer Verbindung der Formel:

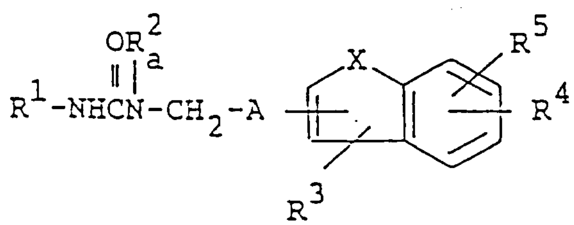

(Ia)

oder ihres Salzes der Dealkylierungsreaktion, um eine Verbindung der Formel:

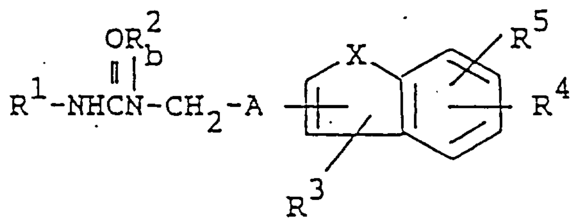

(Ib)

oder ihr Salz bereitzustellen, wobei in den obigen Formeln $R^1$, $R^3$, $R^4$, $R^5$, A und X jeweils wie oben definiert sind,

$R^2_a$ ($C_1$-$C_6$)Alkyl ist, das substituiert ist, mit Phenyl oder ($C_1$-$C_6$)alkylsubstituiertem Phenyl, wovon jedes mit ($C_1$-$C_6$)Alkoxy substituiert ist, und

$R^2_b$ ($C_1$-$C_6$)Alkyl ist, das substituiert ist, mit Phenyl oder ($C_1$-$C_6$)alkylsubstituiertem Phenyl, wovon jedes mit Hydroxy substituiert ist, oder

(d) Unterwerfen einer Verbindung der Formel:

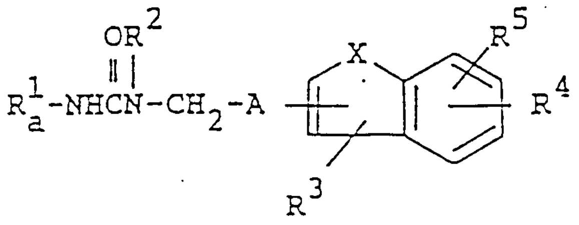

(Ic)

oder Ihres Salzes der Reduktion, um eine Verbindung der Formel:

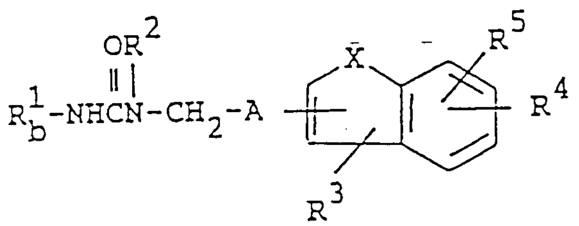

(Id)

oder ihr Salz bereitzustellen, wobei in den obigen Formeln $R^2$, $R^3$, $R^4$, $R^5$, A und X jeweils wie oben definiert sind,
$R^1_a$ Phenyl oder ($C_1$-$C_6$)alkyl-substituiertes Phenyl ist, wovon jedes mit Nitro substituiert ist, und
$R^1_b$ Phenyl oder ($C_1$-$C_6$)alkyl-substituiertes Phenyl ist, wovon jedes mit Amino substituiert ist, oder
(e) Reagieren einer Verbindung der Formel:

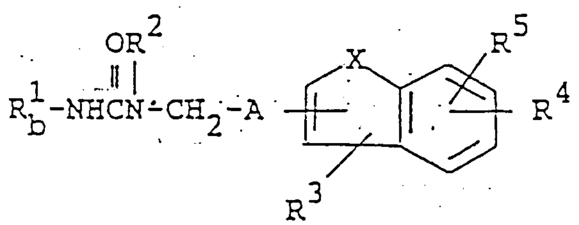

(Id)

oder ihres Salzes mit einem Acylierungsmittel, um eine Verbindung der Formel:

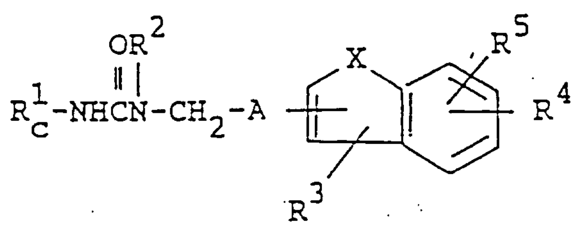

(Ie)

oder ihr Salz bereitzustellen, wobei in den obigen Formeln $R^1_b$ $R^2$, $R^3$, $R^4$, $R^5$, A und X jeweils wie oben definiert sind, und
$R^1_c$ Phenyl oder ($C_1$-$C_6$)alkylsubstituiertes Phenyl ist, wovon jedes mit Acylamino substituiert ist, oder

(f) Reagieren einer Verbindung der Formel:

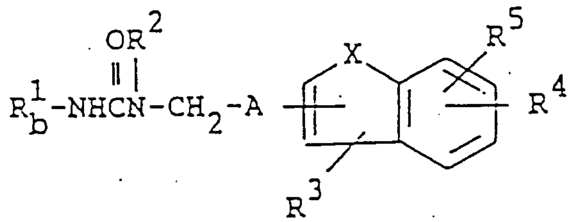

(Id)

oder ihres Salzes mit einem Alkylierungsmittel, um eine Verbindung der Formel:

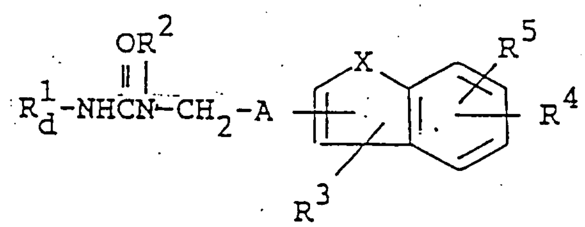

(If)

oder ihr Salz bereitzustellen, wobei in den obigen Formeln $R^1{}_b$, $R^2$, $R^3$, $R^4$, $R^5$, A und X jeweils wie oben definiert sind, und
$R^1{}_d$ Phenyl oder $(C_1\text{-}C_6)$alkylsubstituiertes Phenyl ist, wovon jedes mit $(C_1\text{-}C_6)$Alkylamino substituiert ist.

2. Verfahren nach Anspruch 1, worin $R^2$ Wasserstoff; $(C_1\text{-}C_{20})$-Alkyl; Cyclo$(C_3\text{-}C_{20})$alkyl oder $(C_1\text{-}C_6)$Alkyl ist, das substituiert ist, mit Cyclo$(C_3\text{-}C_6)$alkyl, Furyl oder Phenyl, das wahlweise mit Substituent(en) substituiert ist, ausgewählt aus der Gruppe, bestehend aus $(C_1\text{-}C_6)$Alkyl,Halogen, Hydroxy und $(C_1\text{-}C_6)$-Alkoxy.

3. Verfahren nach Anspruch 2, worin $R^1$ Phenyl ist, das mit $(C_1\text{-}C_6)$Alkyl, Halogen, Nitro, Amino, $(C_1\text{-}C_6)$-Alkylamino oder $(C_1\text{-}C_6)$Alkoxy substituiert sein kann,
$R^2$ $(C_1\text{-}C_{20})$Alkyl; Cyclo$(C_3\text{-}C_{20})$alkyl; oder $(C_1\text{-}C_6)$Alkyl ist, das substituiert ist, mit Cyclo$(C_3\text{-}C_6)$alkyl, Furyl oder Phenyl, das wahlweise mit Substituent(en) substituiert ist, die aus der Gruppe ausgewählt sind, die aus $(C_1\text{-}C_6)$-Alkyl, Halogen, Hydroxy und $(C_1\text{-}C_6)$Alkoxy besteht, und $R^5$ Wasserstoff ist

4. Verfahren nach Anspruch 3, worin $R^2$ $(C_1\text{-}C_{20})$Alkyl; Cyclo$(C_3\text{-}C_{20})$alkyl; oder $(C_1\text{-}C_6)$Alkyl ist, das mit Phenyl substituiert ist, das wahlweise mit $(C_1\text{-}C_6)$Alkyl substituiert ist.

5. Verfahren nach Anspruch 3, worin $R_1$ Phenyl ist, das mit $(C_1\text{-}C_6)$Alkyl, Halogen oder $(C_1\text{-}C_6)$Alkoxy substituiert sein kann, und X ist O oder S.

6. Verfahren nach Anspruch 5, worin $R^3$ Wasserstoff oder Phenyl ist, das mit $(C_1\text{-}C_6)$Alkyl oder Halogen substituiert sein kann, und $R^4$ Wasserstoff, Halogen, $(C_1\text{-}C_6)$Alkyl oder Phenyl ist, das mit $(C_1\text{-}C_6)$Alkyl oder Halogen substituiert ist.

7. Verfahren nach Anspruch 6, worin $R^2$ $(C_1\text{-}C_{20})$Alkyl; Cyclo$(C_3\text{-}C_{20})$alkyl; oder $(C_1\text{-}C_6)$Alkyl ist, das mit Phenyl substituiert ist, das wahlweise mit $(C_1\text{-}C_6)$Alkyl substituiert ist.

8. Verfahren nach Anspruch 6, worin $R^1$ Phenyl ist, das mit 1 bis 3 $(C_1\text{-}C_6)$Alkyl-, Halogen- oder $(C_1\text{-}C_6)$-Alkoxy-Substituent(en) substituiert ist,
$R^2$ $(C_1\text{-}C_{20})$Alkyl; Cyclo$(C_3\text{-}C_{20})$alkyl; oder $(C_1\text{-}C_6)$Alkyl ist, das substitutiert ist, mit Cyclo$(C_3\text{-}C_6)$alkyl, Furyl oder Phenyl, das wahlweise mit Substituent(en) substituiert ist, die aus der Gruppe ausgewählt sind, die aus $(C_1\text{-}C_6)$-Alkyl, Halogen, Hydroxy und $(C_1\text{-}C_6)$Alkoxy besteht; und
$R^3$ Phenyl ist, das mit $(C_1\text{-}C_6)$Alkyl oder Halogen substituiert sein kann;
oder $R^1$ und $R^2$ wie oben definiert sind, und

$R^4$ Halogen oder Phenyl ist, das mit ($C_1$-$C_6$)Alkyl oder Halogen substituiert sein kann.

**9.** Verfahren nach Anspruch 8, worin $R^1$ Phenyl, substituiert mit 1 bis 3 Halogen(en) ist,
$R^2$ ($C_1$-$C_6$)Alkyl ist, das substituiert sein kann, mit Furyl oder Phenyl, das wahlweise mit Substituent(en) substituiert ist, die aus der Gruppe ausgewählt sind, die aus ($C_1$-$C_6$)-Alkyl und Halogen besteht,
$R^3$ Phenyl ist, das mit ($C_1$-$C_6$)Alkyl oder Halogen substituiert ist, oder
$R^4$ Halogen oder ($C_1$-$C_6$)Alkyl ist.

**10.** Verfahren nach Anspruch 9, worin $R^1$ Phenyl, substituiert mit 3 Halogenen, ist,
$R^2$ ($C_1$-$C_6$)Alkyl ist, das mit Furyl oder Phenyl substituiert ist,
$R^3$ Phenyl, substituiert mit Halogen, ist,
$R^4$ ($C_1$-$C_6$)Alkyl ist,
und X ist O.

**11.** Verfahren nach Anspruch 1 zur Herstellung von N-Benzyl-N-[3-(4-chlorphenyl)-5-methylbenzofuran-2-ylmethyl]-N'-(2,4,6-trifluorphenyl)harnstoff.

**12.** Verfahren nach Anspruch 1 zur Herstellung von N-[3-(4-Bromphenyl)-5-methylbenzofuran-2-ylmethyl]-N-furfuryl-N'-(2,4,6-trifluorphenyl)harnstoff.

**13.** Modifizierung des Verfahrens nach Anspruch 1, worin eine Verbindung der Formel (I) oder ein pharmazeutisch verträgliches Salz davon durch Mischen oder Verdünnen dieser Verbindung (I) mit einem pharmazeutisch verträglichen Träger oder Verdünnungsmittel in eine pharmazeutisch verträgliche Form gebracht wird.

**Revendications**
**Revendications pour les Etats contractants suivants : AT, BE, CH, LI DE, DK, FR, GB, IT, LU, NL, PT, SE**

**1.** Composé de la formule :

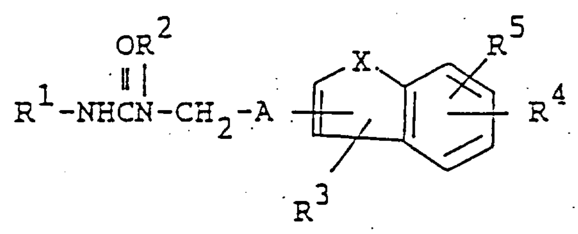

(I)

dans laquelle $R^1$ est un groupe phényl qui peut être substitué par un groupe alkyl en $C_1$-$C_6$, un atome d'halogène, un groupe nitro, un groupe amino, un groupe alkyl(amino en $C_1$-$C_6$), un groupe (alcoxy en $C_1$-$C_6$) ou acylamino,

$R^2$ est un atome d'hydrogène; un groupe alkyl en $C_1$-$C_{20}$; un groupe cyclo(alkyl en $C_3$-$C_{20}$); ou un groupe (alkyl en $C_1$-$C_6$) qui est substitue par un groupe cyclo(alkyl en $C_3$-$C_6$), un groupe cyclo(alcényl en $C_3$-$C_6$), ou un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$;

$R^3$ est un atome d'hydrogène, un groupe alkyl en $C_1$-$C_6$ ou un groupe phényl qui peut être substitué par un groupe alkyl en $C_1$-$C_6$, un atome d'halogène, un groupe nitro, amino ou (alkyl en $C_1$-$C_6$)amino;

$R^4$ est un atome d'hydrogène, un atome d'halogène, un groupe alkyl en $C_1$-$C_6$, alcoxy en $C_1$-$C_6$ ou phényl qui peut être substitué par un groupe alkyl en $C_1$-$C_6$ ouun atome d'halogène;

$R^5$ est un atome d'hydrogène, un atome d'halogène, un groupe alkyl en $C_1$-$C_6$ ou un groupe phényl qui peut être substitué par un groupe alkyl en $C_1$-$C_6$;

A est une liaison simple ou un groupe alkylène en $C_1$-$C_6$; et

X est O, S ou NH,

dans lesquels ou moins l'un de $R^3$, $R^4$ et $R^5$

n'est pas un atome d'hydrogène, à condition que $R^3$ soit un groupe phényl qui peut être substitué par un groupe alkyl en $C_1$-$C_6$, un atome d'halogène, un groupe nitro, amino ou (alkyl en $C_1$-$C_6$)amino, ou

$R^4$ est un atome d'halogène, un groupe alcoxy en $C_1$-$C_6$ ou un groupe phényl qui peut être substitué par un groupe alkyl en $C_1$-$C_6$ ou un atome d'halogène,

lorsque $R^2$ est un groupe cyclo(alkyl en $C_3$-$C_{20}$),

et un de ses sels pharmaceutiquement acceptable.

2. Composé selon la revendication 1,

dans lequel $R^2$ est un atome d'hydrogène; un groupe alkyl en $C_1$-$C_{20}$; un groupe cyclo(alkyl en $C_3$-$C_{20}$); ou un groupe alkyl en $C_1$-$C_6$ qui est substitué par un groupe cyclo(alkyl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$.

3. Composé selon la revendication 2,

dans lequel $R^1$ est un groupe phényl qui peut être substitué par un groupe alkyl en $C_1$-$C_6$, un atome d'halogène, un groupe nitro, un groupe amino, un groupe (alkyl en $C_1$-$C_6$)amino ou un groupe alcoxy en $C_1$-$C_6$ ,

$R^2$ est un groupe alkyl en $C_1$-$C_{20}$; un groupe cyclo(alkyl en $C_3$-$C_{20}$); ou un groupe alkyl en $C_1$-$C_6$ qui est substitué par un groupe cyclo(alkyl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$, et

$R^5$ est un atome d'hydrogène;

4. Composé selon la revendication 3,

dans lequel $R^2$ est un groupe alkyl en $C_1$-$C_{20}$; un groupe cyclo(alkyl en $C_3$-$C_{20}$); ou un groupe alkyl en $C_1$-$C_6$ qui est substitué par un groupe phényl substitué en option par un groupe alkyl en $C_1$-$C_6$.

5. Composé selon la revendication 3,

dans lequel $R^1$ est un groupe phényl qui peut être substitué par un groupe alkyl en $C_1$-$C_6$, un atome d'halogène ou un groupe alcoxy en $C_1$-$C_6$, et

X est O ou S.

6. Composé selon la revendication 5,

dans lequel $R^3$ est un atome d'hydrogène ou un groupe phényl qui peut être substitué par un groupe alkyl en $C_1$-$C_6$ ou un atome d'halogène, et

$R^4$ est un atome d'hydrogène, un atome d'halogène, un groupe alkyl en $C_1$-$C_6$ ou un groupe phényl qui peut être substitué par un groupe alkyl en $C_1$-$C_6$ ou un atome d'halogène.

7. Composé selon la revendication 6,

dans lequel $R^2$ est un groupe alkyl en $C_1$-$C_{20}$; un groupe cyclo(alkyl en $C_3$-$C_{20}$); ou un groupe alkyl en $C_1$-$C_6$ qui est substitué avec un groupe phényl substitué en option par un groupe alkyl en $C_1$-$C_6$ .

8. Composé selon la revendication 6,

dans lequel $R^1$ est un groupe phényl qui est substitué avec un 1 à 3 substituants alkyl en $C_1$-$C_6$, atome d'halogène ou groupe alcoxy en $C_1$-$C_6$.

$R^2$ est un groupe alkyl en $C_1$-$C_{20}$; un groupe cyclo(alkyl en $C_3$-$C_{20}$); ou alkyl en $C_1$-$C_6$ qui est substitué par un groupe cyclo(alkyl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$; et

$R^3$ est un groupe phényl qui peut être substitué par un groupe alkyl en $C_1$-$C_6$ ou un atome d'halogène; ou

$R^1$ et $R^2$ sont chacun tel que défini ci-dessus, et

$R^4$ est un atome d'halogène ou un groupe phényl qui peut être substitué par un groupe alkyl en $C_1$-$C_6$ ouun atome d'halogène.

**9.** Composé selon la revendication 8,

dans lequel $R^1$ est un groupe phényl substitué par 1 à 3 atomes d'halogène,

$R^2$ est un groupe alkyl en $C_1$-$C_6$ qui peut être substitué par un groupe furyl ou un groupe phényl substitué en option par un ou plusieurs substituants choisis dans le groupe constitué d'un groupe alkyl en $C_1$-$C_6$ et d'un atome d'halogène,

$R^3$ est un groupe phényl substitué par un groupe alkyl en $C_1$-$C_6$ ou un atome d'halogène, et

$R^4$ est un atome d'halogène ou un groupe alkyl en $C_1$-$C_6$.

**10.** Composé selon la revendication 9,

dans le quel R' est un groupe phényl substitué par 3 atomes d'halogène,

$R^2$ est un groupe alkyl en $C_1$-$C_6$ substitué par un groupe furyl ou un groupe phényl,

$R^3$ est un groupe phényl substitué par un atome d'halogène,

$R^4$ est un groupe alkyl en $C_1$-$C_6$, et

X est $\theta$.

**11.** N-Benzyl-N-[3-(4-chlorophényl)-5-méthylbenzofuran-2-ylméthyl]-N'-(2,4,6-trifluorophényl)urée.

**12.** N-[3-(4-Bromophényl)-5-méthylbenzofuran-2-ylméthyl]-N-furfuryl-N'-(2,4,6-trifluorophényl)urée.

**13.** Procédé pour préparer un composé de la formule :

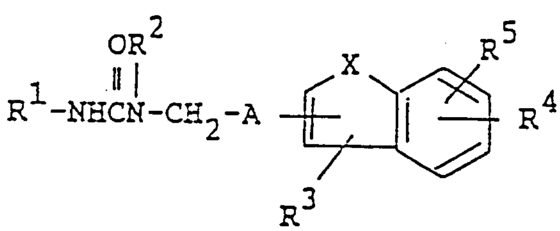

(I)

dans laquelle $R^1$ est un groupe phényl qui peut être substitué par un groupe alkyl en $C_1$-$C_6$, un atome d'halogène, un groupe nitro, un groupe amino, un groupe (alkyl en $C_1$-$C_6$)amino, un groupe alcoxy en $C_1$-$C_6$ ou un groupe acylamino,

$R^2$ est un atome d'hydrogène; un groupe alkyl en $C_1$-$C_{20}$; un groupe cyclo(alkyl en $C_3$-$C_{20}$); ou un groupe alkyl en $C_1$-$C_6$ qui peut être substitué par un groupe cyclo(alkyl en $C_3$-$C_6$), un groupe cyclo-(alcényl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$;

$R^3$ est un atome d'hydrogène, un groupe alkyl en $C_1$-$C_6$ ou un groupe phényl qui peut être substitué par un groupe alkyl en $C_1$-$C_6$, un atome d'halogène, un groupe nitro, un groupe amino ou un groupe (alkyl en $C_1$-$C_6$)amino,

$R^4$ est un atome d'hydrogène, un atome d'halogène, un groupe alkyl en $C_1$-$C_6$, un groupe alcoxy en $C_1$-$C_6$ ou un groupe phényl qui peut être substitué par un groupe alkyl en $C_1$-$C_6$ ou un atome d'halogène,

$R^5$ est un atome d'hydrogène, un atome d'halogène, un groupe alkyl en $C_1$-$C_6$ ou un groupe phényl qui peut être substitué par un groupe alkyl en $C_1$-$C_6$,

A est une liaison simple ou un groupe alkylène en $C_1$-$C_6$, et

X est O, S ou NH,

dans lesquels au moins l'un de $R^3$, $R^4$ et $R^5$ n'est pas un atome d'hydrogène,

à condition que $R^3$ soit un groupe phényl qui peut être substitué par un groupe alkyl en $C_1$-$C_6$, un atome d'halogène, un groupe nitro, un groupe amino ou un groupe (alkyl en $C_1$-$C_6$)amino, ou

$R^4$ est un atome d'halogène, un groupe alcoxy en $C_1$-$C_6$ ou un groupe phényl qui peut être substitué par un groupe alkyl en $C_1$-$C_6$ ou un atome d'halogène,

lorsque $R^2$ est un groupe cyclo(alkyl en $C_3$-$C_{20}$),

ou l'un de ses sels pharmaceutiquement acceptable, qui comprend :

(a) le fait de faire réagir un composé de la formule :

$R^1$-NCO (II)

avec un composé de la formule :

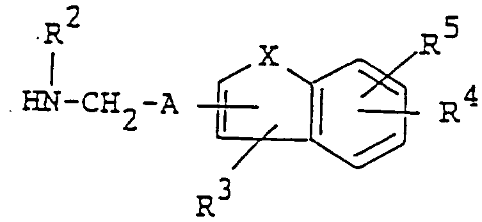

(III)

ou son sel pour fournir un composé de la formule :

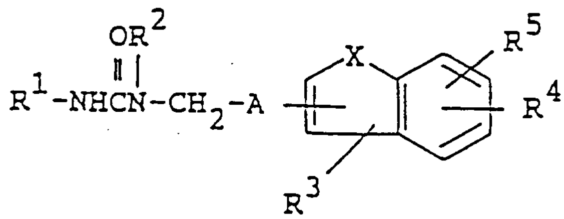

(I)

ou son sel, dans les formules précédentes,

$R^1$, $R^2$, $R^3$, $R^4$, $R^5$, A et X sont chacun tel que défini ci-dessus, ou

(b) le fait de soumettre un composé de la formule :

$R^1$-$NH_2$ (IV)

ou son sel et un composé de la formule :

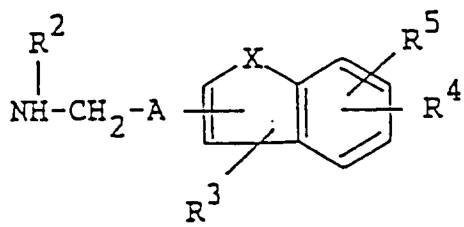

(III)

ou son sel à une réaction de formation d'un groupe uréido pour fournir un composé de la formule :

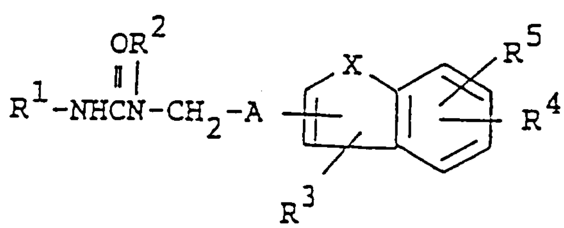

(I)

ou son sel; dans les formules précédentes,

$R^1$, $R^2$, $R^3$, $R^4$, $R^5$, A et X sont chacun tel que défini ci-dessus, ou

(c) le fait de soumettre un composé de la formule :

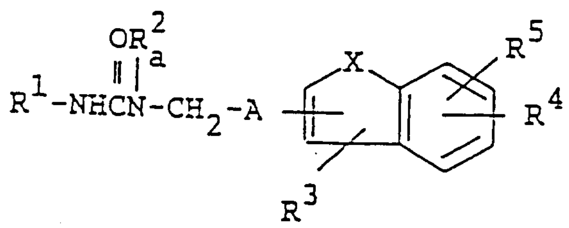

(Ia)

ou son sel à une réaction de déalkylation pour fournir un composé de la formule :

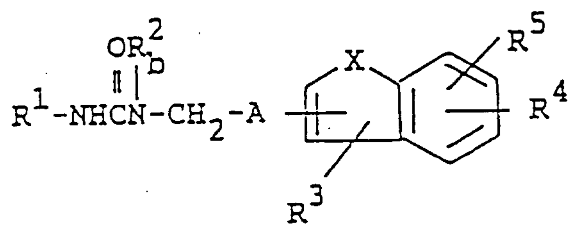

(Ib)

ou son sel; dans les formules ci-dessus :

$R^1$, $R^3$, $R^4$, $R^5$, A et X sont chacun tel que défini ci-dessus,

$R_a^2$ est un groupe alkyl en $C_1$-$C_6$ substitué par un groupe phényl ou un groupe phényl substitué par un groupe alkyl en $C_1$-$C_6$, dont chacun est substitué par un groupe alcoxy en $C_1$-$C_6$, et

$R_b^2$ est un groupe alkyl en $C_1$-$C_6$ substitué par un groupe phényl ou un groupe phényl substitué par un groupe alkyl en $C_1$-$C_6$ substitué par un groupe hydroxy, ou

(d) le fait de soumettre un composé de la formule :

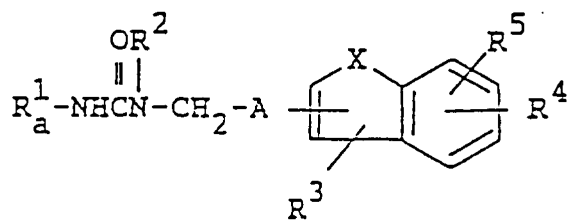

(Ic)

ou son sel à une réduction
afin de fournir un composé de la formule :

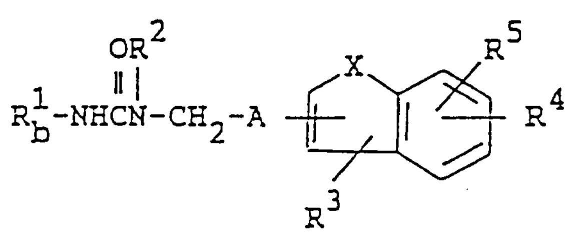

(Id)

ou son sel; dans les formules ci-dessus,

$R^2$, $R^3$, $R^4$, $R^5$, A et X sont chacun tel que défini ci-dessus,

$R_a^1$ est un groupe phényl ou un groupe phényl substitué par un groupe alkyl en $C_1$-$C_6$, dont chaun est substitué par un groupe nitro, et

$R_b^1$ est un groupe phényl ou un groupe phényl substitué par un groupe alkyl en $C_1$-$C_6$ dont

chacun est substitué par un groupe amino, ou
(e) le fait de réagir un composé de la formule :

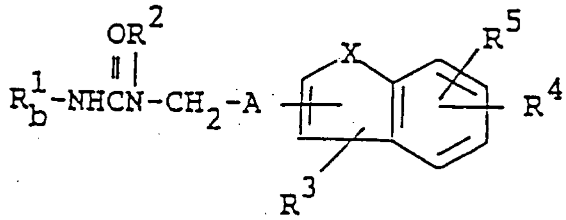
(Id)

ou son sel à un agent d'acylation
pour fournir un composé de la formule :

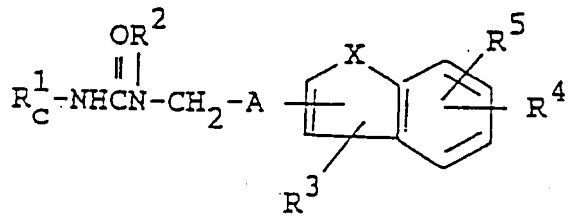
(Ie)

ou son sel; dans les formules ci-dessus,
$R_b^1$ , $R^2$, $R^3$, $R^4$, $R^5$, A et X sont chacun tel que défini ci-dessus, et
$R_c^1$ est un groupe phényl ou un groupe phényl substitué par un groupe alkyl en $C_1$-$C_6$, dont chacun est substitué par un groupe acylamino, ou
(f) le fait de faire réagir un composé de la formule :

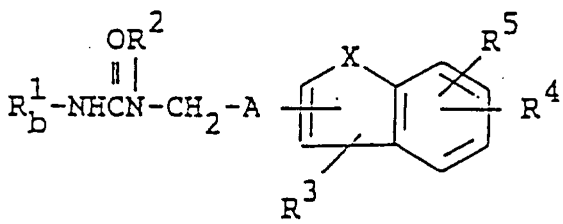
(Id)

ou son sel avec un agent d'alkylation
pour fournir un composé de la formule :

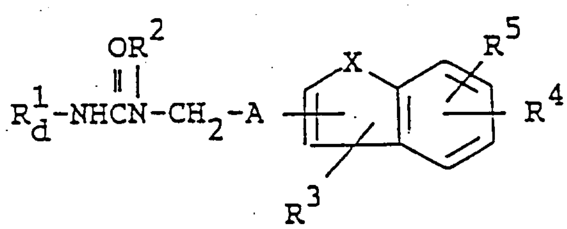
(If)

ou son sel; dans les formules ci-dessus :
$R_b^1$ ,$R^2$, $R^3$, $R^4$, $R^5$, A et X sont chacun tel que défini ci-dessus, et
$R_d^1$ est un groupe phényl ou un groupe phényl substitué par un groupe alkyl en $C_1$-$C_6$, dont chacun est substitué par un groupe (alkyl en $C_1$-$C_6$)amino.

**14.** Composition pharmaceutique comprenant un composé de la revendication 1, comme ingrédient actif, en association avec un support ou un excipient pharmaceutiquement acceptable, sensiblement non toxique.

**15.** Composé de la revendication 1 pour emploi comme médicament.

**16.** Composé de la revendication 1 pour emploi dans le traitement thérapeutique de l'hypercholestérolémie, de l'hyperlipédémie, de l'athérosclérose ou des maladies ainsi provoquées.

**17.** Emploi d'un composé de la revendication 1 pour la fabrication d'un médicament pour le traitement de l'hypercholestérolémie, de l'hyperlipédémie, de l'athérosclérose ou des maladies ainsi provoquées.

**Revendications pour les Etats contractants suivants : ES, GR**

**1.** Procédé pour préparer un composé de la formule :

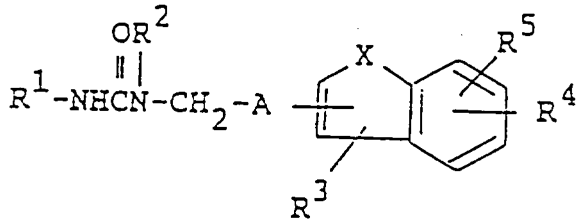

(I)

dans laquelle $R^1$ est un groupe phényl qui peut être substitué par un groupe alkyl en $C_1$-$C_6$, un atome d'halogène, un groupe nitro, un groupe amino, un groupe (alkyl en $C_1$-$C_6$)amino, un groupe alcoxy en $C_1$-$C_6$ ou un groupe acylamino,

$R^2$ est un atome d'hydrogène; un groupe alkyl en $C_1$-$C_{20}$; un groupe cyclo(alkyl en $C_3$-$C_{20}$); ou un groupe alkyl en $C_1$-$C_6$ qui est substitué par un groupe cyclo(alkyl en $C_3$-$C_6$), un groupe cyclo(alcényl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$;

$R^3$ est un atome d'hydrogène, un groupe alkyl en $C_1$-$C_6$ ou un groupe phényl qui peut être substitué par un groupe alkyl en $C_1$-$C_6$, un atome d'halogène, un groupe nitro, un groupe amino ou un groupe (alkyl en $C_1$-$C_6$)amino;

$R^4$ est un atome d'hydrogène, un atome d'halogène, un groupe alkyl en $C_1$-$C_6$, un groupe alcoxy en $C_1$-$C_6$ ou un groupe phényl qui peut être substitué par un groupe alkyl en $C_1$-$C_6$ ou un atome d'halogène;

$R^5$ est un atome d'hydrogène, un atome d'halogène, un groupe alkyl en $C_1$-$C_6$ ou un groupe phényl qui peut être substitué par un groupe alkyl en $C_1$-$C_6$;

A est une liaison simple ou un groupe alkylène en $C_1$-$C_6$; et

X est O, S ou NH,

dans lesquels ou moins l'un de $R^3$, $R^4$ et $R^5$ n'est pas un atome d'hydrogène,

à condition que $R^3$ soit un groupe phényl qui peut être substitué par un groupe alkyl en $C_1$-$C_6$, un atome d'halogène, un groupe nitro, un groupe amino ou un groupe (alkyl en $C_1$-$C_6$)amino, ou

$R^4$ est un atome d'halogène, un groupe alcoxy en $C_1$-$C_6$ ou un groupe phényl qui peut être substitué par un groupe alkyl en $C_1$-$C_6$ ou un atome d'halogène,

lorsque $R^2$ est un groupe cyclo(alkyl en $C_3$-$C_{20}$),

ou un de ses sels pharmaceutiquement acceptable,

qui comprend :

(a) le fait de faire réagir un composé de la formule :

$R^1$-NCO (II)

avec un composé de la formule :

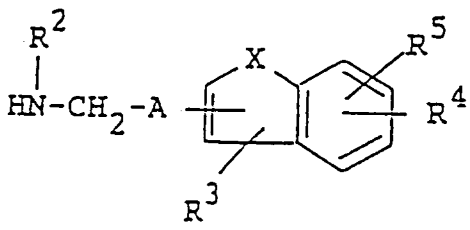

(III)

ou son sel pour fournir un composé de la formule :

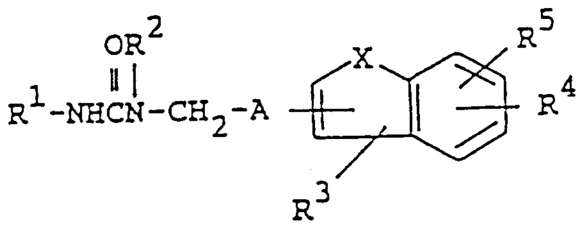

(I)

ou son sel; dans les formules ci-dessus :

$R^1$, $R^2$, $R^3$, $R^4$, $R^5$, A et X sont chacun tel que défini ci-dessus, ou

(b) le fait de soumettre un composé de la formule :

$R^1$-$NH_2$ (IV)

ou son sel et un composé de la formule :

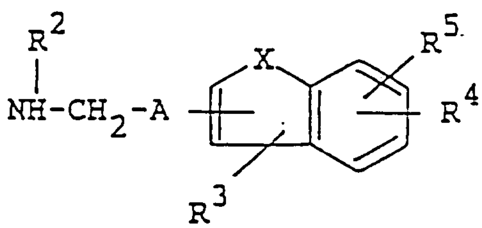

(III)

ou son sel à une réaction de formation du groupe uréido pour fournir un composé de la formule :

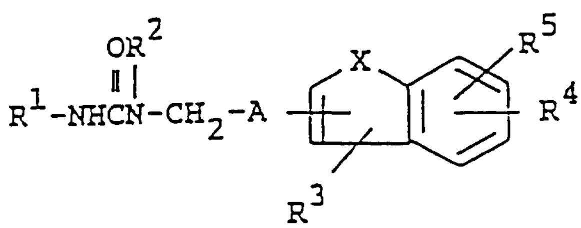

(I)

ou son sel; dans les formules ci-dessus :

$R^1$, $R^2$, $R^3$, $R^4$, $R^5$, A et X sont chacun tel que défini ci-dessus, ou

(c) le fait de soumettre un composé de la formule :

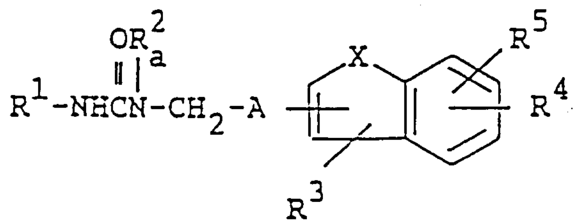

(Ia)

ou son sel à une réaction de déalkylation pour fournir un composé de la formule :

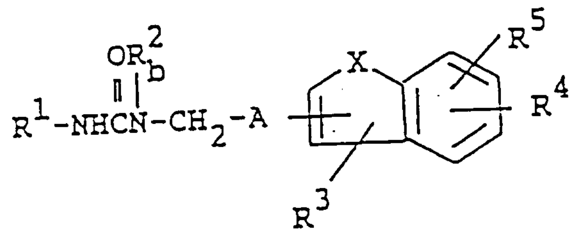

(Ib)

ou son sel; dans les formules ci-dessus,

$R^1$, $R^3$, $R^4$, $R^5$, A et X sont chacun tel que défini ci-dessus,

$R^2_a$ est un groupe alkyl en $C_1$-$C_6$ substitué par un groupe phényl ou un groupe phényl substitué par un groupe alkyl en $C_1$-$C_6$, dont chacun est substitué par un groupe alcoxy $C_1$-$C_6$, et

$R^2_b$ est un groupe alkyl en $C_1$-$C_6$ substitué par un groupe phényl ou un groupe phényl substitué par un groupe alkyl en $C_1$-$C_6$, dont chacun est substitué par un groupe hydroxy, ou

(d) le fait de soumettre un composé de la formule :

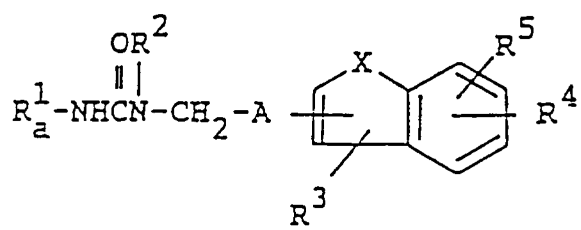

(Ic)

ou son sel à une réduction
pour fournir un composé de la formule :

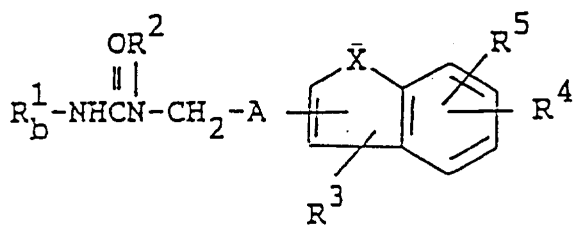

(Id)

ou son sel; dans les formules ci-dessus :

$R^2$, $R^3$, $R^4$, $R^5$, A et X sont chacun tel que défini ci-dessus,

$R^1_{a_1}$

est un groupe phényl ou un groupe phényl substitué par un groupe alkyl en $C_1$-$C_6$, dont chacun est substitué par un groupe nitro, et

$R_b^1$ est un groupe phényl ou un groupe phényl substitué par un groupe alkyl en $C_1$-$C_6$, dont chacun est substitué par un groupe amino, ou

(e) le fait de faire réagir un composé de la formule :

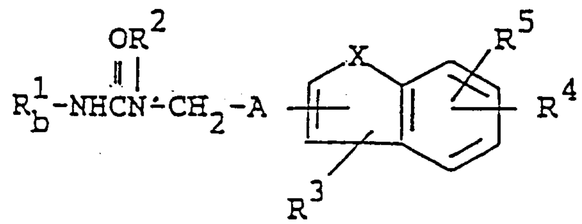

(Id)

ou son sel avec un agent d'acylation
pour fournir un composé de la formule :

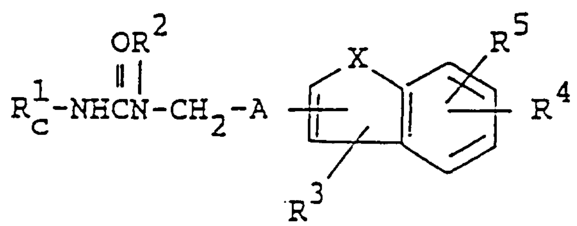

(Ie)

ou son sel; dans les formules ci-dessus,

$R_b^1$ , $R^2$, $R^3$, $R^4$, $R^5$, A et X sont chacun tel que défini ci-dessus, et

$R_b^1$ est un groupe phényl ou un groupe phényl substitué par un groupe alkyl en $C_1$-$C_6$, dont chacun est substitué par un groupe acylamino, ou

(f) le fait de faire réagir un composé de la formule :

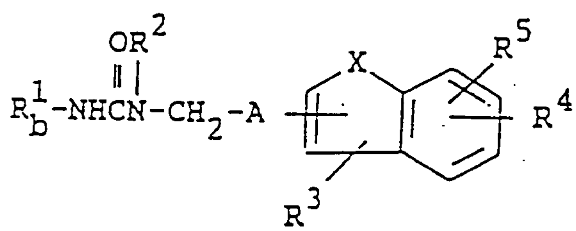

(Id)

ou son sel avec un agent d'akylation
pour fournir un composé de la formule :

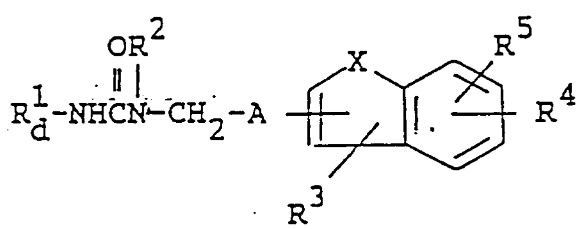

(If)

ou son sel; dans les formules ci-dessus,

$R_b^1$ , $R^2$, $R^3$, $R^4$, $R^5$, A et X sont chacun tel que défini ci-dessus, et

$R_d^1$ est un groupe phényl ou un groupe phényl substitué par un groupe alkyl en $C_1$-$C_6$ dont chacun est substitué par un groupe (alkyl en $C_1$-$C_6$)amino.

**2.** Procédé selon la revendication 1,

dans lequel $R^2$ est un atome d'hydrogène; un groupe alkyl en $C_1$-$C_{20}$; un groupe cyclo(alkyl en $C_3$-$C_{20}$); ou un groupe alkyl en $C_1$-$C_6$ qui est substitué par un groupe cyclo(alkyl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$.

**3.** Procédé selon la revendication 2,

dans lequel $R^1$ est un groupe phényl qui peut être substitué par un groupe alkyl en $C_1$-$C_6$, un atome d'halogène, un groupe nitro, un groupe amino, un groupe (alkyl en $C_1$-$C_6$)amino ou un groupe alcoxy en $C_1$-$C_6$,

$R^2$ est un groupe alkyl en $C_1$-$C_{20}$; un groupe cyclo(alkyl en $C_3$-$C_{20}$); ou un groupe alkyl en $C_1$-$C_6$ qui est substitué par un groupe cyclo(alkyl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$, et

$R^5$ est un atome d'hydrogène.

**4.** Procédé selon la revendication 3,

dans lequel $R^2$ est un groupe alkyl en $C_1$-$C_{20}$; un groupe cyclo(alkyl en $C_3$-$C_{20}$); ou un groupe alkyl en $C_1$-$C_6$ qui est substitué par un groupe phényl substitué en option par un groupe alkyl en $C_1$-$C_6$.

**5.** Procédé selon la revendication 3,

dans lequel $R^1$ est un groupe phényl qui peut être substitué par un groupe alkyl en $C_1$-$C_6$, un atome d'halogène ou un groupe alcoxy en $C_1$-$C_6$, et

X est O ou S.

**6.** Procédé selon la revendication 5,

dans lequel $R^3$ est un atome d'hydrogène ou un groupe phényl qui peut être substitué par un groupe alkyl en $C_1$-$C_6$ ou un atome d'halogène, et

$R^4$ est un atome d'hydrogène, un atome d'halogène, un groupe alkyl en $C_1$-$C_6$ ou un groupe phényl qui peut être substitué par un groupe alkyl en $C_1$-$C_6$ ou un atome d'halogène.

**7.** Procédé selon la revendication 6,

dans lequel $R^2$ est un groupe alkyl en $C_1$-$C_{20}$; un groupe cyclo(alkyl en $C_3$-$C_{20}$); ou un groupe alkyl en $C_1$-$C_6$ qui est substitué par un groupe phényl substitué en option par un groupe alkyl en $C_1$-$C_6$ .

**8.** Procédé selon la revendication 6,

dans lequel $R^1$ est un groupe phényl qui est substitué par un 1 à 3 substituants : groupe alkyl en $C_1$-$C_6$,atome d'halogène ou groupe alcoxy en $C_1$-$C_3$.

$R^2$ est un groupe alkyl en $C_1$-$C_{20}$; un groupe cyclo(alkyl en $C_3$-$C_{20}$); ou alkyl en $C_1$-$C_6$ qui est substitué par un groupe cyclo(alkyl en $C_3$-$C_6$), un groupe furyl ou un groupe phényl substitué en option par un ou plusieurs substituants choisis dans le groupe constitué d'un groupe alkyl en $C_1$-$C_6$, d'un atome d'halogène, d'un groupe hydroxy et d'un groupe alcoxy en $C_1$-$C_6$; et

$R^3$ est un groupe phényl qui peut être substitué avec un groupe alkyl en $C_1$-$C_6$ ou un atome d'halogène; ou

$R^1$ et $R^2$ sont chacun tel que défini ci-dessus, et

$R^4$ est un atome d'halogène ou un groupe phényl qui peut être substitué par un groupe alkyl en $C_1$-$C_6$ ouun atome d'halogène.

**9.** Procédé selon la revendication 8,

dans lequel $R^1$ est un groupe phényl substitué par 1 à 3 atomes d'halogène,

$R^2$ est un groupe alkyl en $C_1$-$C_6$ qui peut être substitué par un groupe furyl ou un groupe phényl substitué en option par un ou plusieurs substituants choisis dans le groupe constitué d'un groupe alkyl en $C_1$-$C_6$ et d'un atome d'halogène,
$R^3$ est un groupe phényl substitué par un groupe alkyl en $C_1$-$C_6$ ou un atome d'halogène, et
$R^4$ est un atome d'halogène ou un groupe alkyl en $C_1$-$C_6$.

**10.** Procédé selon la revendication 9,
dans le quel R' est un groupe phényl substitué par 3 atomes d'halogène,
$R^2$ est un groupe alkyl en $C_1$-$C_6$ substitué par un groupe furyl ou un groupe phényl,
$R^3$ est un groupe phényl substitué par un atome d'halogène,
$R^4$ est un groupe alkyl en $C_1$-$C_6$, et
X est $\theta$.

**11.** Procédé selon la revendication 1 pour préparer la N-Benzyl-N-[3-(4-chlorophényl)-5-méthylbenzofuran-2-ylméthyl]-N'-(2,4,6-trifluorophényl)urée.

**12.** Procédé selon la revendication 1 pour préparer la N-[3-(4-Bromophényl)-5-méthylbenzofuran-2-ylméthyl]-N-furfuryl-N'-(2,4,6-trifluorophényl)urée.

**13.** Modification du procédé selon la revendication 1, dans lequel un composé de la formule I ou un sel pharmaceutiquement acceptable de celui-ci est mis sous forme pharmaceutiquement acceptable, par mélange ou dilution dudit composé (I) avec un support ou un diluant pharmaceutiquement acceptable.